(19) Europäisches Patentamt
European Patent Office
Office européen des brevets

(11) Veröffentlichungsnummer: **0 423 523 A2**

(12) **EUROPÄISCHE PATENTANMELDUNG**

(21) Anmeldenummer: **90118654.4**

(22) Anmeldetag: **28.09.90**

(51) Int. Cl.5: **C07D 333/38**, C07D 307/68, C07D 409/12, C07D 407/12, C07D 417/12, A01N 43/08, A01N 43/10

(30) Priorität: **07.10.89 DE 3933573**

(43) Veröffentlichungstag der Anmeldung: **24.04.91 Patentblatt 91/17**

(84) Benannte Vertragsstaaten: **BE CH DE ES FR GB IT LI NL SE**

(71) Anmelder: **BASF Aktiengesellschaft**
**Carl-Bosch-Strasse 38**
**W-6700 Ludwigshafen(DE)**

(72) Erfinder: **Muenster, Peter, Dr. Dipl.-Chem.**
**Ahornweg 14**
**W-6823 Neulussheim(DE)**

Erfinder: **Steiner, Gerd, Dr. Dipl.-Chem.**
**Oberer Waldweg 1**
**W-6719 Kirchheim(DE)**
Erfinder: **Freund, Wolfgang, Dr. Dipl.-Chem.**
**Johann-Gottlieb-Fichte-Strasse 71**
**W-6730 Neustadt(DE)**
Erfinder: **Wuerzer, Bruno, Dr. Dipl.-Landw.**
**Ruedigerstrasse 13**
**W-6701 Otterstadt(DE)**
Erfinder: **Westphalen, Karl-Otto, Dr. Dipl.-Chem.**
**Mausbergweg 58**
**W-6720 Speyer(DE)**

(54) **Carbonsäureamide, Verfahren zu ihrer Herstellung und ihre Verwendung als Herbizide.**

(57) Carbonsäureamide der Formeln Ia bis Ic

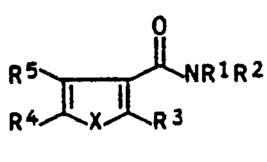
Ia

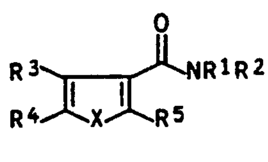
Ib

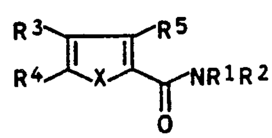
Ic

in denen die Substituenten folgende Bedeutung haben:
X
Sauerstoff oder Schwefel;
$R^1$
Wasserstoff, gegebenenfalls substituiertes Cycloalkyl oder Alkyl;
$R^2$
Hydroxy, Alkoxy, Cyanalkyl, gegebenenfalls substituiertes Alkenyl, Alkinyl, Phenyl oder Naphthyl, gegebenenfalls substituierter 5- bis 6-gliedriger Heterocyclus oder eine der bei $R^1$ genannten Gruppen oder
$R^1$ und $R^2$ gemeinsam eine 4- bis 7-gliedrige Kette, welche neben Methylengruppen eine der folgenden Gruppen als Ringglied enthalten kann: Sauerstoff, Schwefel, N-Methyl oder Carbonyl;
$R^3$, $R^4$
Nitro, Cyano, Halogen, gegebenenfalls substituiertes Amino, Alkoxy, Alkylthio, gegebenenfalls substituierter 5-

EP 0 423 523 A2

bis 6-gliedriger Heterocyclus, gegebenenfalls substituiertes Alkenyl, Alkinyl, Phenyl, Phenoxy oder Phenylthio oder eine der bei $R^1$ genannten Gruppen;
$R^5$
Formyl, 4,5-Dihydrooxazol-2-yl oder eine Gruppe $COYR^6$ (Y = O,S);
$R^6$ Wasserstoff, Cycloalkyl, gegebenenfalls substituiertes Alkyl, Alkenyl, Alkinyl, Cycloalkenyl oder Phenyl, ein 5- bis 6-gliedriger Heterocyclus, Phthalimido, Tetrahydrophthalimido, Succinimido, Maleinimido, Benzotriazolyl oder
eine Gruppe $-N=CR^7R^8$, wobei
$R^7$, $R^8$ Wasserstoff oder Alkyl bedeuten, $R^8$ darüberhinaus Cycloalkyl, Phenyl, Furyl oder $R^7$, $R^8$ gemeinsam eine 4- bis 7-gliedrige Alkylenkette bilden;
wobei sofern
- $R^5$ Carboxyl, Methoxycarbonyl oder Ethoxycarbonyl und $R^2$ Wasserstoff bedeutet, $R^3$ nicht Wasserstoff bedeutet oder $R^4$ nicht Wasserstoff oder Methyl bedeutet und sofern
- $R^5$ Carboxyl, Methoxycarbonyl oder Ethoxycarbonyl und $R^4$ Wasserstoff bedeutet, $R^3$ nicht Wasserstoff oder $R^2$ nicht eine der folgenden Gruppen bedeutet: Wasserstoff; $C_1$-$C_4$-Alkyl, Phenyl; 2-(3,4-Dimethoxyphenyl)ethyl oder 2,5-Dichlorthien-3-yl;
Verfahren zur Herstellung der Verbindung I und diese enthaltende herbizide Mittel.

# CARBONSÄUREAMIDE, VERFAHREN ZU IHRER HERSTELLUNG UND IHRE VERWENDUNG ALS HERBIZIDE

Die vorliegende Erfindung betrifft Carbonsäureamide der allgemeinen Formeln Ia, Ib und Ic

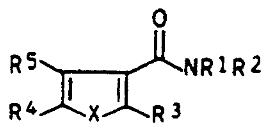

Ia

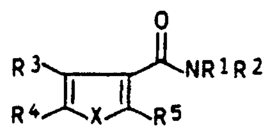

Ib

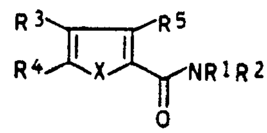

Ic

in denen die Substituenten folgende Bedeutung haben:
X
Sauerstoff oder Schwefel;
$R^1$
Wasserstoff;
$C_3$-$C_8$-Cycloalkyl, welches ein bis drei der folgenden Reste tragen kann: Halogen, $C_1$-$C_4$-Alkyl, $C_1$-$C_4$-Halogenalkyl, $C_1$-$C_4$-Alkoxy und/oder $C_1$-$C_4$-Halogenalkoxy;
$C_1$-$C_6$-Alkyl, welches ein bis drei der folgenden Reste tragen kann: Hydroxy, Halogen, $C_3$-$C_8$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-$C_1$-$C_4$-alkylamino und/oder $C_3$-$C_6$-Cycloalkylamino und/oder einen Rest

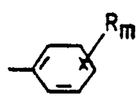

tragen kann, wobei
R Cyano; Nitro; Halogen; $C_1$-$C_4$-Alkyl; $C_1$-$C_4$-Halogenalkyl; $C_1$-$C_4$-Alkoxy; $C_1$-$C_4$-Halogenalkoxy; $C_1$-$C_4$-Alkylthio, $C_2$-$C_4$-Alkinyloxy; $C_1$-$C_4$-Halogenalkylthio, $C_3$-$C_6$-Alkoxycarbonylalkoxy und/oder $C_1$-$C_4$-Alkoxycarbonyl bedeutet und
m für 0, 1, 2 oder 3 steht, wobei die Reste R verschieden sein können, wenn m 2 oder 3 bedeutet;
$R^2$
Hydroxy; $C_1$-$C_4$-Alkoxy, $C_1$-$C_6$-Cyanalkyl;
$C_3$-$C_6$-Alkenyl, $C_3$-$C_6$-Alkinyl, Phenyl oder Naphthyl, wobei diese Gruppen ein bis drei der bei R genannten Reste tragen können;
einen 5- bis 6-gliedrigen Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
eine der bei $R^1$ genannten Gruppen oder
$R^1$ und $R^2$ gemeinsam eine 4- bis 7-gliedrige Kette, welche neben Methylengruppen eine der folgenden Gruppen als Ringglied enthalten kann: Sauerstoff, Schwefel, N-Methyl oder Carbonyl;
$R^3$, $R^4$
Nitro; Cyano; Halogen;
Amino, welches ein oder zwei $C_1$-$C_4$-Alkylgruppen und/oder eine $C_1$-$C_4$-Alkylcarbonylgruppe tragen kann;
$C_1$-$C_4$-Alkoxy oder $C_1$-$C_4$-Alkylthio, wobei diese Gruppen ein bis neun Halogenatome tragen können;
einen 5- bis 6-gliedrigen Heterocyclus, enthaltend ein oder zwei Heteroatome ausgewählt aus der Gruppe Sauerstoff, Schwefel und Stickstoff, wobei dieser Ring ein oder zwei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;
$C_2$-$C_6$-Alkenyl, $C_2$-$C_6$-Alkinyl, Phenyl, Phenoxy oder Phenylthio, wobei diese Gruppen ein bis drei der bei R genannten Reste tragen können, oder
eine der bei $R^1$ genannten Gruppen;
$R^5$ Formyl, 4,5-Dihydrooxazol-2-yl oder eine Gruppe $COYR^6$;
Y Sauerstoff oder Schwefel;
$R^6$ Wasserstoff;

$C_3$-$C_8$-Cycloalkyl;

$C_1$-$C_6$-Alkyl, welches ein bis fünf Halogenatome oder Hydroxygruppen und/oder einen der folgenden Reste tragen kann: Cyano, Aminocarbonyl, Carboxyl, Trimethylsilyl, $C_1$-$C_4$-Alkoxy, $C_1$-$C_4$-Alkoxy-$C_1$-$C_4$-alkoxy, $C_1$-$C_4$-Alkylthio, $C_1$-$C_4$-Alkylamino, Di-$C_1$-$C_4$-Alkylamino, $C_1$-$C_4$-Alkylsulfinyl, $C_1$-$C_4$-Alkylsulfonyl, $C_1$-$C_4$-Alkoxycarbonyl, $C_2$-$C_4$-Alkoxycarbonyl-$C_1$-$C_3$-alkoxy, $C_2$-$C_4$-Alkoxycarbonyl-$C_1$-$C_3$-alkoxycarbonyl, $C_1$-$C_4$-Alkylaminocarbonyl, Di-$C_1$-$C_4$-alkylaminocarbonyl, Di-$C_1$-$C_4$-alkylphosphonyl, $C_1$-$C_4$-Alkyliminoxi, Phenyl, Thienyl, Benzyloxy, Benzylthio, Furyl, Tetrahydrofuryl, Phthalimido, Pyridyl und/oder Benzoyl, wobei die cyclischen Reste ihrerseits ein bis drei der bei R genannten Reste tragen können;

$C_3$-$C_6$-Alkenyl, $C_3$-$C_6$-Alkinyl oder $C_5$-$C_7$-Cycloalkenyl, wobei diese Gruppen einen der folgenden Reste tragen können: Hydroxy, Halogen, $C_1$-$C_4$-Alkoxy oder Phenyl, wobei der Phenylrest seinerseits ein bis drei der bei R genannten Reste tragen kann;

einen 5- bis 6-gliedrigen Heterocyclus, enthaltend ein oder zwei Heteroatome, ausgewählt aus der Gruppe Stickstoff, Sauerstoff und Schwefel;

Phthalimido; Tetrahydrophthalimido; Succinimido; Maleinimido; Benzotriazolyl;

Phenyl, welches ein bis drei der bei R genannten Reste tragen kann;

eine Gruppe -N = $CR^7R^8$, wobei

$R^7$ Wasserstoff oder $C_1$-$C_6$-Alkyl und

$R^8$ $C_3$-$C_6$-Cycloalkyl, Phenyl, Furyl oder einen Rest $R^7$ bedeutet

oder

$R^7$, $R^8$ gemeinsam eine 4- bis 7-gliedrige Alkylenkette bilden, wobei sofern

- $R^5$ Carboxyl, Methoxycarbonyl oder Ethoxycarbonyl und $R^2$ Wasserstoff bedeutet, $R^3$ nicht Wasserstoff bedeutet oder $R^4$ nicht Wasserstoff oder Methyl bedeutet und sofern

- $R^5$ Carboxyl, Methoxycarbonyl oder Ethoxycarbonyl und $R^4$ Wasserstoff bedeutet, $R^3$ nicht Wasserstoff oder $R^2$ nicht eine der folgende Gruppen bedeutet: Wasserstoff; $C_1$-$C_4$-Alkyl; Phenyl; 2-(3,4-Dimethoxyphenyl)ethyl oder 2,5-Dichlorthien-3-yl,

sowie deren landwirtschaftlich brauchbaren Salze.

Außerdem betrifft die Erfindung ein Verfahren zur Herstellung der Verbindungen Ia, Ib oder Ic und herbizide Mittel, enthaltend mindestens ein Carbonsäureamid Ia, Ib oder Ic und/oder ein Carbonsäureamid der allgemeinen Formel IA, 1B oder IC

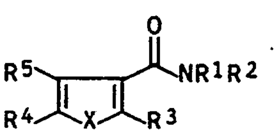

IA

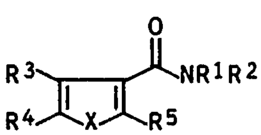

IB .

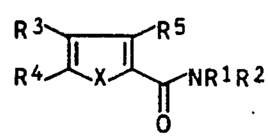

IC

in denen die Substituenten die in Anspruch 1 gegebene Bedeutung haben und zusätzlich

- $R^5$ Carboxyl, Methoxycarbonyl oder Ethoxycarbonyl und $R^2$ Wasserstoff bedeutet, wenn $R^3$ Wasserstoff bedeutet oder $R^4$ nicht Wasserstoff oder Methyl bedeutet und in denen

- $R^5$ Carboxyl, Methoxycarbonyl oder Ethoxycarbonyl und $R^4$ Wasserstoff bedeutet, wenn $R^3$ Wasserstoff oder $R^2$ nicht eine der folgende Gruppen bedeutet: Wasserstoff; $C_1$-$C_4$-Alkyl; Phenyl; 2-(3,4-Dimethoxyphenyl)ethyl oder 2,5-Dichlorthien-3-yl.

Aus der Literatur sind beispielsweise Carbonsäureamide der folgenden Formeln I′, I″ bzw. I″ bekannt.

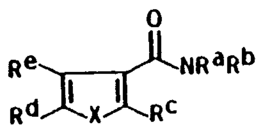

I'

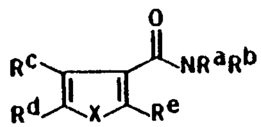

I''

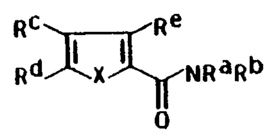

I'''

| Formel | X | $R^a$ | $R^b$ | $R^c$ | $R^d$ | $R^e$ | Literatur |
|---|---|---|---|---|---|---|---|
| I' | O | H | H | H | $CH_3$ | $CO_2CH_3$ | 1. |
| I' | O | H | H | H | H | $CO_2H$ | 1. |
| I' | O | H | H | H | H | $CO_2CH_2CH_3$ | 2. |
| I' | O | H | 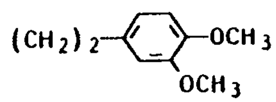 | H | H | $CO_2H$ | 3. |
| I' | O | H | 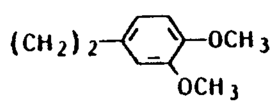 | H | H | $CO_2CH_2CH_3$ | 3. |
| I' | O | H | H | H | $CH_3$ | $CO_2CH_2CH_3$ | 4. |

| Formel | X | $R^a$ | $R^b$ | $R^c$ | $R^d$ | $R^e$ | Literatur |
|---|---|---|---|---|---|---|---|
| I' | S | H | 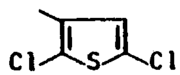 | H | H | $CO_2H$ | 5. |
| I' | S | H | $-C_6H_5$ | H | H | 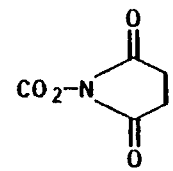 | 5. |
| I' | S | H | $-C_6H_5$ | H | H | $CO_2H$ | 5. |
| I'' | O | H | H | H | H | $CO_2H$ | 1. |
| I'' | O | H | H | H | H | $CO_2CH_2CH_3$ | 6. |
| I'' | S | H | H | H | H | CHO | 7. |
| I'' | S | $CH_3$ | $-C_6H_5$ | H | H | $CO_2CH_3$ | 8. |
| I'' | S | $CH_3$ | $-C_6H_5$ | H | H | $CO_2H$ | 8. |
| I'' | S | H | H | H | H | $CO_2H$ | 9. |
| I''' | O | H | $-C_6H_5$ | H | H | $CO_2H$ | 10. |
| I''' | O | H | H | $CH_3$ | H | $CO_2H$ | 11. |
| I''' | O | H | $C(CH_3)_3$ | H | H | $CO_2H$ | 12. |
| I''' | S | H | H | H | H | CHO | 7b. |
| I''' | S | $CH_3$ | $-C_6H_5$ | H | H | $CO_2CH_3$ | 8. |
| I''' | S | $CH_3$ | $-C_6H_5$ | H | H | $CO_2H$ | 8. |
| I''' | S | H | H | H | H | $CO_2H$ | 9. |
| I''' | S | H | $CH_2CH_3$ | H | H | $CO_2H$ | 12. |
| I''' | S | H | $C(CH_3)_3$ | H | H | CHO | 12. |
| I''' | S | H | $C(CH_3)_3$ | H | H | $CO_2H$ | 12. |

Literatur:

1. Bull. Soc. Chim. Fr., 1970, 1445

2. Rocz. Chem., 38, 511 (1964)

3. DE-A-31 43 876

4. C.R. Acad. Sci., Ser. C, 268, 1884 (1969)

5. DE-A-35 24 743

6. J. Am. Chem. Soc., 77, 4069 (1955)

7a. Bull. Soc. Chim. Fr, 1976, 628

7b. Acad. Sci., Ser. C, 276, 871 (1973)

8. J. Org. Chem., 189, 138 (1953)

9. J. Chem. Soc., 1937, 911

10. Acta Polytochim., 2, 19 (1924/26)

11. Helv. Chim. Acta, 14, 1270 (1931)

12. J. Org. Chem., 50, 4362 (1985).

Der vorliegenden Erfindung lagen neue herbizid wirksame Substanzen als Aufgabe zugrunde.

Demgemäß wurden die eingangs definierten Carbonsäureamide Ia, Ib und Ic und Verfahren zu ihrer Herstellung gefunden.

Des weiteren wurde gefunden, daß sich die Carbonsäureamide Ia, Ib und Ic sowohl als auch die Carbonsäureamide IA, IB und IC zur Bekämpfung unerwünschten Pflanzenwuchses eignen.

Die erfindungsgemäßen Carbonsäureamide Ia, Ib und Ic sind auf verschiedenen Wegen herstellbar. Man erhält sie beispielsweise nach den folgenden Verfahren.

1. Verfahren zur Herstellung der Verbindungen Ia, in denen $R^5$ Carboxyl bedeutet:

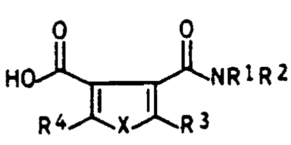

I

Man erhält diese Carbonsäureamide dadurch, daß man ein entsprechendes Dicarbonsäureanhydrid der Formel II in an sich bekannter Weise in einem Amin der Formel III umsetzt.

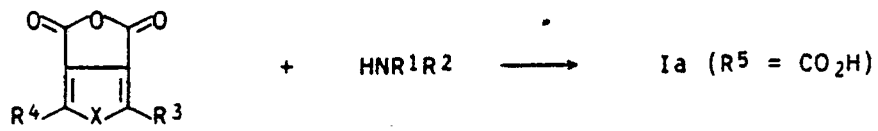


II III

Die Umsetzung wird in der Regel bei Temperaturen von -10 bis 100 °C, vorzugsweise 0 bis 30 °C in einem inerten organischen Lösungsmittel durchgeführt.

Als Lösungsmittel eignen sich Ether wie Methyl-tert.butylether, 1,2-Dimethoxyethan, Diethylenglykoldimethylether, Tetrahydrofuran und Dioxan.

Die Konzentration der Edukte im Lösungsmittel beträgt im allgemeinen 0,1 bis 5 mol/l, bevorzugt 0,2 bis 2 mol/l.

Das molare Verhältnis von II zu III beträgt im allgemeinen 1:5 bis 1:1, vorzugsweise 1:2 bis 1:1.

Die benötigten Dicarbonsäureanhydride II sind bekannt oder lassen sich in an sich bekannter Weise durch die Umsetzung der entsprechenden Dicarbonsäuren mit dem Anhydrid einer niederen Carbonsäure wie insbesondere Essig-säureanhydrid herstellen.

2. Verfahren zur Herstellung der Verbindungen Ia, Ib und Ic in denen $R^3$ und $R^4$ nicht Brom und Iod bedeuten und $R^5$ Formyl oder Carboxyl bedeutet:

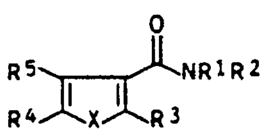 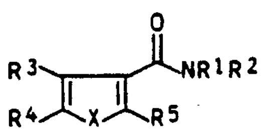 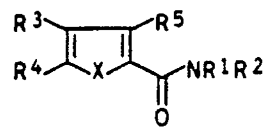

Ia Ib Ic

($R^3$, $R^4$ ≠ Br, J; $R^5$ = CHO, $CO_2H$)

Man erhält diese Verbindungen Ia, Ib und Ic dadurch, daß man eine entsprechende Carbonsäure der Formel IVa, IVb bzw. IVc in an sich bekannter Weise in das Halogenid oder eine andere aktivierte Form der Carbonsäure überführt, diese Derivate anschließend mit einem Amin der Formel III umsetzt und das so erhaltene Carbonsäureamid Va, Vb bzw. Vc danach in Gegenwart einer Base mit einem Formylierungs- oder einem Carboxylierungsreagens umsetzt.

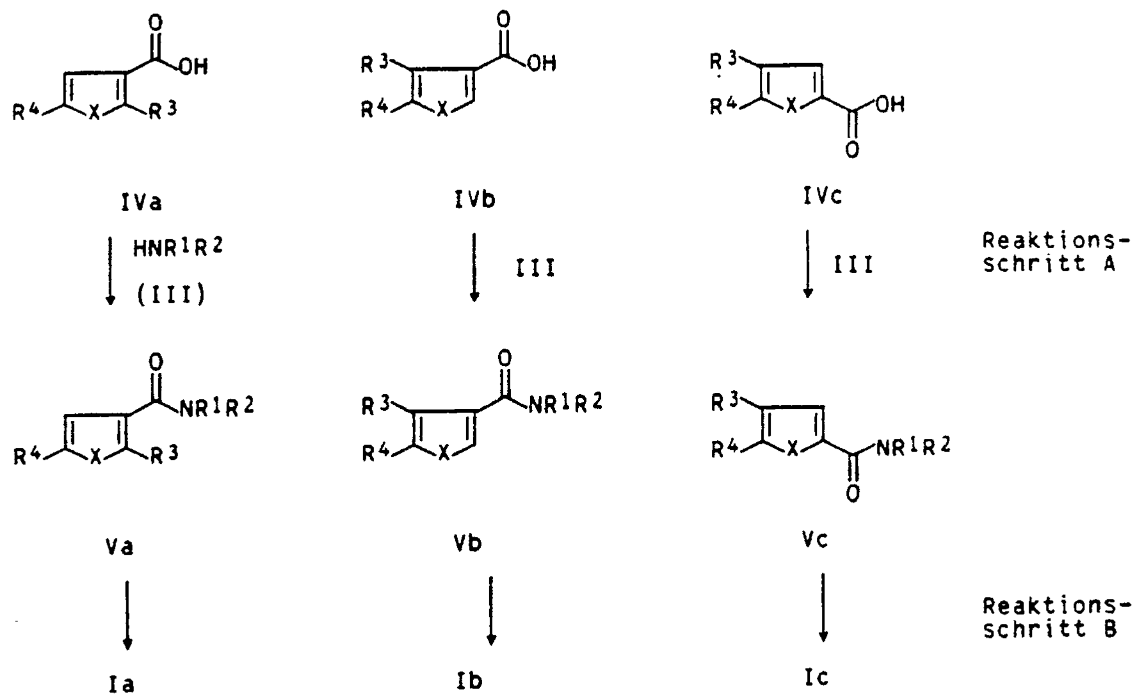


IVa IVb IVc

Va Vb Vc

Ia Ib Ic

$R^3$, $R^4$ ≠ Br, J; $R^5$ = CHO, $CO_2H$)

Die einzelnen Reaktionsschritte dieser Synthesesequenz können im allgemeinen wie folgt durchgeführt werden:

Reaktionsschritt A:

Man erhält die Verbindungen Va, Vb bzw. Vc aus den Säuren IVa, IVb und IVc, in dem man IVa, IVb bzw. IVc zunächst in an sich bekannter Weise in das Halogenid oder eine andere aktivierte Form der Carbonsäurefunktion überführt und diese Derivate anschließend mit einem Amin III amidiert.

Aktivierte Formen der Carbonsäure sind neben Halogeniden wie insbesondere den Chloriden und den Bromiden beispielsweise auch Imidazolide. Im allgemeinen werden die Halogenide bevorzugt.

Man erhält sie durch Umsetzung der Carbonsäuren IVa, IVb bzw. IVc mit einem Halogenierungsmittel wie Thionylchlorid, Thionylbromid, Phosphoroxychlorid bzw. -bromid, Phosphortri- und -pentachlorid bzw. -bromid, Phosgen sowie elementarem Chlor und Brom.

Das Halogenierungsmittel wird in 1 bis 5 mol-äq., vorzugsweise 1 bis 2 mol.äq., eingesetzt.

Die Umsetzung verläuft bei Temperaturen von 20°C bis zum Siedepunkt des Halogenierungsmittels bzw. sofern man in Gegenwart eines inerten organischen Lösungsmittels arbeitet, auch dessen Siedepunkt.

Als Lösungsmittel eignen sich beispielsweise Kohlenwasserstoffe und Halogenkohlenwasserstoffe wie Benzol, Toluol und Dichlormethan.

Üblicherweise werden die aktivierten Carbonsäurederivate isoliert, beispielsweise durch Abdestillieren des Halogenierungsmittels und sofern vorhanden des Lösungsmittels und erst anschließend mit den Aminen III umgesetzt.

In diesem Fall wird die Amidierung bei Temperaturen von -20 bis 50°C, vorzugsweise 0 bis 30°C in einem inerten aprotisch polaren organischen Lösungsmittel durchgeführt.

Für diese Umsetzung eignen sich insbesondere Halogenkohlenwasserstoffe wie Dichlormethan und Ether wie Diethylether und tert.-Butylmethylether als Lösungsmittel.

Da bei der Amidierung von Säurehalogeniden Halogenwasserstoff gebildet wird, empfiehlt es sich, das Amin III in 2 bis 5 mol.-äq. Überschuß, vorzugsweise 2 bis 3 mol.-äq. zuzusetzen. Sofern das Amin in äquimolaren Mengen (1 bis 1,2 mol-äq.) eingesetzt wird, sollte zum Binden des Halogenwasserstoffs eine Base, insbesondere ein tertiäres Amin wie Triethylamin oder Pyridin zugegeben werden.

Reaktionsschritt B:

Die Formylierung bzw. Carboxylierung der Carbonsäureamide Va bzw. Vb bzw. Vc erfolgt in der Regel bei Temperaturen von -100 bis -20 °C, vorzugsweise -80 bis -40 °C in einem aprotisch polaren inerten organischen Lösungsmittel unter Ausschluß von Feuchtigkeit in Gegenwart einer Base.

Als Formylierungsreagens dient insbesondere Dimethylformamid und N-Formyl-morpholin, bevorzugtes Carboxylierungsmittel ist Kohlendioxid.

Geeignete Lösungsmittel sind insbesondere Diethylether, tert.-Butylmethylether, Tetrahydrofuran und Dioxan.

Als Basen finden bevorzugt Alkalimetallkohlenwasserstoffe wie Methyllithium, n-Butyllithium, tert.-Butyllithium und Phenyllithium Verwendung.

Die Umsetzung wird üblicherweise so durchgeführt, daß zunächst eine Lösung des Carbonsäureamids Va bzw. Vb mit 1,3 bis 2,5 mol-äq. der gelösten Base versetzt wird, wobei ein im Ring metallisiertes Carbonsäureamid-derivat entsteht, welches bei der anschließenden Zugabe des elektrophilen Formylierungs- bzw. Carboxylierungsreagens' zum gewünschten Produkt Ia, Ib bzw. Ic abreagiert.

Da im Falle von $R^1$ oder $R^2$ = H das erste mol-Äquivalent Base lediglich den Amid-Stickstoff deprotoniert, werden in diesem Fall mindestens 2 mol-äq. an Base benötigt, um den Heterocyclus zu metallieren, vorzugsweise arbeitet man in diesem Fall in Gegenwart von 2 bis 2,5 mol-äq. der Base.

Die für dieses Verfahren benötigten Carbonsäuren IVa, IVb und IVc sind literaturbekannt oder können nach allgemeinen literaturbekannten Methoden, z.B. durch Oxidation aus den entsprechenden Alkoholen oder Aldehyden oder durch Hydrolyse aus den entsprechenden Nitrilen hergestellt werden (Beilstein, Hauptwerk und 1. bis 5. Ergänzungswerk, Band 18; The Chemistry of Heterocyclic Compounds, Interscience Publishers, New York, 1976, John Wiley & Sons, Inc., 1988, Vol. 44, Part I-III).

3. Verfahren zur Herstellung der Verbindungen Ib und Ic, in denen $R^3$ oder $R^4$ Halogen, $R^5$ eine Gruppe $CO_2R^6$ und $R^6$ Alkyl bedeutet:

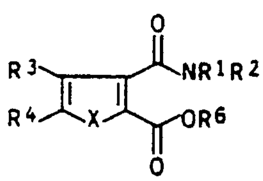

Ib

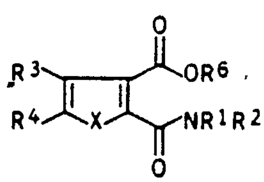

Ic

($R^3$ oder $R^4$ = Halogen; $R^6$ = Alkyl)

Man erhält diese Verbindungen Ib und Ic dadurch, daß man einen Dicarbonsäurediester der allgemeinen Formel VIa oder VIb in an sich bekannter Weise zunächst diazotiert und die diazotierte Verbindung mit einem anorganischen Halogenid in das entsprechende Derivat VIIa bzw. VIIb überführt, VIIa bzw. VIIb anschließend mit einem Amin der Formel III amidiert und das so erhaltene Gemisch der isomeren Verbindungen Ib und Ic in die Einzelkomponenten auftrennt.

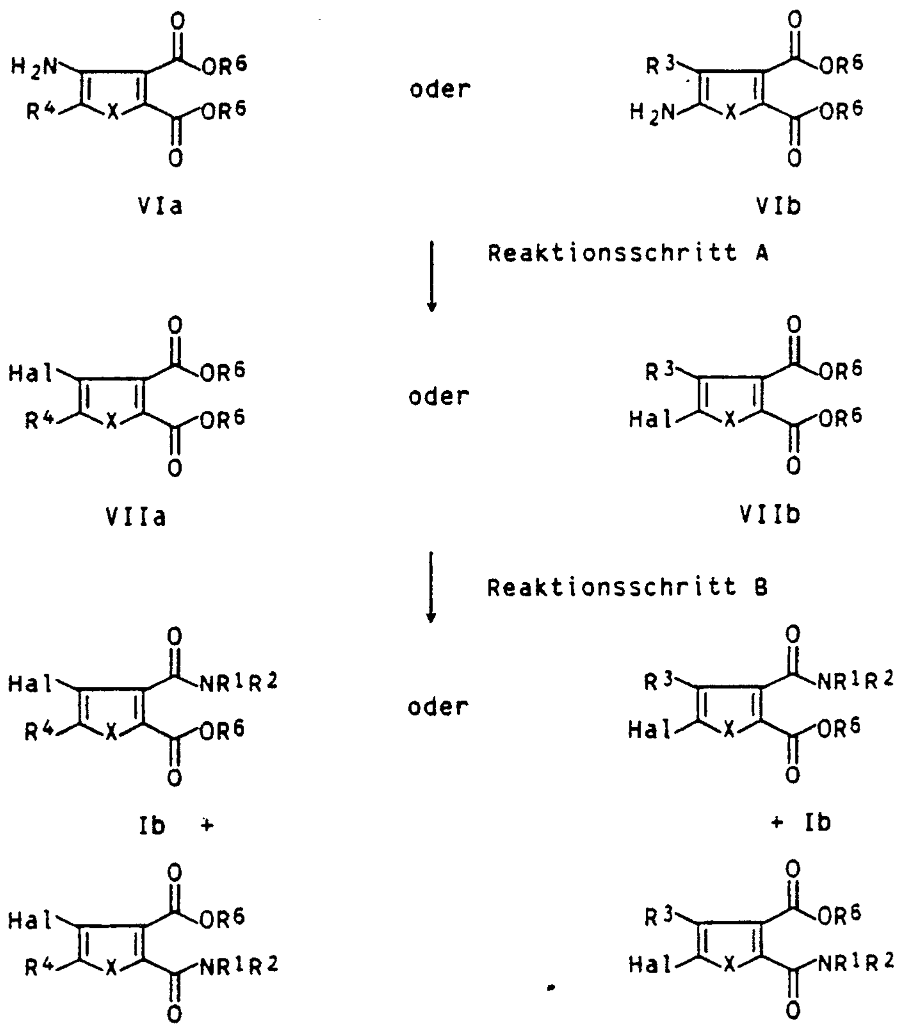


VIa oder VIb

VIIa oder VIIb

Ib + oder + Ib

Ic Ic

($R^3$ = Halogen; $R^6$ = Alkyl) oder ($R^4$ = Halogen; $R^6$ = Alkyl)

Hal bedeutet in den Formeln VIIa, VIIb, Ib und Ic ein Halogenatom wie Fluor, Chlor, Brom und Iod.

Die Reaktionsschritte dieser Synthesesequenz können im allgemeinen wie folgt durchgeführt werden:

Reaktionsschritt A:

Die Diazotierung der Dicarbonsäureester der allgemeinen Formel VIa oder IVb erfolgt in der Regel bei Temperaturen von -20 bis +20 °C, vorzugsweise -5 bis +10 °C in einer Mineralsäure wie insbesondere Salzsäure in Gegenwart eines Alkalimetallnitrits wie Natriumnitrit.

Das so erhaltene Diazoniumsalz wird anschließend in situ mit 1 bis 5 mol, vorzugsweise 1,5 bis 2,5 mol eines anorganischen Halogenids insbesondere eines Kupfer(I)halogenids umgesetzt.

Die Reaktionsbedingungen sind im Rahmen der für die Sandmeyer-Reaktion bekannten Verfahren variabel (s.a. Houben-Weyl, Bd. X/3, S. 1-212 (1965); Chem. Zvesti 36, 401 (1982)).

Reaktionsschritt B:

Die Umsetzung der so erhaltenen Dicarbonsäureester VIIa und VIIb mit dem Amin III erfolgt im allgemeinen und im besonderen analog zu den in Verfahren 1 geschilderten Bedingungen.

Insbesondere finden als Lösungsmittel hierbei jedoch Halogenkohlenwasserstoffe wie Methylenchlorid und Ether wie Diethylether, tert.-Butyl-methylether und Tetrahydrofuran Anwendung.

Das Amin III wird im allgemeinen in äquimolaren Mengen oder im Überschuß, vorzugsweise in Mengen

von 1 bis 1,2 mol-äq, bezogen auf VIIa bzw. VIIb eingesetzt.

Bei diesem Verfahren entstehen die isomeren Carbonsäureamide der Formein Ib und Ic in unterschiedlichen Mengen. Die Auftrennung des Isomerengemischs gelingt entweder durch fraktionierte Kristallisation oder auf chromatographischem Wege.

Die für dieses Verfahren benötigten Dicarbonsäurediester VIa und VIb sind bekannt oder können aus den entsprechenden Oxoestern Xa, beispielsweise analog den in Synthesis, 1977, 200 beschriebenen Bedingungen gemäß dem folgenden Reaktionsschema hergestellt werden:

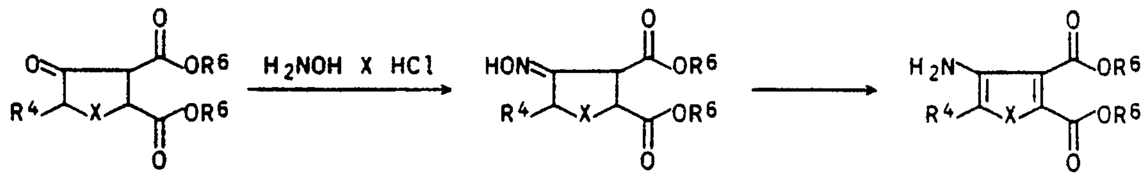


Xa VIa

4. Verfahren zur Herstellung der Verbindungen Ib und Ic, in denen $R^5$ eine Carboxylgruppe bedeutet

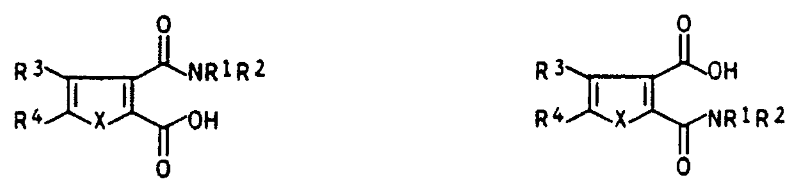

Ib Ic

Man erhält diese Carbonsäureamide Ib und Ic beispielsweise dadurch, daß man ein entsprechendes Carbonsäureamid der Formel Ib oder Ic in der $R^5$ eine Gruppe $CO_2R^6$ und $R^6$ Alkyl bedeutet in an sich bekannter Weise mit einer wäßrigen Base hydrolysiert.

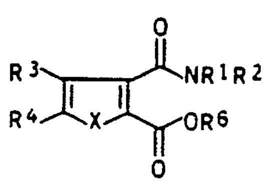

oder

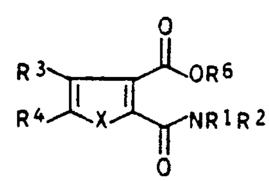

Ib Ic

($R^6$ = Alkyl)

$OH^{\ominus}$

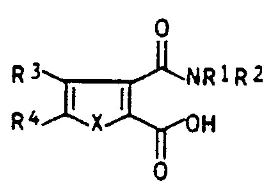

oder

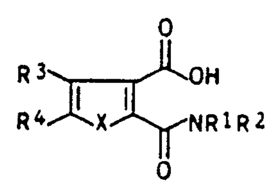

Ib Ic

Die Reaktion wird so durchgeführt, daß man ein Carbonsäureamid Ib bzw. Ic ($R^6$ = Alkyl) in einem inerten Lösungsmittel vorlegt und bei -30 bis 120° C, vorzugsweise bei -10 bis 40° C, mit einer wäßrigen Base umsetzt. Die Carbonsäureamide der Formel Ib bzw. Ic ($R^5$ = $CO_2H$) werden dann bei -30 bis 100° C, vorzugsweise bei -10 bis 10° C, durch Zugabe von Mineralsäuren freigesetzt.

Als Lösungsmittel für diese Esterspaltung kommen Alkohole wie Methanol, Ethanol, Propanol oder Ethylenglykol in Betracht; besonders bevorzugt arbeitet man in dem gleichen Alkohol, der der Esterkomponente $R^6OH$ entspricht. Die Konzentration des Edukts Ib bzw. Ic beträgt im allgemeinen 0,1 bis 5,0 mol/l, bevorzugt 0,2 bis 2,0 mol/l.

Als wäßrige Base setzt man wäßrige Lösungen von Alkali- oder Erdalkalihydroxiden, wie LiOH, NaOH, KOH, $Ca(OH)_2$ oder $Ba(OH)_2$, bevorzugt NaOH oder KOH ein. Die Hydroxide werden dabei in Form einer 5 bis 20 %igen wäßrigen Lösung verwendet.

Die molaren Verhältnisse in denen Ester Ib und Ic und Hydroxide eingesetzt werden, betragen 1:0,95 bis 1:1 für Alkalimetallhydroxide und 1:0,48 bis 1:0,55 für Erdalkalimetallhydroxide.

5. Verfahren zur Herstellung der Verbindungen Ia, Ib und Ic, in denen $R^5$ eine Gruppe $COYR^6$ bedeutet:

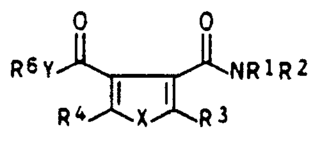
Ia

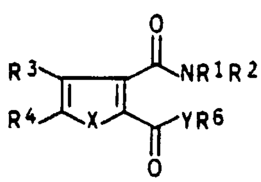
Ib

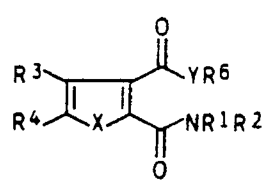
Ic

Man erhält diese Verbindungen beispielsweise, wenn man ein Carbonsäureamid Ia, Ib oder Ic, in dem $R^5$ eine Gruppe $CO_2H$ bedeutet in an sich bekannter Weise in oder eine aktivierte Form der Carbonsäure überführt und diese Derivate anschließend mit einer Verbindung VIII verestert.

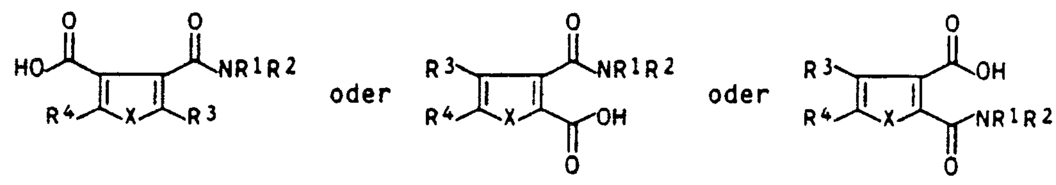

Ia oder Ib oder Ic

Ia oder Ib oder Ic

($R^5$ = $COYR^6$)

Diese Umsetzung wird üblicherweise bei Temperaturen von -20 bis 60°C, vorzugsweise 0 bis 40°C durchgeführt.

Als Lösungsmittel verwendet man zweckmäßigerweise Halogenkohlenwasserstoffe wie Chlorbenzol und 1,2-Dichlorbenzol, Ether, z.B. Methyl-tert.-butylether, 1,2-Dimethoxyethan, Diethylenglykol-dimethylether, Tetrahydrofuran und Dioxan; dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten, z.B. Benzol, Toluol und xylol. Die Konzentration der Edukte im Lösungsmittel beträgt im allgemeinen 0,1 bis 5,0 mol/l, bevorzugt 0,2 bis 2,0 mol/l.

In der Regel werden 1 bis 1,5 mol-äq., vorzugsweise 1 bis 1,15 mol-äq. der Verbindung V, bezogen auf die Carbonsäure Ia, Ib oder Ic ($R^5$ = $CO_2H$), eingesetzt.

Als wasserentziehendes Mittel eignen sich Diimide wie Dicyclohexylcarbodiimid (s.a. Angew. Chem. 90, 556 (1978) oder Anhydride wie Propanphosphonsäureanhydrid. Auch in Anwesenheit von 1-Methyl-2-halogenpyridiniumiodiden als wasserentziehendes Mittel (vgl. Chem. Lett., 1045 (1975); ibid., 13 (1976); ibid., 49 (1976) gelingt die Umsetzung.

Besonders bevorzugt arbeitet man in einem inerten Lösungsmittel wie Tetrahydrofuran, Dichlormethan oder Toluol in Gegenwart von Dicyclohexylcarbodiimid als wasserentziehendem Mittel beim Einsatz von Carbonsäure I, Verbindung IV und wasserentziehendem Mittel in stöchiometrischen Mengen bei 20 bis

40 °C.

Die Umsetzung ist im allgemeinen nach 14 Stunden beendet; die Carbonsäureamide Ia, Ib und Ic werden in an sich bekannter Weise (z.B. durch Verdünnen des Reaktionsgemisches mit Wasser und Extraktion des Produktes mit einem organischen Lösungsmittel) isoliert und mit üblichen Standardmethoden wie Umkristallisation oder Chromatographie gereinigt.

6. Verfahren zur Herstellung der Verbindungen Ia, Ib und Ic, in denen $R^5$ 4,5-Dihydrooxazol-2-yl bedeutet:

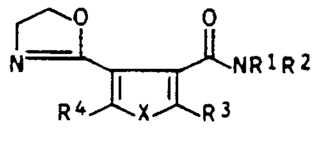
Ia

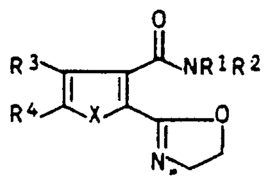
Ib

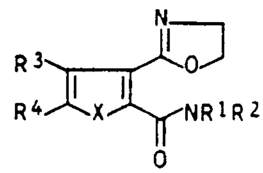
Ic

Man erhält diese Verbindungen Ia, Ib und Ic beispielsweise, indem man ein entsprechendes Dicarbonsäureamid Ia, Ib oder Ic, in dem $R^5$ eine Carboxylgruppe bedeutet in an sich bekannter Weise mit 2-Aminoethanol IX cyclisiert. Der Übersichtlichkeit halber ist diese Umsetzung im folgenden repräsentativ für die Verbindungen Ia beschrieben.

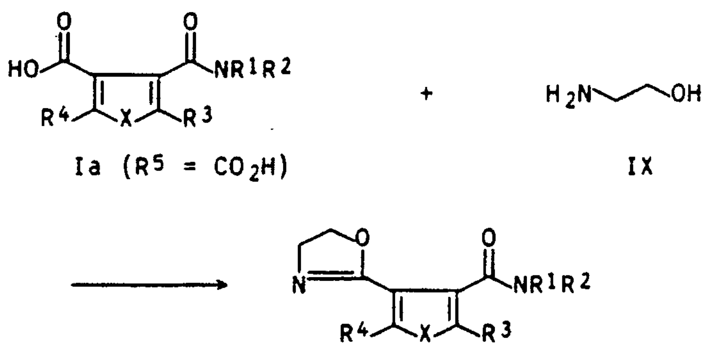
Ia ($R^5$ = $CO_2H$) + IX

Ia ($R^5$ = 4,5-Dihydroxazol-2-yl)

Die Reaktion wird so durchgeführt, daß man die Verbindungen bei 0 bis 180 °C, vorzugsweise bei Rückflußtemperatur des verwendeten Gemisches mit einem Aminoalkohol IX, gegebenenfalls in Gegenwart eines inerten Lösungsmittels umsetzt. Ester oder Carbonsäure Ia, Ib bzw. Ic und Aminoalkohol IX werden dabei im Verhältnis 1:1 bis 1:2,5, vorzugsweise 1:1 bis 1:1,5 eingesetzt.

Als Lösungsmittel verwendet man zweckmäßigerweise Halogenkohlenwasserstoffe wie Chlorbenzol und 1,2-Dichlorbenzol, Ether, z.B. Methyl-tert.-butylether, 1,2-Dimethoxyethan, Diethylenglykol-dimethylether, Tetrahydrofuran und Dioxan; Alkohole wie Methanol, Ethanol, Propanol oder Ethylenglykol, dipolare aprotische Lösungsmittel, z.B. Acetonitril, Dimethylformamid, Dimethylacetamid, Dimethylsulfoxid, N-Methylpyrrolidon, 1,3-Dimethyltetrahydro-2(1H)-pyrimidinon und 1,3-Dimethylimidazolin-2-on oder Aromaten, z.B. Benzol, Toluol und Xylol. Die Konzentration der Edukte im Lösungsmittel beträgt im allgemeinen 0,1 bis 5,0 mol/l, bevorzugt 0,2 bis 2,0 mol/l.

Die Umsetzung ist im allgemeinen nach 14 Stunden beendet; die Carbonsäureamide Ia und Ib werden dann gegebenenfalls durch Zugabe von Wasser ausgefällt, abgesaugt oder mit einem organischen Lösungsmittel extrahiert und mit üblichen Standardmethoden wie Umkristallisation oder Chromatographie gereinigt.

Neben den vorstehend geschilderten Verfahren zur Herstellung der Verbindungen Ia, Ib und Ic gibt es weitere Synthesemöglichkeiten, die den folgenden Literaturstellen zu entnehmen sind:

Beilstein, Hauptwerk sowie 1.-5. Erg.Werk, Band 27; R.W. Wiley, The Chemistry of Heterocyclic Compounds, Five- and Six-Membered Compounds with Nitrogen and Oxygen, Interscience Pubishers, New York, London (1962), Heterocyclic Chemistry, Vol. 6, Five-membered Rings with Two or More Oxygen,

Sulfur or Nitrogen Atoms, Programon Press, 1984, J. March, Advanced Organic Chemistry, Third Adition, John Wiley and Sons, 1985, Houben-Weyl, Methoden der organischen Chemie, 4. Auflage, Thieme Verlag, Bände IV, VI, VII, VIII, X.

Im Hinblick auf die bestimmungsgemäße Verwendung der Verbindungen IA, IB und IC kommen als Substituenten bevorzugt folgende Reste in Betracht:

$R^1$

Wasserstoff;

$C_3$-$C_8$-Cycloalkyl wie Cyclopropyl, Cyclobutyl, Cyclopentyl, Cyclohexyl, Cycloheptyl und Cyclooctyl, insbesondere Cyclopropyl, Cyclobutyl, Cyclopentyl und Cyclohexyl, welches ein bis drei der folgenden Reste tragen kann: Halogen wie Fluor, Chlor, Brom und Iod, insbesondere Fluor und Chlor; Alkyl wie Methyl, Ethyl, Propyl, 1-Methylethyl, Butyl, 1-Methylpropyl, 2-Methylpropyl und 1,1-Dimethylethyl, insbesondere Methyl und Ethyl; Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl; Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy und Ethoxy; Halogenalkoxy, wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;

Alkyl wie vorstehend genannt, sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, 1-Methylethyl und 1,1-Dimethylethyl, welches ein bis drei der folgenden Reste tragen kann: Hydroxy; Halogen wie vorstehend genannt, insbesondere Fluor und Chlor; Cycloalkyl wie vorstehend genannt, insbesondere Cyclopropyl; Alkoxy wie vorstehend genannt, insbesondere Methoxy und Ethoxy; Halogenalkoxy wie vorstehend genannt, insbesondere Trifluormethoxy; Alkylthio, wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio und Ethylthio; Halogenhalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio; Alkylamino wie Methylamino, Ethylamino, Propylamino, iso-Propylamino, insbesondere Methylamino; Dialkylamino wie Dimethylamino, Diethylamino, Dirpropylamino, Diisopropylamino, Methylethylamino, insbe sondere Dimethylamino; Cycloalkylamino wie Cyclopropylamino, Cyclobutylamino, Cyclopentylamino und Cyclohexylamino, insbesondere Cyclopropylamino; und/oder einen Rest

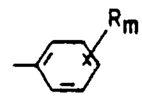

tragen kann, wobei

R Cyano; Nitro; Halogen wie insbesondere Fluor und Chlor; Alkyl wie insbesondere Methyl, Ethyl und 1-Methylethyl; Halogenalkyl wie insbesondere Trifluormethyl; Alkoxy wie insbesondere Methoxy, Ethoxy und 1-Methylethoxy; Halogenalkoxy wie insbesondere Difluormethoxy und Trifluormethoxy; Alkinyloxy wie Propargyloxy; Alkylthio wie insbesondere Methylthio und Ethylthio; Halogenalkylthio wie insbesondere Difluormethylthio und Trifluormethylthio, Alkoxycarbonylalkoxy wie insbesondere Methoxy- oder Ethoxycarbonylmethoxy und/oder Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl und 1,1-Dimethylethoxycarbonyl, insbesondere Methoxycarbonyl und Ethoxycarbonyl, bedeutet und

m für 0, 1, 2 oder 3 steht, wobei die Reste R verschieden sein können, wenn m 2 oder 3 bedeutet;

$R^2$

Hydroxy;

Alkoxy wie insbesondere Methoxy und Ethoxy;

Cyanalkyl wie Cyanmethyl, Cyanbutyl, 2-Cyan-3-methylbut-2-yl;

Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 3-Methyl-2-butenyl, 1-Methyl-3-butenyl, 2-Methyl-3-butenyl, 3-methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-but enyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl; Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl3-butinyl, 2,2-Dimethyl-3-butinyl, 1-Ethyl-2-butinyl, 1-Ethyl-3-butinyl, 2-Ethyl-3-butinyl und 1-Ethyl-1-Methyl-2-propinyl; insbesondere 2-Propenyl und 2-Propinyl, sowie Phenyl und Naphthyl, wobei diese Gruppen ein bis drei der bei R im allgemeinen und im besonderen genannten Reste tragen können;

einen 5- bis 6-gliedrigen Heterocyclus enthaltend ein oder zwei Heteroatome, ausgewählt aus der Gruppe Stickstoff, Sauerstoff und Schwefel, wie 2-Tetrahydrofuranyl, 3-Tetrahydrofuranyl, 2-Tetrahyrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 4-Pyrrolyl, 3-Pyrazolyl, 4-Pyrazolyl, 5-Pyrazolyl, 2-Pyridyl, 3-Pyridyl, 4-Pyridyl und 2-(4,6-Dimethyl-pyrimidinyl), wobei dieser Ring ein oder zwei der folgenden Reste tragen kann: Halogen, Alkyl, Halogenalkyl, Alkoxy, Halogenalkoxy und/oder Alkylthio wie im allgemeinen und im besonderen bei $R^1$ genannt;

eine der im allgemeinen oder im besonderen bei $R^1$ genannten Gruppen oder $R^1$ und $R^2$ gemeinsam

eine 4- bis 7-gliedrige Kette, welche neben Methylengruppen eine der folgenden Gruppen als Ringglied enthalten kann: Sauerstoff, Schwefel, $N(CH_3)$- oder -CO- wie $-(CH_2)_3-$, $-(CH_2)_4-$, $-(CH_2)_5-$, $(CH_2)_6-$, $-CH_2-O-CH_2-$, $-CH_2-CH_2-O-CH_2-CH_2-$, $-CH_2-S-CH_2-$, $-CH_2-CH_2-S-CH_2-CH_2-$, $-CH_2-CH_2-N(CH_3)-CH_2-CH_2$ und --$(CH_2)_3-CO-$, insbesondere $-(CH_2)_5-$ und $-CH_2-CH_2-O-CH_2-CH_2$;

$R^3$, $R^3$

Nitro; Cyano;

Halogen wie insbesondere Fluor, Chlor und Brom;

Amino, welches ein oder zwei $C_1$-$C_4$-Alkylgruppen wie bei $R^1$ genannt, insbesondere Methyl und Ethyl und/oder eine Alkylcarbonylgruppe wie Methylcarbonyl, Ethylcarbonyl, Propylcarbonyl, 1-Methylethylcarbonyl, Butylcarbonyl, 1-Methylpropylcarbonyl, 2-Methylpropylcarbonyl und 1,1-Dimethylethylcarbonyl, insbesondere Methylcarbonyl und Ethylcarbonyl tragen kann;

Alkoxy oder Alkylthio wie insbesondere Methoxy, Ethoxy, Methylthio und Ethylthio, wobei diese Gruppen ein bis neun Halogenatome wie insbesondere Fluor und Chlor tragen können;

einen 5- bis 6-gliedrigen Heterocyclus enthaltend ein oder zwei Heteroatome ausgewählt aus der Gruppe Sauerstoff, Schwefel und Stickstoff wie bei $R^2$ genannt, wobei dieser Ring ein oder zwei der folgenden Reste tragen kann: Halogen wie insbesondere Fluor und Chlor, Alkyl wie insbesondere Methyl, Halogenalkyl wie insbesondere Trifluormethyl und Chlordifluormethyl, Alkoxy wie insbesondere Methoxy und Ethoxy, Halogenalkoxy wie insbesondere Trifluormethoxy, Trichlormethoxy und Pentafluorethoxy und/oder Alkylthio wie insbesondere Methylthio;

Alkenyl wie Ethenyl, 1-Propenyl, 1-Methylethenyl, 1-Butenyl, 1-Methyl-1-propenyl, 2-Methyl-1-propenyl, 1-Pentenyl, 1-Methyl-1-butenyl, 2-Methyl-1-butenyl, 1,1-Dimethyl-1-propenyl, 1-Ethyl-1-propenyl, 1-Hexenyl, 1-Methyl-1-pentenyl, 2-Methyl-1-pentenyl, 3-Methyl-1-pentenyl, 4-Methyl-1-pentenyl, 1,2-Dimethyl-1-butenyl, 1,3-Dimethyl-1-butenyl, 2,3-Dimethyl-1-butenyl, 3,3-Dimethyl-1-butenyl, 1-Ethyl-1-butenyl, 2-Ethyl-1-butenyl, 1-Ethyl-2-methyl-1-propenyl;

insbesondere 2-Propenyl;

Alkinyl wie Ethinyl, 1-Propinyl, 1-Butinyl, 1-Pentinyl, 1-Methyl-3-butinyl, 1-Hexinyl, 3-Methyl-1-pentinyl, 4-Methyl-1-pentinyl, 3,3-Dimethyl-1-butinyl,

insbesondere 2-Propinyl oder Phenyl, wobei diese Gruppen ein bis drei der im allgemeinen und im besonderen bei R genannten Reste tragen können, oder eine der im allgemeinen und im besonderen bei $R^1$ genannten Gruppen;

$R^5$

Formyl; 4,5-Dihydrooxazol-2-yl oder eine Grupe $COYR^6$;

Y Sauerstoff oder Schwefel

$R^6$

Wasserstoff;

Cycloalkyl wie bei $R^1$ genannt, insbesondere Cyclopentyl und Cyclohexyl;

Alkyl wie bei $R^1$ genannt, insbesondere Methyl, Ethyl, Propyl, 1-Methyl-ethyl und Hexyl, welches ein bis fünf Halogenatome wie insbesondere Fluor und Chlor oder Hydroxygruppen und/oder einen der folgenden Reste tragen kann: Cyano, Aminocarbonyl, Carboxyl, Trimethylsilyl, Alkoxy wie insbesondere Methoxy und Ethoxy, Alkoxyalkoxy wie Methoxyethoxy, Ethoxyethoxy und Propyloxyethoxy, insbesondere Methoxyethoxy, Alkylthio wie insbesondere Methylthio und Ethylthio; Alkylamino wie insbesondere Methylamino und Ethylamino, Dialkylamino wie insbesondere Dimethylamino und Methylethylamino, Alkylsulfinyl wie Methylsulfinyl, Ethylsulfinyl, Propylsulfinyl, iso-Propylsulfinyl, insbesondere Methylsulfinyl und Ethylsulfinyl; Alkylsulfonyl wie Methylsulfonyl, Ethylsulfonyl, Propylsulfonyl und iso-Propylsulfonyl, insbesondere Methylsulfonyl und Ethylsulfonyl; Alkoxycarbonyl wie insbesondere Methoxycarbonyl, Alkoxycarbonylalkoxy wie Methoxycarbonylmethoxy, Methoxycarbonylethoxy, Ethoxycarbonylethoxy; Alkoxycarbonylalkoxycarbonyl wie Methoxycarbonylmethoxycarbonyl, Methoxycarbonylethoxycarbonyl, Ethoxycarbonylethoxycarbonyl; Alkylaminocarbonyl wie Methylaminocarbonyl, Ethylaminocarbonyl, Propylaminocarbonyl und iso-Propylaminocarbonyl, insbesondere Methylaminocarbonyl und Ethylaminocarbonyl, Dialkylaminocarbonyl wie Dimethylaminocarbonyl, Diethylaminocarbonyl, Dipropylaminocarbonyl, Diisopropylaminocarbonyl, Dicyclopropylaminocarbonyl und Methylethylaminocarbonyl, insbesondere Dimethylaminocarbonyl und Diethylaminocarbonyl; Dialkoxyphosphonyl wie Dimethoxyphosphonyl, Diethoxyphosphonyl, Dipropoxyphosphonyl und Diisopropoxyphosphonyl, insbesondere Dimethoxyphosphonyl und Diethoxyphosphonyl; Alkaminoxy wie insbesondere 2-Propaniminoxy; Phenyl, Thienyl, Benzyloxy, Benzylthio, Furyl, Tetrahydrofuryl, Phthalimido und/oder Benzoyl, wobei die cyclischen Reste ihrerseits ein bis drei der im allgemeinen und im besonderen bei R genannten Reste tragen können;

Alkenyl wie insbesondere 2-Propenyl und 2-Butenyl, Alkinyl wie insbesondere 2-Propinyl oder Cycloalkenyl wie insbesondere 2-Cyclopentenyl und 2-Cyclohexenyl, wobei diese Gruppen einen der folgenden Reste tragen können: Hydroxy, Halogen wie insbesondere Fluor und Chlor, Alkoxy wie insbesondere Methoxy und Ethoxy, oder Phenyl, wobei der Phenylrest seinerseits ein bis drei der im allgemeinen und im besonderen bei R genannten Reste tragen kann;

einen 5- bis 6-gliedrigen Heterocyclus enthaltend ein oder zwei Heteroatcme ausgewählt aus der Gruppe Stickstoff, Sauerstoff und Schwefel wie bei $R^2$ genannt, insbesondere Tetrahydrofuranyl und Tetrahydropyranyl;

Phthalimido; Tetrahydrophthalimido; Succinimido; Maleinimido; Benzotriazolyl;

Phenyl, welches ein bis drei der im allgemeinen und im besonderen bei R genannten Reste tragen kann;

eine Gruppe $N=CR^7R^8$ wobei

$R^7$

Wasserstoff oder Alkyl wie bei $R^1$ genannt, insbesondere Methyl, Ethyl und 1-Methylethyl und

$R^8$

Cycloalkyl wie insbesondere Cyclopropyl, Phenyl, Furyl oder eine der bei $R^7$ genannten Gruppen bedeutet oder

$R^7$, $R^8$

gemeinsam eine Alkylenkette wie Butylen, Pentylen, Hexylen und Heptylen, insbesondere Butylen und Pentylen bilden.

Tabelle

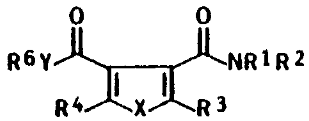

Ia/IA

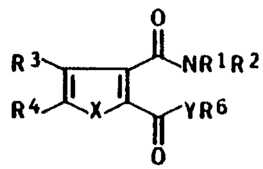

Ib/IB

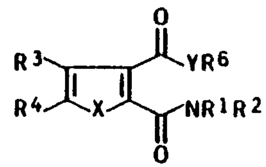

Ic/IC

| $R^3$ | $R^4$ | $R^1$ | $R^2$ | Y | $R^6$ | X = O oder S |
|---|---|---|---|---|---|---|
| H | F | H | Methyl | O | H | |
| H | F | H | Ethyl | O | H | |
| H | F | H | n-Propyl | O | H | |
| H | F | H | iso-Propyl | O | H | |
| H | F | H | Cyclopropyl | O | H | |
| H | F | H | n-Butyl | O | H | |
| H | F | H | iso-Butyl | O | H | |
| H | F | H | sek.-Butyl | O | H | |
| H | F | H | tert.-Butyl | O | H | |
| H | F | H | n-Pentyl | O | H | |
| H | F | H | 2-Pentyl | O | H | |
| H | F | H | 3-Pentyl | O | H | |
| H | F | H | n-Hexyl | O | H | |
| H | F | H | 2-Hexyl | O | H | |
| H | F | H | 3-Hexyl | O | H | |
| H | F | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | F | H | cyclo-Propylmethyl | O | H | |
| H | F | H | cyclo-Butyl | O | H | |
| H | F | H | cyclo-Pentyl | O | H | |
| H | F | H | cyclo-Hexyl | O | H | |
| H | F | H | 1-Methylcyclohexyl | O | H | |
| H | F | H | 3-Triflormethylcyclohexyl | O | H | |
| H | F | H | Allyl | O | H | |
| H | F | H | 1-Buten-3-yl | O | H | |
| H | F | H | Crotyl | O | H | |
| H | F | H | Propargyl | O | H | |
| H | F | H | 1-Butin-3-yl | O | H | |
| H | F | H | 3-Methyl-1-butin-3-yl | O | H | |
| H | F | H | 2-Pentin-4-yl | O | H | |
| H | F | H | Benzyl | O | H | |
| H | F | H | 2-Phenylethyl | O | H | |
| H | F | H | 2-Methylthioethyl | O | H | |
| H | F | H | 2-Chlorethyl | O | H | |
| H | F | H | 2-Methoxyethyl | O | H | |
| H | F | H | 2-(N,N-Dimethylamino)ethyl | O | H | |
| H | F | H | Phenyl | O | H | |
| H | F | H | 2-$CH_3$-Phenyl | O | H | |
| H | F | H | 3-$CH_3$-Phenyl | O | H | |
| H | F | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | F | H | 2,4-($CH_3$,$CH_3$)-Phenyl | O | H | |
| H | F | H | 2,3,5-($CH_3$,$CH_3$,$CH_3$)-Phenyl | O | H | |
| H | F | H | 3-$CF_3$-Phenyl | O | H | |
| H | F | H | 3-F-Phenyl | O | H | |
| H | F | H | 2-Cl-Phenyl | O | H | |
| H | F | H | 4-Cl-Phenyl | O | H | |
| H | F | H | 2,4-(F,F)-Phenyl | O | H | |
| H | F | H | 2,3,5-(Cl,Cl,Cl)-Phenyl | O | H | |
| H | F | H | 2-CN-Phenyl | O | H | |
| H | F | H | 2-$OCH_3$-Phenyl | O | H | |
| H | F | H | 2,3-($OCH_3$,$OCH_3$)-Phenyl | O | H | |
| H | F | H | 3,4,5-($OCH_3$,$OCH_3$,$OCH_3$)-Phenyl | O | H | |
| H | F | H | 3-$OCF_3$-Phenyl | O | H | |
| H | F | H | 3-$OCF_2OHF_2$-Phenyl | O | H | |
| H | F | H | 4-$OCF_2CHF_2$-Phenyl | O | H | |
| H | F | H | 2-$SCH_3$-Phenyl | O | H | |
| H | F | H | 2,4-($SCH_3$,$SCH_3$)-Phenyl | O | H | |
| H | F | H | 2-$SCF_3$-Phenyl | O | H | |
| H | F | H | 4-$NO_2$-Phenyl | O | H | |
| H | F | H | 2,4-($NO_2$,$NO_2$)-Phenyl | O | H | |
| H | F | H | 3-$COCH_3$-Phenyl | O | H | |
| H | F | H | 3-$COCF_3$-Phenyl | O | H | |
| H | F | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | F | H | 2-Naphthyl | O | H | |
| H | F | H | Piperidino | O | H | |
| H | F | H | 3-Tetrahydrofuranyl | O | H | |
| H | F | H | 4-Tetrahydropyranyl | O | H | |
| H | F | H | 2-Thiazolyl | O | H | |
| H | F | H | 5-$CH_3$-2-Thiazolyl | O | H | |
| H | F | H | 4-$CH_3$-5-COOH-2-Thiazolyl | O | H | |
| H | Cl | H | Methyl | O | H | |
| H | Cl | H | Ethyl | O | H | |
| H | Cl | H | n-Propyl | O | H | |
| H | Cl | H | iso-Propyl | O | H | |
| H | Cl | H | Cyclopropyl | O | H | |
| H | Cl | H | n-Butyl | O | H | |
| H | Cl | H | iso-Butyl | O | H | |
| H | Cl | H | sek.-Butyl | O | H | |
| H | Cl | H | tert.-Butyl | O | H | |
| H | Cl | H | n-Pentyl | O | H | |
| H | Cl | H | 2-Pentyl | O | H | |
| H | Cl | H | 3-Pentyl | O | H | |
| H | Cl | H | n-Hexyl | O | H | |
| H | Cl | H | 2-Hexyl | O | H | |
| H | Cl | H | 3-Hexyl | O | H | |
| H | Cl | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | cyclo-Propylmethyl | O | H | |
| H | Cl | H | cyclo-Butyl | O | H | |
| H | Cl | H | cyclo-Pentyl | O | H | |
| H | Cl | H | cyclo-Hexyl | O | H | |
| H | Cl | H | 1-Methylcyclohexyl | O | H | |
| H | Cl | H | 3-Triflormethylcyclohexyl | O | H | |
| H | Cl | H | Allyl | O | H | |
| H | Cl | H | 1-Buten-3-yl | O | H | |
| H | Cl | H | Crotyl | O | H | |
| H | Cl | H | Propargyl | O | H | |
| H | Cl | H | 1-Butin-3-yl | O | H | |
| H | Cl | H | 3-Methyl-1-butin-3-yl | O | H | |
| H | Cl | H | 2-Pentin-4-yl | O | H | |
| H | Cl | H | Benzyl | O | H | |
| H | Cl | H | 2-Phenylethyl | O | H | |
| H | Cl | H | 2-Methylthioethyl | O | H | |
| H | Cl | H | 2-Chlorethyl | O | H | |
| H | Cl | H | 2-Methoxyethyl | O | H | |
| H | Cl | H | 2-(N,N-Dimethylamino)ethyl | O | H | |
| H | Cl | H | Phenyl | O | H | |
| H | Cl | H | 2-$CH_3$-Phenyl | O | H | |
| H | Cl | H | 3-$CH_3$-Phenyl | O | H | |
| H | Cl | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2,4-($CH_3$,$CH_3$)-Phenyl | O | H | |
| H | Cl | H | 2,3,5-($CH_3$,$CH_3$,$CH_3$)-Phenyl | O | H | |
| H | Cl | H | 3-$CF_3$-Phenyl | O | H | |
| H | Cl | H | 3-F-Phenyl | O | H | |
| H | Cl | H | 2-Cl-Phenyl | O | H | |
| H | Cl | H | 4-Cl-Phenyl | O | H | |
| H | Cl | H | 2,4-(F,F)-Phenyl | O | H | |
| H | Cl | H | 2,3,5-(Cl,Cl,Cl)-Phenyl | O | H | |
| H | Cl | H | 2-CN-Phenyl | O | H | |
| H | Cl | H | 2-$OCH_3$-Phenyl | O | H | |
| H | Cl | H | 2,3-($OCH_3$,$OCH_3$)-Phenyl | O | H | |
| H | Cl | H | 3,4,5-($OCH_3$,$OCH_3$,$OCH_3$)-Phenyl | O | H | |
| H | Cl | H | 3-$OCF_3$-Phenyl | O | H | |
| H | Cl | H | 3-$OCF_2OHF_2$-Phenyl | O | H | |
| H | Cl | H | 4-$OCF_2CHF_2$-Phenyl | O | H | |
| H | Cl | H | 2-$SCH_3$-Phenyl | O | H | |
| H | Cl | H | 2,4-($SCH_3$,$SCH_3$)-Phenyl | O | H | |
| H | Cl | H | 2-$SCF_3$-Phenyl | O | H | |
| H | Cl | H | 4-$NO_2$-Phenyl | O | H | |
| H | Cl | H | 2,4-($NO_2$,$NO_2$)-Phenyl | O | H | |
| H | Cl | H | 3-$COCH_3$-Phenyl | O | H | |
| H | Cl | H | 3-$COCF_3$-Phenyl | O | H | |
| H | Cl | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2-Naphthyl | O | H | |
| H | Cl | H | Piperidino | O | H | |
| H | Cl | H | 3-Tetrahydrofuranyl | O | H | |
| H | Cl | H | 4-Tetrahydropyranyl | O | H | |
| H | Cl | H | 2-Thiazolyl | O | H | |
| H | Cl | H | 5-$CH_3$-2-Thiazolyl | O | H | |
| H | Cl | H | 4-$CH_3$-5-COOH-2-Thiazolyl | O | H | |
| H | Br | H | Methyl | O | H | |
| H | Br | H | Ethyl | O | H | |
| H | Br | H | n-Propyl | O | H | |
| H | Br | H | iso-Propyl | O | H | |
| H | Br | H | Cyclopropyl | O | H | |
| H | Br | H | n-Butyl | O | H | |
| H | Br | H | iso-Butyl | O | H | |
| H | Br | H | sek.-Butyl | O | H | |
| H | Br | H | tert.-Butyl | O | H | |
| H | Br | H | n-Pentyl | O | H | |
| H | Br | H | 2-Pentyl | O | H | |
| H | Br | H | 3-Pentyl | O | H | |
| H | Br | H | n-Hexyl | O | H | |
| H | Br | H | 2-Hexyl | O | H | |
| H | Br | H | 3-Hexyl | O | H | |
| H | Br | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Br | H | cyclo-Propylmethyl | O | H | |
| H | Br | H | cyclo-Butyl | O | H | |
| H | Br | H | cyclo-Pentyl | O | H | |
| H | Br | H | cyclo-Hexyl | O | H | |
| H | Br | H | 1-Methylcyclohexyl | O | H | |
| H | Br | H | 3-Triflormethylcyclohexyl | O | H | |
| H | Br | H | Allyl | O | H | |
| H | Br | H | 1-Buten-3-yl | O | H | |
| H | Br | H | Crotyl | O | H | |
| H | Br | H | Propargyl | O | H | |
| H | Br | H | 1-Butin-3-yl | O | H | |
| H | Br | H | 3-Methyl-1-butin-3-yl | O | H | |
| H | Br | H | 2-Pentin-4-yl | O | H | |
| H | Br | H | Benzyl | O | H | |
| H | Br | H | 2-Phenylethyl | O | H | |
| H | Br | H | 2-Methylthioethyl | O | H | |
| H | Br | H | 2-Chlorethyl | O | H | |
| H | Br | H | 2-Methoxyethyl | O | H | |
| H | Br | H | 2-(N,N-Dimethylamino)ethyl | O | H | |
| H | Br | H | Phenyl | O | H | |
| H | Br | H | 2-$CH_3$-Phenyl | O | H | |
| H | Br | H | 3-$CH_3$-Phenyl | O | H | |
| H | Br | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Br | H | 2,4-($CH_3$,$CH_3$)-Phenyl | O | H | |
| H | Br | H | 2,3,5-($CH_3$,$CH_3$,$CH_3$)-Phenyl | O | H | |
| H | Br | H | 3-$CF_3$-Phenyl | O | H | |
| H | Br | H | 3-F-Phenyl | O | H | |
| H | Br | H | 2-Cl-Phenyl | O | H | |
| H | Br | H | 4-Cl-Phenyl | O | H | |
| H | Br | H | 2,4-(F,F)-Phenyl | O | H | |
| H | Br | H | 2,3,5-(Cl,Cl,Cl)-Phenyl | O | H | |
| H | Br | H | 2-CN-Phenyl | O | H | |
| H | Br | H | 2-$OCH_3$-Phenyl | O | H | |
| H | Br | H | 2,3-($OCH_3$,$OCH_3$)-Phenyl | O | H | |
| H | Br | H | 3,4,5-($OCH_3$,$OCH_3$,$OCH_3$)-Phenyl | O | H | |
| H | Br | H | 3-$OCF_3$-Phenyl | O | H | |
| H | Br | H | 3-$OCF_2OHF_2$-Phenyl | O | H | |
| H | Br | H | 4-$OCF_2CHF_2$-Phenyl | O | H | |
| H | Br | H | 2-$SCH_3$-Phenyl | O | H | |
| H | Br | H | 2,4-($SCH_3$,$SCH_3$)-Phenyl | O | H | |
| H | Br | H | 2-$SCF_3$-Phenyl | O | H | |
| H | Br | H | 4-$NO_2$-Phenyl | O | H | |
| H | Br | H | 2,4-($NO_2$,$NO_2$)-Phenyl | O | H | |
| H | Br | H | 3-$COCH_3$-Phenyl | O | H | |
| H | Br | H | 3-$COCF_3$-Phenyl | O | H | |
| H | Br | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | Br | H | 2-Naphthyl | O | H | |
| H | Br | H | Piperidino | O | H | |
| H | Br | H | 3-Tetrahydrofuranyl | O | H | |
| H | Br | H | 4-Tetrahydropyranyl | O | H | |
| H | Br | H | 2-Thiazolyl | O | H | |
| H | Br | H | 5-$CH_3$-2-Thiazolyl | O | H | |
| H | Br | H | 4-$CH_3$-5-COOH-2-Thiazolyl | O | H | |
| H | J | H | Methyl | O | H | |
| H | J | H | Ethyl | O | H | |
| H | J | H | n-Propyl | O | H | |
| H | J | H | iso-Propyl | O | H | |
| H | J | H | Cyclopropyl | O | H | |
| H | J | H | n-Butyl | O | H | |
| H | J | H | iso-Butyl | O | H | |
| H | J | H | sek.-Butyl | O | H | |
| H | J | H | tert.-Butyl | O | H | |
| H | J | H | n-Pentyl | O | H | |
| H | J | H | 2-Pentyl | O | H | |
| H | J | H | 3-Pentyl | O | H | |
| H | J | H | n-Hexyl | O | H | |
| H | J | H | 2-Hexyl | O | H | |
| H | J | H | 3-Hexyl | O | H | |
| H | J | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | J | H | cyclo-Propylmethyl | O | H | |
| H | J | H | cyclo-Butyl | O | H | |
| H | J | H | cyclo-Pentyl | O | H | |
| H | J | H | cyclo-Hexyl | O | H | |
| H | J | H | 1-Methylcyclohexyl | O | H | |
| H | J | H | 3-Triflormethylcyclohexyl | O | H | |
| H | J | H | Allyl | O | H | |
| H | J | H | 1-Buten-3-yl | O | H | |
| H | J | H | Crotyl | O | H | |
| H | J | H | Propargyl | O | H | |
| H | J | H | 1-Butin-3-yl | O | H | |
| H | J | H | 3-Methyl-1-butin-3-yl | O | H | |
| H | J | H | 2-Pentin-4-yl | O | H | |
| H | J | H | Benzyl | O | H | |
| H | J | H | 2-Phenylethyl | O | H | |
| H | J | H | 2-Methylthioethyl | O | H | |
| H | J | H | 2-Chlorethyl | O | H | |
| H | J | H | 2-Methoxyethyl | O | H | |
| H | J | H | 2-(N,N-Dimethylamino)ethyl | O | H | |
| H | J | H | Phenyl | O | H | |
| H | J | H | 2-$CH_3$-Phenyl | O | H | |
| H | J | H | 3-$CH_3$-Phenyl | O | H | |
| H | J | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | J | H | 2,4-($CH_3$,$CH_3$)-Phenyl | O | H | |
| H | J | H | 2,3,5-($CH_3$,$CH_3$,$CH_3$)-Phenyl | O | H | |
| H | J | H | 3-$CF_3$-Phenyl | O | H | |
| H | J | H | 3-F-Phenyl | O | H | |
| H | J | H | 2-Cl-Phenyl | O | H | |
| H | J | H | 4-Cl-Phenyl | O | H | |
| H | J | H | 2,4-(F,F)-Phenyl | O | H | |
| H | J | H | 2,3,5-(Cl,Cl,Cl)-Phenyl | O | H | |
| H | J | H | 2-CN-Phenyl | O | H | |
| H | J | H | 2-$OCH_3$-Phenyl | O | H | |
| H | J | H | 2,3-($OCH_3$,$OCH_3$)-Phenyl | O | H | |
| H | J | H | 3,4,5-($OCH_3$,$OCH_3$,$OCH_3$)-Phenyl | O | H | |
| H | J | H | 3-$OCF_3$-Phenyl | O | H | |
| H | J | H | 3-$OCF_2OHF_2$-Phenyl | O | H | |
| H | J | H | 4-$OCF_2CHF_2$-Phenyl | O | H | |
| H | J | H | 2-$SCH_3$-Phenyl | O | H | |
| H | J | H | 2,4-($SCH_3$,$SCH_3$)-Phenyl | O | H | |
| H | J | H | 2-$SCF_3$-Phenyl | O | H | |
| H | J | H | 4-$NO_2$-Phenyl | O | H | |
| H | J | H | 2,4-($NO_2$,$NO_2$)-Phenyl | O | H | |
| H | J | H | 3-$COCH_3$-Phenyl | O | H | |
| H | J | H | 3-$COCF_3$-Phenyl | O | H | |
| H | J | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | J | H | 2-Naphthyl | O | H | |
| H | J | H | Piperidino | O | H | |
| H | J | H | 3-Tetrahydrofuranyl | O | H | |
| H | J | H | 4-Tetrahydropyranyl | O | H | |
| H | J | H | 2-Thiazolyl | O | H | |
| H | J | H | 5-$CH_3$-2-Thiazolyl | O | H | |
| H | J | H | 4-$CH_3$-5-COOH-2-Thiazolyl | O | H | |
| F | H | H | Methyl | O | H | |
| F | H | H | Ethyl | O | H | |
| F | H | H | n-Propyl | O | H | |
| F | H | H | iso-Propyl | O | H | |
| F | H | H | Cyclopropyl | O | H | |
| F | H | H | n-Butyl | O | H | |
| F | H | H | iso-Butyl | O | H | |
| F | H | H | sek.-Butyl | O | H | |
| F | H | H | tert.-Butyl | O | H | |
| F | H | H | n-Pentyl | O | H | |
| F | H | H | 2-Pentyl | O | H | |
| F | H | H | 3-Pentyl | O | H | |
| F | H | H | n-Hexyl | O | H | |
| F | H | H | 2-Hexyl | O | H | |
| F | H | H | 3-Hexyl | O | H | |
| F | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| F | H | H | cyclo-Propylmethyl | O | H | |
| F | H | H | cyclo-Butyl | O | H | |
| F | H | H | cyclo-Pentyl | O | H | |
| F | H | H | cyclo-Hexyl | O | H | |
| F | H | H | 1-Methylcyclohexyl | O | H | |
| F | H | H | 3-Triflormethylcyclohexyl | O | H | |
| F | H | H | Allyl | O | H | |
| F | H | H | 1-Buten-3-yl | O | H | |
| F | H | H | Crotyl | O | H | |
| F | H | H | Propargyl | O | H | |
| F | H | H | 1-Butin-3-yl | O | H | |
| F | H | H | 3-Methyl-1-butin-3-yl | O | H | |
| F | H | H | 2-Pentin-4-yl | O | H | |
| F | H | H | Benzyl | O | H | |
| F | H | H | 2-Phenylethyl | O | H | |
| F | H | H | 2-Methylthioethyl | O | H | |
| F | H | H | 2-Chlorethyl | O | H | |
| F | H | H | 2-Methoxyethyl | O | H | |
| F | H | H | 2-(N,N-Dimethylamino)ethyl | O | H | |
| F | H | H | Phenyl | O | H | |
| F | H | H | 2-$CH_3$-Phenyl | O | H | |
| F | H | H | 3-$CH_3$-Phenyl | O | H | |
| F | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| F | H | H | 2,4-($CH_3$,$CH_3$)-Phenyl | O | H | |
| F | H | H | 2,3,5-($CH_3$,$CH_3$,$CH_3$)-Phenyl | O | H | |
| F | H | H | 3-$CF_3$-Phenyl | O | H | |
| F | H | H | 3-F-Phenyl | O | H | |
| F | H | H | 2-Cl-Phenyl | O | H | |
| F | H | H | 4-Cl-Phenyl | O | H | |
| F | H | H | 2,4-(F,F)-Phenyl | O | H | |
| F | H | H | 2,3,5-(Cl,Cl,Cl)-Phenyl | O | H | |
| F | H | H | 2-CN-Phenyl | O | H | |
| F | H | H | 2-$OCH_3$-Phenyl | O | H | |
| F | H | H | 2,3-($OCH_3$,$OCH_3$)-Phenyl | O | H | |
| F | H | H | 3,4,5-($OCH_3$,$OCH_3$,$OCH_3$)-Phenyl | O | H | |
| F | H | H | 3-$OCF_3$-Phenyl | O | H | |
| F | H | H | 3-$OCF_2OHF_2$-Phenyl | O | H | |
| F | H | H | 4-$OCF_2CHF_2$-Phenyl | O | H | |
| F | H | H | 2-$SCH_3$-Phenyl | O | H | |
| F | H | H | 2,4-($SCH_3$,$SCH_3$)-Phenyl | O | H | |
| F | H | H | 2-$SCF_3$-Phenyl | O | H | |
| F | H | H | 4-$NO_2$-Phenyl | O | H | |
| F | H | H | 2,4-($NO_2$,$NO_2$)-Phenyl | O | H | |
| F | H | H | 3-$COCH_3$-Phenyl | O | H | |
| F | H | H | 3-$COCF_3$-Phenyl | O | H | |
| F | H | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| F | H | H | 2-Naphthyl | O | H | |
| F | H | H | Piperidino | O | H | |
| F | H | H | 3-Tetrahydrofuranyl | O | H | |
| F | H | H | 4-Tetrahydropyranyl | O | H | |
| F | H | H | 2-Thiazolyl | O | H | |
| F | H | H | 5-$CH_3$-2-Thiazolyl | O | H | |
| F | H | H | 4-$CH_3$-5-COOH-2-Thiazolyl | O | H | |
| Cl | H | H | Methyl | O | H | |
| Cl | H | H | Ethyl | O | H | |
| Cl | H | H | n-Propyl | O | H | |
| Cl | H | H | iso-Propyl | O | H | |
| Cl | H | H | Cyclopropyl | O | H | |
| Cl | H | H | n-Butyl | O | H | |
| Cl | H | H | iso-Butyl | O | H | |
| Cl | H | H | sek.-Butyl | O | H | |
| Cl | H | H | tert.-Butyl | O | H | |
| Cl | H | H | n-Pentyl | O | H | |
| Cl | H | H | 2-Pentyl | O | H | |
| Cl | H | H | 3-Pentyl | O | H | |
| Cl | H | H | n-Hexyl | O | H | |
| Cl | H | H | 2-Hexyl | O | H | |
| Cl | H | H | 3-Hexyl | O | H | |
| Cl | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | H | H | cyclo-Propylmethyl | O | H | |
| Cl | H | H | cyclo-Butyl | O | H | |
| Cl | H | H | cyclo-Pentyl | O | H | |
| Cl | H | H | cyclo-Hexyl | O | H | |
| Cl | H | H | 1-Methylcyclohexyl | O | H | |
| Cl | H | H | 3-Triflormethylcyclohexyl | O | H | |
| Cl | H | H | Allyl | O | H | |
| Cl | H | H | 1-Buten-3-yl | O | H | |
| Cl | H | H | Crotyl | O | H | |
| Cl | H | H | Propargyl | O | H | |
| Cl | H | H | 1-Butin-3-yl | O | H | |
| Cl | H | H | 3-Methyl-1-butin-3-yl | O | H | |
| Cl | H | H | 2-Pentin-4-yl | O | H | |
| Cl | H | H | Benzyl | O | H | |
| Cl | H | H | 2-Phenylethyl | O | H | |
| Cl | H | H | 2-Methylthioethyl | O | H | |
| Cl | H | H | 2-Chlorethyl | O | H | |
| Cl | H | H | 2-Methoxyethyl | O | H | |
| Cl | H | H | 2-(N,N-Dimethylamino)ethyl | O | H | |
| Cl | H | H | Phenyl | O | H | |
| Cl | H | H | 2-$CH_3$-Phenyl | O | H | |
| Cl | H | H | 3-$CH_3$-Phenyl | O | H | |
| Cl | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | H | H | 2,4-($CH_3$,$CH_3$)-Phenyl | O | H | |
| Cl | H | H | 2,3,5-($CH_3$,$CH_3$,$CH_3$)-Phenyl | O | H | |
| Cl | H | H | 3-$CF_3$-Phenyl | O | H | |
| Cl | H | H | 3-F-Phenyl | O | H | |
| Cl | H | H | 2-Cl-Phenyl | O | H | |
| Cl | H | H | 4-Cl-Phenyl | O | H | |
| Cl | H | H | 2,4-(F,F)-Phenyl | O | H | |
| Cl | H | H | 2,3,5-(Cl,Cl,Cl)-Phenyl | O | H | |
| Cl | H | H | 2-CN-Phenyl | O | H | |
| Cl | H | H | 2-$OCH_3$-Phenyl | O | H | |
| Cl | H | H | 2,3-($OCH_3$,$OCH_3$)-Phenyl | O | H | |
| Cl | H | H | 3,4,5-($OCH_3$,$OCH_3$,$OCH_3$)-Phenyl | O | H | |
| Cl | H | H | 3-$OCF_3$-Phenyl | O | H | |
| Cl | H | H | 3-$OCF_2OHF_2$-Phenyl | O | H | |
| Cl | H | H | 4-$OCF_2CHF_2$-Phenyl | O | H | |
| Cl | H | H | 2-$SCH_3$-Phenyl | O | H | |
| Cl | H | H | 2,4-($SCH_3$,$SCH_3$)-Phenyl | O | H | |
| Cl | H | H | 2-$SCF_3$-Phenyl | O | H | |
| Cl | H | H | 4-$NO_2$-Phenyl | O | H | |
| Cl | H | H | 2,4-($NO_2$,$NO_2$)-Phenyl | O | H | |
| Cl | H | H | 3-$COCH_3$-Phenyl | O | H | |
| Cl | H | H | 3-$COCF_3$-Phenyl | O | H | |
| Cl | H | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | H | H | 2-Naphthyl | O | H | |
| Cl | H | H | Piperidino | O | H | |
| Cl | H | H | 3-Tetrahydrofuranyl | O | H | |
| Cl | H | H | 4-Tetrahydropyranyl | O | H | |
| Cl | H | H | 2-Thiazolyl | O | H | |
| Cl | H | H | 5-$CH_3$-2-Thiazolyl | O | H | |
| Cl | H | H | 4-$CH_3$-5-COOH-2-Thiazolyl | O | H | |
| Br | H | H | Methyl | O | H | |
| Br | H | H | Ethyl | O | H | |
| Br | H | H | n-Propyl | O | H | |
| Br | H | H | iso-Propyl | O | H | |
| Br | H | H | Cyclopropyl | O | H | |
| Br | H | H | n-Butyl | O | H | |
| Br | H | H | iso-Butyl | O | H | |
| Br | H | H | sek.-Butyl | O | H | |
| Br | H | H | tert.-Butyl | O | H | |
| Br | H | H | n-Pentyl | O | H | |
| Br | H | H | 2-Pentyl | O | H | |
| Br | H | H | 3-Pentyl | O | H | |
| Br | H | H | n-Hexyl | O | H | |
| Br | H | H | 2-Hexyl | O | H | |
| Br | H | H | 3-Hexyl | O | H | |
| Br | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | H | H | cyclo-Propylmethyl | O | H | |
| Br | H | H | cyclo-Butyl | O | H | |
| Br | H | H | cyclo-Pentyl | O | H | |
| Br | H | H | cyclo-Hexyl | O | H | |
| Br | H | H | 1-Methylcyclohexyl | O | H | |
| Br | H | H | 3-Triflormethylcyclohexyl | O | H | |
| Br | H | H | Allyl | O | H | |
| Br | H | H | 1-Buten-3-yl | O | H | |
| Br | H | H | Crotyl | O | H | |
| Br | H | H | Propargyl | O | H | |
| Br | H | H | 1-Butin-3-yl | O | H | |
| Br | H | H | 3-Methyl-1-butin-3-yl | O | H | |
| Br | H | H | 2-Pentin-4-yl | O | H | |
| Br | H | H | Benzyl | O | H | |
| Br | H | H | 2-Phenylethyl | O | H | |
| Br | H | H | 2-Methylthioethyl | O | H | |
| Br | H | H | 2-Chlorethyl | O | H | |
| Br | H | H | 2-Methoxyethyl | O | H | |
| Br | H | H | 2-(N,N-Dimethylamino)ethyl | O | H | |
| Br | H | H | Phenyl | O | H | |
| Br | H | H | 2-$CH_3$-Phenyl | O | H | |
| Br | H | H | 3-$CH_3$-Phenyl | O | H | |
| Br | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | H | H | 2,4-($CH_3$,$CH_3$)-Phenyl | O | H | |
| Br | H | H | 2,3,5-($CH_3$,$CH_3$,$CH_3$)-Phenyl | O | H | |
| Br | H | H | 3-$CF_3$-Phenyl | O | H | |
| Br | H | H | 3-F-Phenyl | O | H | |
| Br | H | H | 2-Cl-Phenyl | O | H | |
| Br | H | H | 4-Cl-Phenyl | O | H | |
| Br | H | H | 2,4-(F,F)-Phenyl | O | H | |
| Br | H | H | 2,3,5-(Cl,Cl,Cl)-Phenyl | O | H | |
| Br | H | H | 2-CN-Phenyl | O | H | |
| Br | H | H | 2-$OCH_3$-Phenyl | O | H | |
| Br | H | H | 2,3-($OCH_3$,$OCH_3$)-Phenyl | O | H | |
| Br | H | H | 3,4,5-($OCH_3$,$OCH_3$,$OCH_3$)-Phenyl | O | H | |
| Br | H | H | 3-$OCF_3$-Phenyl | O | H | |
| Br | H | H | 3-$OCF_2OHF_2$-Phenyl | O | H | |
| Br | H | H | 4-$OCF_2CHF_2$-Phenyl | O | H | |
| Br | H | H | 2-$SCH_3$-Phenyl | O | H | |
| Br | H | H | 2,4-($SCH_3$,$SCH_3$)-Phenyl | O | H | |
| Br | H | H | 2-$SCF_3$-Phenyl | O | H | |
| Br | H | H | 4-$NO_2$-Phenyl | O | H | |
| Br | H | H | 2,4-($NO_2$,$NO_2$)-Phenyl | O | H | |
| Br | H | H | 3-$COCH_3$-Phenyl | O | H | |
| Br | H | H | 3-$COCF_3$-Phenyl | O | H | |
| Br | H | H | 1-Naphthyl | O | H | |

Tabelle (Fortsetzung)

| $R^3$ | $R^4$ | $R^1$ | $R^2$ | Y | $R^6$ | X = 0 oder S |
|---|---|---|---|---|---|---|
| Br | H | H | 2-Naphthyl | O | H | |
| Br | H | H | Piperidino | O | H | |
| Br | H | H | 3-Tetrahydrofuranyl | O | H | |
| Br | H | H | 4-Tetrahydropyranyl | O | H | |
| Br | H | H | 2-Thiazolyl | O | H | |
| Br | H | H | 5-$CH_3$-2-Thiazolyl | O | H | |
| Br | H | H | 4-$CH_3$-5-COOH-2-Thiazolyl | O | H | |
| Cl | Cl | H | Methyl | O | H | |
| Cl | Cl | H | Ethyl | O | H | |
| Cl | Cl | H | n-Propyl | O | H | |
| Cl | Cl | H | iso-Propyl | O | H | |
| Cl | Cl | H | Cyclopropyl | O | H | |
| Cl | Cl | H | n-Butyl | O | H | |
| Cl | Cl | H | iso-Butyl | O | H | |
| Cl | Cl | H | sek.-Butyl | O | H | |
| Cl | Cl | H | tert.-Butyl | O | H | |
| Cl | Cl | H | n-Pentyl | O | H | |
| Cl | Cl | H | 2-Pentyl | O | H | |
| Cl | Cl | H | 3-Pentyl | O | H | |
| Cl | Cl | H | n-Hexyl | O | H | |
| Cl | Cl | H | 2-Hexyl | O | H | |
| Cl | Cl | H | 3-Hexyl | O | H | |
| Cl | Cl | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Cl | H | cyclo-Propylmethyl | O | H | |
| Cl | Cl | H | cyclo-Butyl | O | H | |
| Cl | Cl | H | cyclo-Pentyl | O | H | |
| Cl | Cl | H | cyclo-Hexyl | O | H | |
| Cl | Cl | H | 1-Methylcyclohexyl | O | H | |
| Cl | Cl | H | 3-Triflormethylcyclohexyl | O | H | |
| Cl | Cl | H | Allyl | O | H | |
| Cl | Cl | H | 1-Buten-3-yl | O | H | |
| Cl | Cl | H | Crotyl | O | H | |
| Cl | Cl | H | Propargyl | O | H | |
| Cl | Cl | H | 1-Butin-3-yl | O | H | |
| Cl | Cl | H | 3-Methyl-1-butin-3-yl | O | H | |
| Cl | Cl | H | 2-Pentin-4-yl | O | H | |
| Cl | Cl | H | Benzyl | O | H | |
| Cl | Cl | H | 2-Phenylethyl | O | H | |
| Cl | Cl | H | 2-Methylthioethyl | O | H | |
| Cl | Cl | H | 2-Chlorethyl | O | H | |
| Cl | Cl | H | 2-Methoxyethyl | O | H | |
| Cl | Cl | H | 2-(N,N-Dimethylamino)ethyl | O | H | |
| Cl | Cl | H | Phenyl | O | H | |
| Cl | Cl | H | 2-$CH_3$-Phenyl | O | H | |
| Cl | Cl | H | 3-$CH_3$-Phenyl | O | H | |
| Cl | Cl | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Cl | H | 2,4-($CH_3$,$CH_3$)-Phenyl | O | H | |
| Cl | Cl | H | 2,3,5-($CH_3$,$CH_3$,$CH_3$)-Phenyl | O | H | |
| Cl | Cl | H | 3-$CF_3$-Phenyl | O | H | |
| Cl | Cl | H | 3-F-Phenyl | O | H | |
| Cl | Cl | H | 2-Cl-Phenyl | O | H | |
| Cl | Cl | H | 4-Cl-Phenyl | O | H | |
| Cl | Cl | H | 2,4-(F,F)-Phenyl | O | H | |
| Cl | Cl | H | 2,3,5-(Cl,Cl,Cl)-Phenyl | O | H | |
| Cl | Cl | H | 2-CN-Phenyl | O | H | |
| Cl | Cl | H | 2-$OCH_3$-Phenyl | O | H | |
| Cl | Cl | H | 2,3-($OCH_3$,$OCH_3$)-Phenyl | O | H | |
| Cl | Cl | H | 3,4,5-($OCH_3$,$OCH_3$,$OCH_3$)-Phenyl | O | H | |
| Cl | Cl | H | 3-$OCF_3$-Phenyl | O | H | |
| Cl | Cl | H | 3-$OCF_2OHF_2$-Phenyl | O | H | |
| Cl | Cl | H | 4-$OCF_2CHF_2$-Phenyl | O | H | |
| Cl | Cl | H | 2-$SCH_3$-Phenyl | O | H | |
| Cl | Cl | H | 2,4-($SCH_3$,$SCH_3$)-Phenyl | O | H | |
| Cl | Cl | H | 2-$SCF_3$-Phenyl | O | H | |
| Cl | Cl | H | 4-$NO_2$-Phenyl | O | H | |
| Cl | Cl | H | 2,4-($NO_2$,$NO_2$)-Phenyl | O | H | |
| Cl | Cl | H | 3-$COCH_3$-Phenyl | O | H | |
| Cl | Cl | H | 3-$COCF_3$-Phenyl | O | H | |
| Cl | Cl | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Cl | H | 2-Naphthyl | O | H | |
| Cl | Cl | H | Piperidino | O | H | |
| Cl | Cl | H | 3-Tetrahydrofuranyl | O | H | |
| Cl | Cl | H | 4-Tetrahydropyranyl | O | H | |
| Cl | Cl | H | 2-Thiazolyl | O | H | |
| Cl | Cl | H | 5-$CH_3$-2-Thiazolyl | O | H | |
| Cl | Cl | H | 4-$CH_3$-5-COOH-2-Thiazolyl | O | H | |
| Cl | Br | H | Methyl | O | H | |
| Cl | Br | H | Ethyl | O | H | |
| Cl | Br | H | n-Propyl | O | H | |
| Cl | Br | H | iso-Propyl | O | H | |
| Cl | Br | H | Cyclopropyl | O | H | |
| Cl | Br | H | n-Butyl | O | H | |
| Cl | Br | H | iso-Butyl | O | H | |
| Cl | Br | H | sek.-Butyl | O | H | |
| Cl | Br | H | tert.-Butyl | O | H | |
| Cl | Br | H | n-Pentyl | O | H | |
| Cl | Br | H | 2-Pentyl | O | H | |
| Cl | Br | H | 3-Pentyl | O | H | |
| Cl | Br | H | n-Hexyl | O | H | |
| Cl | Br | H | 2-Hexyl | O | H | |
| Cl | Br | H | 3-Hexyl | O | H | |
| Cl | Br | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Br | H | cyclo-Propylmethyl | O | H | |
| Cl | Br | H | cyclo-Butyl | O | H | |
| Cl | Br | H | cyclo-Pentyl | O | H | |
| Cl | Br | H | cyclo-Hexyl | O | H | |
| Cl | Br | H | 1-Methylcyclohexyl | O | H | |
| Cl | Br | H | 3-Triflormethylcyclohexyl | O | H | |
| Cl | Br | H | Allyl | O | H | |
| Cl | Br | H | 1-Buten-3-yl | O | H | |
| Cl | Br | H | Crotyl | O | H | |
| Cl | Br | H | Propargyl | O | H | |
| Cl | Br | H | 1-Butin-3-yl | O | H | |
| Cl | Br | H | 3-Methyl-1-butin-3-yl | O | H | |
| Cl | Br | H | 2-Pentin-4-yl | O | H | |
| Cl | Br | H | Benzyl | O | H | |
| Cl | Br | H | 2-Phenylethyl | O | H | |
| Cl | Br | H | 2-Methylthioethyl | O | H | |
| Cl | Br | H | 2-Chlorethyl | O | H | |
| Cl | Br | H | 2-Methoxyethyl | O | H | |
| Cl | Br | H | 2-(N,N-Dimethylamino)ethyl | O | H | |
| Cl | Br | H | Phenyl | O | H | |
| Cl | Br | H | 2-$CH_3$-Phenyl | O | H | |
| Cl | Br | H | 3-$CH_3$-Phenyl | O | H | |
| Cl | Br | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Br | H | 2,4-($CH_3$,$CH_3$)-Phenyl | O | H | |
| Cl | Br | H | 2,3,5-($CH_3$,$CH_3$,$CH_3$)-Phenyl | O | H | |
| Cl | Br | H | 3-$CF_3$-Phenyl | O | H | |
| Cl | Br | H | 3-F-Phenyl | O | H | |
| Cl | Br | H | 2-Cl-Phenyl | O | H | |
| Cl | Br | H | 4-Cl-Phenyl | O | H | |
| Cl | Br | H | 2,4-(F,F)-Phenyl | O | H | |
| Cl | Br | H | 2,3,5-(Cl,Cl,Cl)-Phenyl | O | H | |
| Cl | Br | H | 2-CN-Phenyl | O | H | |
| Cl | Br | H | 2-$OCH_3$-Phenyl | O | H | |
| Cl | Br | H | 2,3-($OCH_3$,$OCH_3$)-Phenyl | O | H | |
| Cl | Br | H | 3,4,5-($OCH_3$,$OCH_3$,$OCH_3$)-Phenyl | O | H | |
| Cl | Br | H | 3-$OCF_3$-Phenyl | O | H | |
| Cl | Br | H | 3-$OCF_2OHF_2$-Phenyl | O | H | |
| Cl | Br | H | 4-$OCF_2CHF_2$-Phenyl | O | H | |
| Cl | Br | H | 2-$SCH_3$-Phenyl | O | H | |
| Cl | Br | H | 2,4-($SCH_3$,$SCH_3$)-Phenyl | O | H | |
| Cl | Br | H | 2-$SCF_3$-Phenyl | O | H | |
| Cl | Br | H | 4-$NO_2$-Phenyl | O | H | |
| Cl | Br | H | 2,4-($NO_2$,$NO_2$)-Phenyl | O | H | |
| Cl | Br | H | 3-$COCH_3$-Phenyl | O | H | |
| Cl | Br | H | 3-$COCF_3$-Phenyl | O | H | |
| Cl | Br | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| Cl | Br | H | 2-Naphthyl | O | H | |
| Cl | Br | H | Piperidino | O | H | |
| Cl | Br | H | 3-Tetrahydrofuranyl | O | H | |
| Cl | Br | H | 4-Tetrahydropyranyl | O | H | |
| Cl | Br | H | 2-Thiazolyl | O | H | |
| Cl | Br | H | 5-$CH_3$-2-Thiazolyl | O | H | |
| Cl | Br | H | 4-$CH_3$-5-COOH-2-Thiazolyl | O | H | |
| Br | Cl | H | Methyl | O | H | |
| Br | Cl | H | Ethyl | O | H | |
| Br | Cl | H | n-Propyl | O | H | |
| Br | Cl | H | iso-Propyl | O | H | |
| Br | Cl | H | Cyclopropyl | O | H | |
| Br | Cl | H | n-Butyl | O | H | |
| Br | Cl | H | iso-Butyl | O | H | |
| Br | Cl | H | sek.-Butyl | O | H | |
| Br | Cl | H | tert.-Butyl | O | H | |
| Br | Cl | H | n-Pentyl | O | H | |
| Br | Cl | H | 2-Pentyl | O | H | |
| Br | Cl | H | 3-Pentyl | O | H | |
| Br | Cl | H | n-Hexyl | O | H | |
| Br | Cl | H | 2-Hexyl | O | H | |
| Br | Cl | H | 3-Hexyl | O | H | |
| Br | Cl | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | Cl | H | cyclo-Propylmethyl | O | H | |
| Br | Cl | H | cyclo-Butyl | O | H | |
| Br | Cl | H | cyclo-Pentyl | O | H | |
| Br | Cl | H | cyclo-Hexyl | O | H | |
| Br | Cl | H | 1-Methylcyclohexyl | O | H | |
| Br | Cl | H | 3-Triflormethylcyclohexyl | O | H | |
| Br | Cl | H | Allyl | O | H | |
| Br | Cl | H | 1-Buten-3-yl | O | H | |
| Br | Cl | H | Crotyl | O | H | |
| Br | Cl | H | Propargyl | O | H | |
| Br | Cl | H | 1-Butin-3-yl | O | H | |
| Br | Cl | H | 3-Methyl-1-butin-3-yl | O | H | |
| Br | Cl | H | 2-Pentin-4-yl | O | H | |
| Br | Cl | H | Benzyl | O | H | |
| Br | Cl | H | 2-Phenylethyl | O | H | |
| Br | Cl | H | 2-Methylthioethyl | O | H | |
| Br | Cl | H | 2-Chlorethyl | O | H | |
| Br | Cl | H | 2-Methoxyethyl | O | H | |
| Br | Cl | H | 2-(N,N-Dimethylamino)ethyl | O | H | |
| Br | Cl | H | Phenyl | O | H | |
| Br | Cl | H | 2-$CH_3$-Phenyl | O | H | |
| Br | Cl | H | 3-$CH_3$-Phenyl | O | H | |
| Br | Cl | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | Cl | H | 2,4-($CH_3$,$CH_3$)-Phenyl | O | H | |
| Br | Cl | H | 2,3,5-($CH_3$,$CH_3$,$CH_3$)-Phenyl | O | H | |
| Br | Cl | H | 3-$CF_3$-Phenyl | O | H | |
| Br | Cl | H | 3-F-Phenyl | O | H | |
| Br | Cl | H | 2-Cl-Phenyl | O | H | |
| Br | Cl | H | 4-Cl-Phenyl | O | H | |
| Br | Cl | H | 2,4-(F,F)-Phenyl | O | H | |
| Br | Cl | H | 2,3,5-(Cl,Cl,Cl)-Phenyl | O | H | |
| Br | Cl | H | 2-CN-Phenyl | O | H | |
| Br | Cl | H | 2-$OCH_3$-Phenyl | O | H | |
| Br | Cl | H | 2,3-($OCH_3$,$OCH_3$)-Phenyl | O | H | |
| Br | Cl | H | 3,4,5-($OCH_3$,$OCH_3$,$OCH_3$)-Phenyl | O | H | |
| Br | Cl | H | 3-$OCF_3$-Phenyl | O | H | |
| Br | Cl | H | 3-$QCF_2OHF_2$-Phenyl | O | H | |
| Br | Cl | H | 4-$OCF_2CHF_2$-Phenyl | O | H | |
| Br | Cl | H | 2-$SCH_3$-Phenyl | O | H | |
| Br | Cl | H | 2,4-($SCH_3$,$SCH_3$)-Phenyl | O | H | |
| Br | Cl | H | 2-$SCF_3$-Phenyl | O | H | |
| Br | Cl | H | 4-$NO_2$-Phenyl | O | H | |
| Br | Cl | H | 2,4-($NO_2$,$NO_2$)-Phenyl | O | H | |
| Br | Cl | H | 3-$COCH_3$-Phenyl | O | H | |
| Br | Cl | H | 3-$COCF_3$-Phenyl | O | H | |
| Br | Cl | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | Cl | H | 2-Naphthyl | O | H | |
| Br | Cl | H | Piperidino | O | H | |
| Br | Cl | H | 3-Tetrahydrofuranyl | O | H | |
| Br | Cl | H | 4-Tetrahydropyranyl | O | H | |
| Br | Cl | H | 2-Thiazolyl | O | H | |
| Br | Cl | H | 5-$CH_3$-2-Thiazolyl | O | H | |
| Br | Cl | H | 4-$CH_3$-5-COOH-2-Thiazolyl | O | H | |
| H | $OCH_3$ | H | Methyl | O | H | |
| H | $OCH_3$ | H | Ethyl | O | H | |
| H | $OCH_3$ | H | n-Propyl | O | H | |
| H | $OCH_3$ | H | iso-Propyl | O | H | |
| H | $OCH_3$ | H | Cyclopropyl | O | H | |
| H | $OCH_3$ | H | n-Butyl | O | H | |
| H | $OCH_3$ | H | iso-Butyl | O | H | |
| H | $OCH_3$ | H | sek.-Butyl | O | H | |
| H | $OCH_3$ | H | tert.-Butyl | O | H | |
| H | $OCH_3$ | H | n-Pentyl | O | H | |
| H | $OCH_3$ | H | 2-Pentyl | O | H | |
| H | $OCH_3$ | H | 3-Pentyl | O | H | |
| H | $OCH_3$ | H | n-Hexyl | O | H | |
| H | $OCH_3$ | H | 2-Hexyl | O | H | |
| H | $OCH_3$ | H | 3-Hexyl | O | H | |
| H | $OCH_3$ | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | cyclo-Propylmethyl | O | H | |
| H | $OCH_3$ | H | cyclo-Butyl | O | H | |
| H | $OCH_3$ | H | cyclo-Pentyl | O | H | |
| H | $OCH_3$ | H | cyclo-Hexyl | O | H | |
| H | $OCH_3$ | H | 1-Methylcyclohexyl | O | H | |
| H | $OCH_3$ | H | 3-Triflormethylcyclohexyl | O | H | |
| H | $OCH_3$ | H | Allyl | O | H | |
| H | $OCH_3$ | H | 1-Buten-3-yl | O | H | |
| H | $OCH_3$ | H | Crotyl | O | H | |
| H | $OCH_3$ | H | Propargyl | O | H | |
| H | $OCH_3$ | H | 1-Butin-3-yl | O | H | |
| H | $OCH_3$ | H | 3-Methyl-1-butin-3-yl | O | H | |
| H | $OCH_3$ | H | 2-Pentin-4-yl | O | H | |
| H | $OCH_3$ | H | Benzyl | O | H | |
| H | $OCH_3$ | H | 2-Phenylethyl | O | H | |
| H | $OCH_3$ | H | 2-Methylthioethyl | O | H | |
| H | $OCH_3$ | H | 2-Chlorethyl | O | H | |
| H | $OCH_3$ | H | 2-Methoxyethyl | O | H | |
| H | $OCH_3$ | H | 2-(N,N-Dimethylamino)ethyl | O | H | |
| H | $OCH_3$ | H | Phenyl | O | H | |
| H | $OCH_3$ | H | 2-$CH_3$-Phenyl | O | H | |
| H | $OCH_3$ | H | 3-$CH_3$-Phenyl | O | H | |
| H | $OCH_3$ | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | 2,4-($CH_3$,$CH_3$)-Phenyl | O | H | |
| H | $OCH_3$ | H | 2,3,5-($CH_3$,$CH_3$,$CH_3$)-Phenyl | O | H | |
| H | $OCH_3$ | H | 3-$CF_3$-Phenyl | O | H | |
| H | $OCH_3$ | H | 3-F-Phenyl | O | H | |
| H | $OCH_3$ | H | 2-Cl-Phenyl | O | H | |
| H | $OCH_3$ | H | 4-Cl-Phenyl | O | H | |
| H | $OCH_3$ | H | 2,4-(F,F)-Phenyl | O | H | |
| H | $OCH_3$ | H | 2,3,5-(Cl,Cl,Cl)-Phenyl | O | H | |
| H | $OCH_3$ | H | 2-CN-Phenyl | O | H | |
| H | $OCH_3$ | H | 2-$OCH_3$-Phenyl | O | H | |
| H | $OCH_3$ | H | 2,3-($OCH_3$,$OCH_3$)-Phenyl | O | H | |
| H | $OCH_3$ | H | 3,4,5-($OCH_3$,$OCH_3$,$OCH_3$)-Phenyl | O | H | |
| H | $OCH_3$ | H | 3-$OCF_3$-Phenyl | O | H | |
| H | $OCH_3$ | H | 3-$OCF_2OHF_2$-Phenyl | O | H | |
| H | $OCH_3$ | H | 4-$OCF_2CHF_2$-Phenyl | O | H | |
| H | $OCH_3$ | H | 2-$SCH_3$-Phenyl | O | H | |
| H | $OCH_3$ | H | 2,4-($SCH_3$,$SCH_3$)-Phenyl | O | H | |
| H | $OCH_3$ | H | 2-$SCF_3$-Phenyl | O | H | |
| H | $OCH_3$ | H | 4-$NO_2$-Phenyl | O | H | |
| H | $OCH_3$ | H | 2,4-($NO_2$,$NO_2$)-Phenyl | O | H | |
| H | $OCH_3$ | H | 3-$COCH_3$-Phenyl | O | H | |
| H | $OCH_3$ | H | 3-$COCF_3$-Phenyl | O | H | |
| H | $OCH_3$ | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | 2-Naphthyl | O | H | |
| H | $OCH_3$ | H | Piperidino | O | H | |
| H | $OCH_3$ | H | 3-Tetrahydrofuranyl | O | H | |
| H | $OCH_3$ | H | 4-Tetrahydropyranyl | O | H | |
| H | $OCH_3$ | H | 2-Thiazolyl | O | H | |
| H | $OCH_3$ | H | 5-$CH_3$-2-Thiazolyl | O | H | |
| H | $OCH_3$ | H | 4-$CH_3$-5-COOH-2-Thiazolyl | O | H | |
| $OCH_3$ | H | H | Methyl | O | H | |
| $OCH_3$ | H | H | Ethyl | O | H | |
| $OCH_3$ | H | H | n-Propyl | O | H | |
| $OCH_3$ | H | H | iso-Propyl | O | H | |
| $OCH_3$ | H | H | Cyclopropyl | O | H | |
| $OCH_3$ | H | H | n-Butyl | O | H | |
| $OCH_3$ | H | H | iso-Butyl | O | H | |
| $OCH_3$ | H | H | sek.-Butyl | O | H | |
| $OCH_3$ | H | H | tert.-Butyl | O | H | |
| $OCH_3$ | H | H | n-Pentyl | O | H | |
| $OCH_3$ | H | H | 2-Pentyl | O | H | |
| $OCH_3$ | H | H | 3-Pentyl | O | H | |
| $OCH_3$ | H | H | n-Hexyl | O | H | |
| $OCH_3$ | H | H | 2-Hexyl | O | H | |
| $OCH_3$ | H | H | 3-Hexyl | O | H | |
| $OCH_3$ | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH_3$ | H | H | cyclo-Propylmethyl | O | H | |
| $OCH_3$ | H | H | cyclo-Butyl | O | H | |
| $OCH_3$ | H | H | cyclo-Pentyl | O | H | |
| $OCH_3$ | H | H | cyclo-Hexyl | O | H | |
| $OCH_3$ | H | H | 1-Methylcyclohexyl | O | H | |
| $OCH_3$ | H | H | 3-Triflormethylcyclohexyl | O | H | |
| $OCH_3$ | H | H | Allyl | O | H | |
| $OCH_3$ | H | H | 1-Buten-3-yl | O | H | |
| $OCH_3$ | H | H | Crotyl | O | H | |
| $OCH_3$ | H | H | Propargyl | O | H | |
| $OCH_3$ | H | H | 1-Butin-3-yl | O | H | |
| $OCH_3$ | H | H | 3-Methyl-1-butin-3-yl | O | H | |
| $OCH_3$ | H | H | 2-Pentin-4-yl | O | H | |
| $OCH_3$ | H | H | Benzyl | O | H | |
| $OCH_3$ | H | H | 2-Phenylethyl | O | H | |
| $OCH_3$ | H | H | 2-Methylthioethyl | O | H | |
| $OCH_3$ | H | H | 2-Chlorethyl | O | H | |
| $OCH_3$ | H | H | 2-Methoxyethyl | O | H | |
| $OCH_3$ | H | H | 2-(N,N-Dimethylamino)ethyl | O | H | |
| $OCH_3$ | H | H | Phenyl | O | H | |
| $OCH_3$ | H | H | 2-$CH_3$-Phenyl | O | H | |
| $OCH_3$ | H | H | 3-$CH_3$-Phenyl | O | H | |
| $OCH_3$ | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH_3$ | H | H | 2,4-($CH_3$,$CH_3$)-Phenyl | O | H | |
| $OCH_3$ | H | H | 2,3,5-($CH_3$,$CH_3$,$CH_3$)-Phenyl | O | H | |
| $OCH_3$ | H | H | 3-$CF_3$-Phenyl | O | H | |
| $OCH_3$ | H | H | 3-F-Phenyl | O | H | |
| $OCH_3$ | H | H | 2-Cl-Phenyl | O | H | |
| $OCH_3$ | H | H | 4-Cl-Phenyl | O | H | |
| $OCH_3$ | H | H | 2,4-(F,F)-Phenyl | O | H | |
| $OCH_3$ | H | H | 2,3,5-(Cl,Cl,Cl)-Phenyl | O | H | |
| $OCH_3$ | H | H | 2-CN-Phenyl | O | H | |
| $OCH_3$ | H | H | 2-$OCH_3$-Phenyl | O | H | |
| $OCH_3$ | H | H | 2,3-($OCH_3$,$OCH_3$)-Phenyl | O | H | |
| $OCH_3$ | H | H | 3,4,5-($OCH_3$,$OCH_3$,$OCH_3$)-Phenyl | O | H | |
| $OCH_3$ | H | H | 3-$OCF_3$-Phenyl | O | H | |
| $OCH_3$ | H | H | 3-$OCF_2OHF_2$-Phenyl | O | H | |
| $OCH_3$ | H | H | 4-$OCF_2CHF_2$-Phenyl | O | H | |
| $OCH_3$ | H | H | 2-$SCH_3$-Phenyl | O | H | |
| $OCH_3$ | H | H | 2,4-($SCH_3$,$SCH_3$)-Phenyl | O | H | |
| $OCH_3$ | H | H | 2-$SCF_3$-Phenyl | O | H | |
| $OCH_3$ | H | H | 4-$NO_2$-Phenyl | O | H | |
| $OCH_3$ | H | H | 2,4-($NO_2$,$NO_2$)-Phenyl | O | H | |
| $OCH_3$ | H | H | 3-$COCH_3$-Phenyl | O | H | |
| $OCH_3$ | H | H | 3-$COCF_3$-Phenyl | O | H | |
| $OCH_3$ | H | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH_3$ | H | H | 2-Naphthyl | O | H | |
| $OCH_3$ | H | H | Piperidino | O | H | |
| $OCH_3$ | H | H | 3-Tetrahydrofuranyl | O | H | |
| $OCH_3$ | H | H | 4-Tetrahydropyranyl | O | H | |
| $OCH_3$ | H | H | 2-Thiazolyl | O | H | |
| $OCH_3$ | H | H | 5-$CH_3$-2-Thiazolyl | O | H | |
| $OCH_3$ | H | H | 4-$CH_3$-5-COOH-2-Thiazolyl | O | H | |
| H | $CH_3$ | H | Methyl | O | H | |
| H | $CH_3$ | H | Ethyl | O | H | |
| H | $CH_3$ | H | n-Propyl | O | H | |
| H | $CH_3$ | H | iso-Propyl | O | H | |
| H | $CH_3$ | H | Cyclopropyl | O | H | |
| H | $CH_3$ | H | n-Butyl | O | H | |
| H | $CH_3$ | H | iso-Butyl | O | H | |
| H | $CH_3$ | H | sek.-Butyl | O | H | |
| H | $CH_3$ | H | tert.-Butyl | O | H | |
| H | $CH_3$ | H | n-Pentyl | O | H | |
| H | $CH_3$ | H | 2-Pentyl | O | H | |
| H | $CH_3$ | H | 3-Pentyl | O | H | |
| H | $CH_3$ | H | n-Hexyl | O | H | |
| H | $CH_3$ | H | 2-Hexyl | O | H | |
| H | $CH_3$ | H | 3-Hexyl | O | H | |
| H | $CH_3$ | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH_3$ | H | cyclo-Propylmethyl | O | H | |
| H | $CH_3$ | H | cyclo-Butyl | O | H | |
| H | $CH_3$ | H | cyclo-Pentyl | O | H | |
| H | $CH_3$ | H | cyclo-Hexyl | O | H | |
| H | $CH_3$ | H | 1-Methylcyclohexyl | O | H | |
| H | $CH_3$ | H | 3-Triflormethylcyclohexyl | O | H | |
| H | $CH_3$ | H | Allyl | O | H | |
| H | $CH_3$ | H | 1-Buten-3-yl | O | H | |
| H | $CH_3$ | H | Crotyl | O | H | |
| H | $CH_3$ | H | Propargyl | O | H | |
| H | $CH_3$ | H | 1-Butin-3-yl | O | H | |
| H | $CH_3$ | H | 3-Methyl-1-butin-3-yl | O | H | |
| H | $CH_3$ | H | 2-Pentin-4-yl | O | H | |
| H | $CH_3$ | H | Benzyl | O | H | |
| H | $CH_3$ | H | 2-Phenylethyl | O | H | |
| H | $CH_3$ | H | 2-Methylthioethyl | O | H | |
| H | $CH_3$ | H | 2-Chlorethyl | O | H | |
| H | $CH_3$ | H | 2-Methoxyethyl | O | H | |
| H | $CH_3$ | H | 2-(N,N-Dimethylamino)ethyl | O | H | |
| H | $CH_3$ | H | Phenyl | O | H | |
| H | $CH_3$ | H | 2-$CH_3$-Phenyl | O | H | |
| H | $CH_3$ | H | 3-$CH_3$-Phenyl | O | H | |
| H | $CH_3$ | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH_3$ | H | 2,4-($CH_3$,$CH_3$)-Phenyl | O | H | |
| H | $CH_3$ | H | 2,3,5-($CH_3$,$CH_3$,$CH_3$)-Phenyl | O | H | |
| H | $CH_3$ | H | 3-$CF_3$-Phenyl | O | H | |
| H | $CH_3$ | H | 3-F-Phenyl | O | H | |
| H | $CH_3$ | H | 2-Cl-Phenyl | O | H | |
| H | $CH_3$ | H | 4-Cl-Phenyl | O | H | |
| H | $CH_3$ | H | 2,4-(F,F)-Phenyl | O | H | |
| H | $CH_3$ | H | 2,3,5-(Cl,Cl,Cl)-Phenyl | O | H | |
| H | $CH_3$ | H | 2-CN-Phenyl | O | H | |
| H | $CH_3$ | H | 2-$OCH_3$-Phenyl | O | H | |
| H | $CH_3$ | H | 2,3-($OCH_3$,$OCH_3$)-Phenyl | O | H | |
| H | $CH_3$ | H | 3,4,5-($OCH_3$,$OCH_3$,$OCH_3$)-Phenyl | O | H | |
| H | $CH_3$ | H | 3-$OCF_3$-Phenyl | O | H | |
| H | $CH_3$ | H | 3-$OCF_2OHF_2$-Phenyl | O | H | |
| H | $CH_3$ | H | 4-$OCF_2CHF_2$-Phenyl | O | H | |
| H | $CH_3$ | H | 2-$SCH_3$-Phenyl | O | H | |
| H | $CH_3$ | H | 2,4-($SCH_3$,$SCH_3$)-Phenyl | O | H | |
| H | $CH_3$ | H | 2-$SCF_3$-Phenyl | O | H | |
| H | $CH_3$ | H | 4-$NO_2$-Phenyl | O | H | |
| H | $CH_3$ | H | 2,4-($NO_2$,$NO_2$)-Phenyl | O | H | |
| H | $CH_3$ | H | 3-$COCH_3$-Phenyl | O | H | |
| H | $CH_3$ | H | 3-$COCF_3$-Phenyl | O | H | |
| H | $CH_3$ | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH_3$ | H | 2-Naphthyl | O | H | |
| H | $CH_3$ | H | Piperidino | O | H | |
| H | $CH_3$ | H | 3-Tetrahydrofuranyl | O | H | |
| H | $CH_3$ | H | 4-Tetrahydropyranyl | O | H | |
| H | $CH_3$ | H | 2-Thiazolyl | O | H | |
| H | $CH_3$ | H | 5-$CH_3$-2-Thiazolyl | O | H | |
| H | $CH_3$ | H | 4-$CH_3$-5-COOH-2-Thiazolyl | O | H | |
| $CH_3$ | H | H | Methyl | O | H | |
| $CH_3$ | H | H | Ethyl | O | H | |
| $CH_3$ | H | H | n-Propyl | O | H | |
| $CH_3$ | H | H | iso-Propyl | O | H | |
| $CH_3$ | H | H | Cyclopropyl | O | H | |
| $CH_3$ | H | H | n-Butyl | O | H | |
| $CH_3$ | H | H | iso-Butyl | O | H | |
| $CH_3$ | H | H | sek.-Butyl | O | H | |
| $CH_3$ | H | H | tert.-Butyl | O | H | |
| $CH_3$ | H | H | n-Pentyl | O | H | |
| $CH_3$ | H | H | 2-Pentyl | O | H | |
| $CH_3$ | H | H | 3-Pentyl | O | H | |
| $CH_3$ | H | H | n-Hexyl | O | H | |
| $CH_3$ | H | H | 2-Hexyl | O | H | |
| $CH_3$ | H | H | 3-Hexyl | O | H | |
| $CH_3$ | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | H | H | cyclo-Propylmethyl | O | H | |
| $CH_3$ | H | H | cyclo-Butyl | O | H | |
| $CH_3$ | H | H | cyclo-Pentyl | O | H | |
| $CH_3$ | H | H | cyclo-Hexyl | O | H | |
| $CH_3$ | H | H | 1-Methylcyclohexyl | O | H | |
| $CH_3$ | H | H | 3-Triflormethylcyclohexyl | O | H | |
| $CH_3$ | H | H | Allyl | O | H | |
| $CH_3$ | H | H | 1-Buten-3-yl | O | H | |
| $CH_3$ | H | H | Crotyl | O | H | |
| $CH_3$ | H | H | Propargyl | O | H | |
| $CH_3$ | H | H | 1-Butin-3-yl | O | H | |
| $CH_3$ | H | H | 3-Methyl-1-butin-3-yl | O | H | |
| $CH_3$ | H | H | 2-Pentin-4-yl | O | H | |
| $CH_3$ | H | H | Benzyl | O | H | |
| $CH_3$ | H | H | 2-Phenylethyl | O | H | |
| $CH_3$ | H | H | 2-Methylthioethyl | O | H | |
| $CH_3$ | H | H | 2-Chlorethyl | O | H | |
| $CH_3$ | H | H | 2-Methoxyethyl | O | H | |
| $CH_3$ | H | H | 2-(N,N-Dimethylamino)ethyl | O | H | |
| $CH_3$ | H | H | Phenyl | O | H | |
| $CH_3$ | H | H | 2-$CH_3$-Phenyl | O | H | |
| $CH_3$ | H | H | 3-$CH_3$-Phenyl | O | H | |
| $CH_3$ | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | H | H | 2,4-($CH_3$,$CH_3$)-Phenyl | O | H | |
| $CH_3$ | H | H | 2,3,5-($CH_3$,$CH_3$,$CH_3$)-Phenyl | O | H | |
| $CH_3$ | H | H | 3-$CF_3$-Phenyl | O | H | |
| $CH_3$ | H | H | 3-F-Phenyl | O | H | |
| $CH_3$ | H | H | 2-Cl-Phenyl | O | H | |
| $CH_3$ | H | H | 4-Cl-Phenyl | O | H | |
| $CH_3$ | H | H | 2,4-(F,F)-Phenyl | O | H | |
| $CH_3$ | H | H | 2,3,5-(Cl,Cl,Cl)-Phenyl | O | H | |
| $CH_3$ | H | H | 2-CN-Phenyl | O | H | |
| $CH_3$ | H | H | 2-$OCH_3$-Phenyl | O | H | |
| $CH_3$ | H | H | 2,3-($OCH_3$,$OCH_3$)-Phenyl | O | H | |
| $CH_3$ | H | H | 3,4,5-($OCH_3$,$OCH_3$,$OCH_3$)-Phenyl | O | H | |
| $CH_3$ | H | H | 3-$OCF_3$-Phenyl | O | H | |
| $CH_3$ | H | H | 3-$OCF_2OHF_2$-Phenyl | O | H | |
| $CH_3$ | H | H | 4-$OCF_2CHF_2$-Phenyl | O | H | |
| $CH_3$ | H | H | 2-$SCH_3$-Phenyl | O | H | |
| $CH_3$ | H | H | 2,4-($SCH_3$,$SCH_3$)-Phenyl | O | H | |
| $CH_3$ | H | H | 2-$SCF_3$-Phenyl | O | H | |
| $CH_3$ | H | H | 4-$NO_2$-Phenyl | O | H | |
| $CH_3$ | H | H | 2,4-($NO_2$,$NO_2$)-Phenyl | O | H | |
| $CH_3$ | H | H | 3-$COCH_3$-Phenyl | O | H | |
| $CH_3$ | H | H | 3-$COCF_3$-Phenyl | O | H | |
| $CH_3$ | H | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | H | H | 2-Naphthyl | O | H | |
| $CH_3$ | H | H | Piperidino | O | H | |
| $CH_3$ | H | H | 3-Tetrahydrofuranyl | O | H | |
| $CH_3$ | H | H | 4-Tetrahydropyranyl | O | H | |
| $CH_3$ | H | H | 2-Thiazolyl | O | H | |
| $CH_3$ | H | H | 5-$CH_3$-2-Thiazolyl | O | H | |
| $CH_3$ | H | H | 4-$CH_3$-5-COOH-2-Thiazolyl | O | H | |
| $CH_3$ | $CH_3$ | H | Methyl | O | H | |
| $CH_3$ | $CH_3$ | H | Ethyl | O | H | |
| $CH_3$ | $CH_3$ | H | n-Propyl | O | H | |
| $CH_3$ | $CH_3$ | H | iso-Propyl | O | H | |
| $CH_3$ | $CH_3$ | H | Cyclopropyl | O | H | |
| $CH_3$ | $CH_3$ | H | n-Butyl | O | H | |
| $CH_3$ | $CH_3$ | H | iso-Butyl | O | H | |
| $CH_3$ | $CH_3$ | H | sek.-Butyl | O | H | |
| $CH_3$ | $CH_3$ | H | tert.-Butyl | O | H | |
| $CH_3$ | $CH_3$ | H | n-Pentyl | O | H | |
| $CH_3$ | $CH_3$ | H | 2-Pentyl | O | H | |
| $CH_3$ | $CH_3$ | H | 3-Pentyl | O | H | |
| $CH_3$ | $CH_3$ | H | n-Hexyl | O | H | |
| $CH_3$ | $CH_3$ | H | 2-Hexyl | O | H | |
| $CH_3$ | $CH_3$ | H | 3-Hexyl | O | H | |
| $CH_3$ | $CH_3$ | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | H | cyclo-Propylmethyl | O | H | |
| $CH_3$ | $CH_3$ | H | cyclo-Butyl | O | H | |
| $CH_3$ | $CH_3$ | H | cyclo-Pentyl | O | H | |
| $CH_3$ | $CH_3$ | H | cyclo-Hexyl | O | H | |
| $CH_3$ | $CH_3$ | H | 1-Methylcyclohexyl | O | H | |
| $CH_3$ | $CH_3$ | H | 3-Triflormethylcyclohexyl | O | H | |
| $CH_3$ | $CH_3$ | H | Allyl | O | H | |
| $CH_3$ | $CH_3$ | H | 1-Buten-3-yl | O | H | |
| $CH_3$ | $CH_3$ | H | Crotyl | O | H | |
| $CH_3$ | $CH_3$ | H | Propargyl | O | H | |
| $CH_3$ | $CH_3$ | H | 1-Butin-3-yl | O | H | |
| $CH_3$ | $CH_3$ | H | 3-Methyl-1-butin-3-yl | O | H | |
| $CH_3$ | $CH_3$ | H | 2-Pentin-4-yl | O | H | |
| $CH_3$ | $CH_3$ | H | Benzyl | O | H | |
| $CH_3$ | $CH_3$ | H | 2-Phenylethyl | O | H | |
| $CH_3$ | $CH_3$ | H | 2-Methylthioethyl | O | H | |
| $CH_3$ | $CH_3$ | H | 2-Chlorethyl | O | H | |
| $CH_3$ | $CH_3$ | H | 2-Methoxyethyl | O | H | |
| $CH_3$ | $CH_3$ | H | 2-(N,N-Dimethylamino)ethyl | O | H | |
| $CH_3$ | $CH_3$ | H | Phenyl | O | H | |
| $CH_3$ | $CH_3$ | H | 2-$CH_3$-Phenyl | O | H | |
| $CH_3$ | $CH_3$ | H | 3-$CH_3$-Phenyl | O | H | |
| $CH_3$ | $CH_3$ | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | H | 2,4-($CH_3$,$CH_3$)-Phenyl | O | H | |
| $CH_3$ | $CH_3$ | H | 2,3,5-($CH_3$,$CH_3$,$CH_3$)-Phenyl | O | H | |
| $CH_3$ | $CH_3$ | H | 3-$CF_3$-Phenyl | O | H | |
| $CH_3$ | $CH_3$ | H | 3-F-Phenyl | O | H | |
| $CH_3$ | $CH_3$ | H | 2-Cl-Phenyl | O | H | |
| $CH_3$ | $CH_3$ | H | 4-Cl-Phenyl | O | H | |
| $CH_3$ | $CH_3$ | H | 2,4-(F,F)-Phenyl | O | H | |
| $CH_3$ | $CH_3$ | H | 2,3,5-(Cl,Cl,Cl)-Phenyl | O | H | |
| $CH_3$ | $CH_3$ | H | 2-CN-Phenyl | O | H | |
| $CH_3$ | $CH_3$ | H | 2-$OCH_3$-Phenyl | O | H | |
| $CH_3$ | $CH_3$ | H | 2,3-($OCH_3$,$OCH_3$)-Phenyl | O | H | |
| $CH_3$ | $CH_3$ | H | 3,4,5-($OCH_3$,$OCH_3$,$OCH_3$)-Phenyl | O | H | |
| $CH_3$ | $CH_3$ | H | 3-$OCF_3$-Phenyl | O | H | |
| $CH_3$ | $CH_3$ | H | 3-$OCF_2OHF_2$-Phenyl | O | H | |
| $CH_3$ | $CH_3$ | H | 4-$OCF_2CHF_2$-Phenyl | O | H | |
| $CH_3$ | $CH_3$ | H | 2-$SCH_3$-Phenyl | O | H | |
| $CH_3$ | $CH_3$ | H | 2,4-($SCH_3$,$SCH_3$)-Phenyl | O | H | |
| $CH_3$ | $CH_3$ | H | 2-$SCF_3$-Phenyl | O | H | |
| $CH_3$ | $CH_3$ | H | 4-$NO_2$-Phenyl | O | H | |
| $CH_3$ | $CH_3$ | H | 2,4-($NO_2$,$NO_2$)-Phenyl | O | H | |
| $CH_3$ | $CH_3$ | H | 3-$COCH_3$-Phenyl | O | H | |
| $CH_3$ | $CH_3$ | H | 3-$COCF_3$-Phenyl | O | H | |
| $CH_3$ | $CH_3$ | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | H | 2-Naphthyl | O | H | |
| $CH_3$ | $CH_3$ | H | Piperidino | O | H | |
| $CH_3$ | $CH_3$ | H | 3-Tetrahydrofuranyl | O | H | |
| $CH_3$ | $CH_3$ | H | 4-Tetrahydropyranyl | O | H | |
| $CH_3$ | $CH_3$ | H | 2-Thiazolyl | O | H | |
| $CH_3$ | $CH_3$ | H | 5-$CH_3$-2-Thiazolyl | O | H | |
| $CH_3$ | $CH_3$ | H | 4-$CH_3$-5-COOH-2-Thiazolyl | O | H | |
| H | $CH(CH_3)_2$ | H | Methyl | O | H | |
| H | $CH(CH_3)_2$ | H | Ethyl | O | H | |
| H | $CH(CH_3)_2$ | H | n-Propyl | O | H | |
| H | $CH(CH_3)_2$ | H | iso-Propyl | O | H | |
| H | $CH(CH_3)_2$ | H | Cyclopropyl | O | H | |
| H | $CH(CH_3)_2$ | H | n-Butyl | O | H | |
| H | $CH(CH_3)_2$ | H | iso-Butyl | O | H | |
| H | $CH(CH_3)_2$ | H | sek.-Butyl | O | H | |
| H | $CH(CH_3)_2$ | H | tert.-Butyl | O | H | |
| H | $CH(CH_3)_2$ | H | n-Pentyl | O | H | |
| H | $CH(CH_3)_2$ | H | 2-Pentyl | O | H | |
| H | $CH(CH_3)_2$ | H | 3-Pentyl | O | H | |
| H | $CH(CH_3)_2$ | H | n-Hexyl | O | H | |
| H | $CH(CH_3)_2$ | H | 2-Hexyl | O | H | |
| H | $CH(CH_3)_2$ | H | 3-Hexyl | O | H | |
| H | $CH(CH_3)_2$ | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH(CH_3)_2$ | H | cyclo-Propylmethyl | O | H | |
| H | $CH(CH_3)_2$ | H | cyclo-Butyl | O | H | |
| H | $CH(CH_3)_2$ | H | cyclo-Pentyl | O | H | |
| H | $CH(CH_3)_2$ | H | cyclo-Hexyl | O | H | |
| H | $CH(CH_3)_2$ | H | 1-Methylcyclohexyl | O | H | |
| H | $CH(CH_3)_2$ | H | 3-Triflormethylcyclohexyl | O | H | |
| H | $CH(CH_3)_2$ | H | Allyl | O | H | |
| H | $CH(CH_3)_2$ | H | 1-Buten-3-yl | O | H | |
| H | $CH(CH_3)_2$ | H | Crotyl | O | H | |
| H | $CH(CH_3)_2$ | H | Propargyl | O | H | |
| H | $CH(CH_3)_2$ | H | 1-Butin-3-yl | O | H | |
| H | $CH(CH_3)_2$ | H | 3-Methyl-1-butin-3-yl | O | H | |
| H | $CH(CH_3)_2$ | H | 2-Pentin-4-yl | O | H | |
| H | $CH(CH_3)_2$ | H | Benzyl | O | H | |
| H | $CH(CH_3)_2$ | H | 2-Phenylethyl | O | H | |
| H | $CH(CH_3)_2$ | H | 2-Methylthioethyl | O | H | |
| H | $CH(CH_3)_2$ | H | 2-Chlorethyl | O | H | |
| H | $CH(CH_3)_2$ | H | 2-Methoxyethyl | O | H | |
| H | $CH(CH_3)_2$ | H | 2-(N,N-Dimethylamino)ethyl | O | H | |
| H | $CH(CH_3)_2$ | H | Phenyl | O | H | |
| H | $CH(CH_3)_2$ | H | 2-$CH_3$-Phenyl | O | H | |
| H | $CH(CH_3)_2$ | H | 3-$CH_3$-Phenyl | O | H | |
| H | $CH(CH_3)_2$ | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH(CH_3)_2$ | H | 2,4-($CH_3$,$CH_3$)-Phenyl | O | H | |
| H | $CH(CH_3)_2$ | H | 2,3,5-($CH_3$,$CH_3$,$CH_3$)-Phenyl | O | H | |
| H | $CH(CH_3)_2$ | H | 3-$CF_3$-Phenyl | O | H | |
| H | $CH(CH_3)_2$ | H | 3-F-Phenyl | O | H | |
| H | $CH(CH_3)_2$ | H | 2-Cl-Phenyl | O | H | |
| H | $CH(CH_3)_2$ | H | 4-Cl-Phenyl | O | H | |
| H | $CH(CH_3)_2$ | H | 2,4-(F,F)-Phenyl | O | H | |
| H | $CH(CH_3)_2$ | H | 2,3,5-(Cl,Cl,Cl)-Phenyl | O | H | |
| H | $CH(CH_3)_2$ | H | 2-CN-Phenyl | O | H | |
| H | $CH(CH_3)_2$ | H | 2-$OCH_3$-Phenyl | O | H | |
| H | $CH(CH_3)_2$ | H | 2,3-($OCH_3$,$OCH_3$)-Phenyl | O | H | |
| H | $CH(CH_3)_2$ | H | 3,4,5-($OCH_3$,$OCH_3$,$OCH_3$)-Phenyl | O | H | |
| H | $CH(CH_3)_2$ | H | 3-$OCF_3$-Phenyl | O | H | |
| H | $CH(CH_3)_2$ | H | 3-$OCF_2OHF_2$-Phenyl | O | H | |
| H | $CH(CH_3)_2$ | H | 4-$OCF_2CHF_2$-Phenyl | O | H | |
| H | $CH(CH_3)_2$ | H | 2-$SCH_3$-Phenyl | O | H | |
| H | $CH(CH_3)_2$ | H | 2,4-($SCH_3$,$SCH_3$)-Phenyl | O | H | |
| H | $CH(CH_3)_2$ | H | 2-$SCF_3$-Phenyl | O | H | |
| H | $CH(CH_3)_2$ | H | 4-$NO_2$-Phenyl | O | H | |
| H | $CH(CH_3)_2$ | H | 2,4-($NO_2$,$NO_2$)-Phenyl | O | H | |
| H | $CH(CH_3)_2$ | H | 3-$COCH_3$-Phenyl | O | H | |
| H | $CH(CH_3)_2$ | H | 3-$COCF_3$-Phenyl | O | H | |
| H | $CH(CH_3)_2$ | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| H | $CH(CH_3)_2$ | H | 2-Naphthyl | O | H | |
| H | $CH(CH_3)_2$ | H | Piperidino | O | H | |
| H | $CH(CH_3)_2$ | H | 3-Tetrahydrofuranyl | O | H | |
| H | $CH(CH_3)_2$ | H | 4-Tetrahydropyranyl | O | H | |
| H | $CH(CH_3)_2$ | H | 2-Thiazolyl | O | H | |
| H | $CH(CH_3)_2$ | H | 5-$CH_3$-2-Thiazolyl | O | H | |
| H | $CH(CH_3)_2$ | H | 4-$CH_3$-5-COOH-2-Thiazolyl | O | H | |
| $OCH(CH_3)_2$ | H | H | Methyl | O | H | |
| $OCH(CH_3)_2$ | H | H | Ethyl | O | H | |
| $OCH(CH_3)_2$ | H | H | n-Propyl | O | H | |
| $OCH(CH_3)_2$ | H | H | iso-Propyl | O | H | |
| $OCH(CH_3)_2$ | H | H | Cyclopropyl | O | H | |
| $OCH(CH_3)_2$ | H | H | n-Butyl | O | H | |
| $OCH(CH_3)_2$ | H | H | iso-Butyl | O | H | |
| $OCH(CH_3)_2$ | H | H | sek.-Butyl | O | H | |
| $OCH(CH_3)_2$ | H | H | tert.-Butyl | O | H | |
| $OCH(CH_3)_2$ | H | H | n-Pentyl | O | H | |
| $OCH(CH_3)_2$ | H | H | 2-Pentyl | O | H | |
| $OCH(CH_3)_2$ | H | H | 3-Pentyl | O | H | |
| $OCH(CH_3)_2$ | H | H | n-Hexyl | O | H | |
| $OCH(CH_3)_2$ | H | H | 2-Hexyl | O | H | |
| $OCH(CH_3)_2$ | H | H | 3-Hexyl | O | H | |
| $OCH(CH_3)_2$ | H | H | 2-Methyl-2-pentyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH(CH_3)_2$ | H | H | cyclo-Propylmethyl | O | H | |
| $OCH(CH_3)_2$ | H | H | cyclo-Butyl | O | H | |
| $OCH(CH_3)_2$ | H | H | cyclo-Pentyl | O | H | |
| $OCH(CH_3)_2$ | H | H | cyclo-Hexyl | O | H | |
| $OCH(CH_3)_2$ | H | H | 1-Methylcyclohexyl | O | H | |
| $OCH(CH_3)_2$ | H | H | 3-Triflormethylcyclohexyl | O | H | |
| $OCH(CH_3)_2$ | H | H | Allyl | O | H | |
| $OCH(CH_3)_2$ | H | H | 1-Buten-3-yl | O | H | |
| $OCH(CH_3)_2$ | H | H | Crotyl | O | H | |
| $OCH(CH_3)_2$ | H | H | Propargyl | O | H | |
| $OCH(CH_3)_2$ | H | H | 1-Butin-3-yl | O | H | |
| $OCH(CH_3)_2$ | H | H | 3-Methyl-1-butin-3-yl | O | H | |
| $OCH(CH_3)_2$ | H | H | 2-Pentin-4-yl | O | H | |
| $OCH(CH_3)_2$ | H | H | Benzyl | O | H | |
| $OCH(CH_3)_2$ | H | H | 2-Phenylethyl | O | H | |
| $OCH(CH_3)_2$ | H | H | 2-Methylthioethyl | O | H | |
| $OCH(CH_3)_2$ | H | H | 2-Chlorethyl | O | H | |
| $OCH(CH_3)_2$ | H | H | 2-Methoxyethyl | O | H | |
| $OCH(CH_3)_2$ | H | H | 2-(N,N-Dimethylamino)ethyl | O | H | |
| $OCH(CH_3)_2$ | H | H | Phenyl | O | H | |
| $OCH(CH_3)_2$ | H | H | 2-$CH_3$-Phenyl | O | H | |
| $OCH(CH_3)_2$ | H | H | 3-$CH_3$-Phenyl | O | H | |
| $OCH(CH_3)_2$ | H | H | 4-$CH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH(CH_3)_2$ | H | H | 2,4-($CH_3$,$CH_3$)-Phenyl | O | H | |
| $OCH(CH_3)_2$ | H | H | 2,3,5-($CH_3$,$CH_3$,$CH_3$)-Phenyl | O | H | |
| $OCH(CH_3)_2$ | H | H | 3-$CF_3$-Phenyl | O | H | |
| $OCH(CH_3)_2$ | H | H | 3-F-Phenyl | O | H | |
| $OCH(CH_3)_2$ | H | H | 2-Cl-Phenyl | O | H | |
| $OCH(CH_3)_2$ | H | H | 4-Cl-Phenyl | O | H | |
| $OCH(CH_3)_2$ | H | H | 2,4-(F,F)-Phenyl | O | H | |
| $OCH(CH_3)_2$ | H | H | 2,3,5-(Cl,Cl,Cl)-Phenyl | O | H | |
| $OCH(CH_3)_2$ | H | H | 2-CN-Phenyl | O | H | |
| $OCH(CH_3)_2$ | H | H | 2-$OCH_3$-Phenyl | O | H | |
| $OCH(CH_3)_2$ | H | H | 2,3-($OCH_3$,$OCH_3$)-Phenyl | O | H | |
| $OCH(CH_3)_2$ | H | H | 3,4,5-($OCH_3$,$OCH_3$,$OCH_3$)-Phenyl | O | H | |
| $OCH(CH_3)_2$ | H | H | 3-$OCF_3$-Phenyl | O | H | |
| $OCH(CH_3)_2$ | H | H | 3-$OCF_2OHF_2$-Phenyl | O | H | |
| $OCH(CH_3)_2$ | H | H | 4-$OCF_2CHF_2$-Phenyl | O | H | |
| $OCH(CH_3)_2$ | H | H | 2-$SCH_3$-Phenyl | O | H | |
| $OCH(CH_3)_2$ | H | H | 2,4-($SCH_3$,$SCH_3$)-Phenyl | O | H | |
| $OCH(CH_3)_2$ | H | H | 2-$SCF_3$-Phenyl | O | H | |
| $OCH(CH_3)_2$ | H | H | 4-$NO_2$-Phenyl | O | H | |
| $OCH(CH_3)_2$ | H | H | 2,4-($NO_2$,$NO_2$)-Phenyl | O | H | |
| $OCH(CH_3)_2$ | H | H | 3-$COCH_3$-Phenyl | O | H | |
| $OCH(CH_3)_2$ | H | H | 3-$COCF_3$-Phenyl | O | H | |
| $OCH(CH_3)_2$ | H | H | 1-Naphthyl | O | H | |

Tabelle (Fortsetzung)

| $R^3$ | $R^4$ | $R^1$ | $R^2$ | Y | $R^6$ | X = O oder S |
|---|---|---|---|---|---|---|
| $OCH(CH_3)_2$ | H | H | 2-Naphthyl | O | H | |
| $OCH(CH_3)_2$ | H | H | Piperidino | O | H | |
| $OCH(CH_3)_2$ | H | H | 3-Tetrahydrofuranyl | O | H | |
| $OCH(CH_3)_2$ | H | H | 4-Tetrahydropyranyl | O | H | |
| $OCH(CH_3)_2$ | H | H | 2-Thiazolyl | O | H | |
| $OCH(CH_3)_2$ | H | H | 5-$CH_3$-2-Thiazolyl | O | H | |
| $OCH(CH_3)_2$ | H | H | 4-$CH_3$-5-COOH-2-Thiazolyl | O | H | |
| H | Cl | H | Methyl | O | 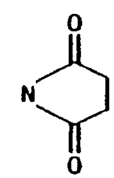 | |
| H | Cl | H | Ethyl | O | " | |
| H | Cl | H | n-Propyl | O | " | |
| H | Cl | H | iso-Propyl | O | " | |
| H | Cl | H | Cyclopropyl | O | " | |
| H | Cl | H | n-Butyl | O | " | |
| H | Cl | H | iso-Butyl | O | " | |
| H | Cl | H | sek.-Butyl | O | " | |
| H | Cl | H | tert.-Butyl | O | " | |
| H | Cl | H | n-Pentyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2-Penyl | O | 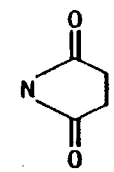 | |
| H | Cl | H | 3-Pentyl | O | " | |
| H | Cl | H | n-Hexyl | O | " | |
| H | Cl | H | 2-Hexyl | O | " | |
| H | Cl | H | 3-Hexyl | O | " | |
| H | Cl | H | 2-Methyl-2-pentyl | O | " | |
| H | Cl | H | cyclo-Propylmethyl | O | " | |
| H | Cl | H | cyclo-Butyl | O | " | |
| H | Cl | H | cyclo-Pentyl | O | " | |
| H | Cl | H | cyclo-Hexyl | O | " | |
| H | Cl | H | 1-Methylcyclohexyl | O | " | |
| H | Cl | H | 3-Triflormethylcyclohexyl | O | " | |
| H | Cl | H | Allyl | O | " | |
| H | Cl | H | 1-Buten-3-yl | O | " | |
| H | Cl | H | Crotyl | O | " | |
| H | Cl | H | Propargyl | O | " | |
| H | Cl | H | 1-Butin-3-yl | O | " | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 3-Methyl-1-butin-3-yl | O | 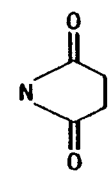 | |
| H | Cl | H | 2-Pentin-4-yl | O | " | |
| H | Cl | H | Benzyl | O | " | |
| H | Cl | H | 2-Phenylethyl | O | " | |
| H | Cl | H | 2-Methylthioethyl | O | " | |
| H | Cl | H | 2-Chlorethyl | O | " | |
| H | Cl | H | 2-Methoxyethyl | O | " | |
| H | Cl | H | 2-(N,N-Dimethylamino)ethyl | O | " | |
| H | Cl | H | Phenyl | O | " | |
| H | Cl | H | 2-$CH_3$-Phenyl | O | " | |
| H | Cl | H | 3-$CH_3$-Phenyl | O | " | |
| H | Cl | H | 4-$CH_3$-Phenyl | O | " | |
| H | Cl | H | 2,4-($CH_3$,$CH_3$)-Phenyl | O | " | |
| H | Cl | H | 2,3,5-($CH_3$,$CH_3$,$CH_3$)-Phenyl | O | " | |
| H | Cl | H | 3-$CF_3$-Phenyl | O | " | |
| H | Cl | H | 3-F-Phenyl | O | " | |
| H | Cl | H | 2-Cl-Phenyl | O | " | |
| H | Cl | H | 4-Cl-Phenyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2,4-(F,F)-Phenyl | O | 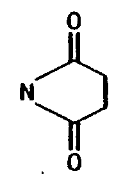 | |
| H | Cl | H | 2,3,5-(Cl,Cl,Cl)-Phenyl | O | " | |
| H | Cl | H | 2-CN-Phenyl | O | " | |
| H | Cl | H | 2-$OCH_3$-Phenyl | O | " | |
| H | Cl | H | 2,3-($OCH_3$,$OCH_3$)-Phenyl | O | " | |
| H | Cl | H | 3,4,5-($OCH_3$,$OCH_3$,$OCH_3$)-Phenyl | O | " | |
| H | Cl | H | 3-$OCF_3$-Phenyl | O | " | |
| H | Cl | H | 3-$OCF_2OHF_2$-Phenyl | O | " | |
| H | Cl | H | 4-$OCF_2CHF_2$-Phenyl | O | " | |
| H | Cl | H | 2-$SCH_3$-Phenyl | O | " | |
| H | Cl | H | 2,4-($SCH_3$,$SCH_3$)-Phenyl | O | " | |
| H | Cl | H | 2-$SCF_3$-Phenyl | O | " | |
| H | Cl | H | 4-$NO_2$-Phenyl | O | " | |
| H | Cl | H | 2,4-($NO_2$,$NO_2$)-Phenyl | O | " | |
| H | Cl | H | 3-$COCH_3$-Phenyl | O | " | |
| H | Cl | H | 3-$COCF_3$-Phenyl | O | " | |
| H | Cl | H | 1-Naphthyl | O | " | |
| H | Cl | H | 2-Naphthyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | Piperidino | O | 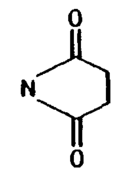 | |
| H | Cl | H | 3-Tetrahydrofuranyl | O | " | |
| H | Cl | H | 4-Tetrahydropyranyl | O | " | |
| H | Cl | H | 2-Thiazolyl | O | " | |
| H | Cl | H | 5-$CH_3$-2-Thiazolyl | O | " | |
| H | Cl | H | 4-$CH_3$-5-COOH-2-Thiazolyl | O | " | |
| H | Cl | H | Methyl | O | $N=C(CH_3)_2$ | |
| H | Cl | H | Ethyl | O | $N=C(CH_3)_2$ | |
| H | Cl | H | n-Propyl | O | $N=C(CH_3)_2$ | |
| H | Cl | H | iso-Propyl | O | $N=C(CH_3)_2$ | |
| H | Cl | H | Cyclopropyl | O | $N=C(CH_3)_2$ | |
| H | Cl | H | n-Butyl | O | $N=C(CH_3)_2$ | |
| H | Cl | H | iso-Butyl | O | $N=C(CH_3)_2$ | |
| H | Cl | H | sek.-Butyl | O | $N=C(CH_3)_2$ | |
| H | Cl | H | tert.-Butyl | O | $N=C(CH_3)_2$ | |
| H | Cl | H | n-Pentyl | O | $N=C(CH_3)_2$ | |
| H | Cl | H | 2-Pentyl | O | $N=C(CH_3)_2$ | |
| H | Cl | H | 3-Pentyl | O | $N=C(CH_3)_2$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | n-Hexyl | O | $N=C(CH_3)_2$ | |
| H | Cl | H | 2-Hexyl | O | $N=C(CH_3)_2$ | |
| H | Cl | H | 3-Hexyl | O | $N=C(CH_3)_2$ | |
| H | Cl | H | 2-Methyl-2-pentyl | O | $N=C(CH_3)_2$ | |
| H | Cl | H | cyclo-Propylmethyl | O | $N=C(CH_3)_2$ | |
| H | Cl | H | cyclo-Butyl | O | $N=C(CH_3)_2$ | |
| H | Cl | H | cyclo-Pentyl | O | $N=C(CH_3)_2$ | |
| H | Cl | H | cyclo-Hexyl | O | $N=C(CH_3)_2$ | |
| H | Cl | H | 1-Methylcyclohexyl | O | $N=C(CH_3)_2$ | |
| H | Cl | H | 3-Triflormethylcyclohexyl | O | $N=C(CH_3)_2$ | |
| H | Cl | H | Allyl | O | $N=C(CH_3)_2$ | |
| H | Cl | H | 1-Buten-3-yl | O | $N=C(CH_3)_2$ | |
| H | Cl | H | Crotyl | O | $N=C(CH_3)_2$ | |
| H | Cl | H | Propargyl | O | $N=C(CH_3)_2$ | |
| H | Cl | H | 1-Butin-3-yl | O | $N=C(CH_3)_2$ | |
| H | Cl | H | 3-Methyl-1-butin-3-yl | O | $N=C(CH_3)_2$ | |
| H | Cl | H | 2-Pentin-4-yl | O | $N=C(CH_3)_2$ | |
| H | Cl | H | Benzyl | O | $N=C(CH_3)_2$ | |
| H | Cl | H | 2-Phenylethyl | O | $N=C(CH_3)_2$ | |
| H | Cl | H | 2-Methylthioethyl | O | $N=C(CH_3)_2$ | |
| H | Cl | H | 2-Chlorethyl | O | $N=C(CH_3)_2$ | |
| H | Cl | H | 2-Methoxyethyl | O | $N=C(CH_3)_2$ | |
| H | Cl | H | 2-(N,N-Dimethylamino)ethyl | O | $N=C(CH_3)_2$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | Phenyl | O | $N=C(CH_3)_2$ | |
| H | Cl | H | $2-CH_3$-Phenyl | O | $N=C(CH_3)_2$ | |
| H | Cl | H | $3-CH_3$-Phenyl | O | $N=C(CH_3)_2$ | |
| H | Cl | H | $4-CH_3$-Phenyl | O | $N=C(CH_3)_2$ | |
| H | Cl | H | $2,4-(CH_3,CH_3)$-Phenyl | O | $N=C(CH_3)_2$ | |
| H | Cl | H | $2,3,5-(CH_3,CH_3,CH_3)$-Phenyl | O | $N=C(CH_3)_2$ | |
| H | Cl | H | $3-CF_3$-Phenyl | O | $N=C(CH_3)_2$ | |
| H | Cl | H | 3-F-Phenyl | O | $N=C(CH_3)_2$ | |
| H | Cl | H | 2-Cl-Phenyl | O | $N=C(CH_3)_2$ | |
| H | Cl | H | 4-Cl-Phenyl | O | $N=C(CH_3)_2$ | |
| H | Cl | H | 2,4-(F,F)-Phenyl | O | $N=C(CH_3)_2$ | |
| H | Cl | H | 2,3,5-(Cl,Cl,Cl)-Phenyl | O | $N=C(CH_3)_2$ | |
| H | Cl | H | 2-CN-Phenyl | O | $N=C(CH_3)_2$ | |
| H | Cl | H | $2-OCH_3$-Phenyl | O | $N=C(CH_3)_2$ | |
| H | Cl | H | $2,3-(OCH_3,OCH_3)$-Phenyl | O | $N=C(CH_3)_2$ | |
| H | Cl | H | $3,4,5-(OCH_3,OCH_3,OCH_3)$-Phenyl | O | $N=C(CH_3)_2$ | |
| H | Cl | H | $3-OCF_3$-Phenyl | O | $N=C(CH_3)_2$ | |
| H | Cl | H | $3-OCF_2OHF_2$-Phenyl | O | $N=C(CH_3)_2$ | |
| H | Cl | H | $4-OCF_2CHF_2$-Phenyl | O | $N=C(CH_3)_2$ | |
| H | Cl | H | $2-SCH_3$-Phenyl | O | $N=C(CH_3)_2$ | |
| H | Cl | H | $2,4-(SCH_3,SCH_3)$-Phenyl | O | $N=C(CH_3)_2$ | |
| H | Cl | H | $2-SCF_3$-Phenyl | O | $N=C(CH_3)_2$ | |
| H | Cl | H | $4-NO_2$-Phenyl | O | $N=C(CH_3)_2$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2,4-($NO_2$,$NO_2$)-Phenyl | O | $N=C(CH_3)_2$ | |
| H | Cl | H | 3-$COCH_3$-Phenyl | O | $N=C(CH_3)_2$ | |
| H | Cl | H | 3-$COCF_3$-Phenyl | O | $N=C(CH_3)_2$ | |
| H | Cl | H | 1-Naphthyl | O | $N=C(CH_3)_2$ | |
| H | Cl | H | 2-Naphthyl | O | $N=C(CH_3)_2$ | |
| H | Cl | H | Piperidino | O | $N=C(CH_3)_2$ | |
| H | Cl | H | 3-Tetrahydrofuranyl | O | $N=C(CH_3)_2$ | |
| H | Cl | H | 4-Tetrahydropyranyl | O | $N=C(CH_3)_2$ | |
| H | Cl | H | 2-Thiazolyl | O | $N=C(CH_3)_2$ | |
| H | Cl | H | 5-$CH_3$-2-Thiazolyl | O | $N=C(CH_3)_2$ | |
| H | Cl | H | 4-$CH_3$-5-COOH-2-Thiazolyl | O | $N=C(CH_3)_2$ | |
| H | $OCH_3$ | H | Methyl | O | 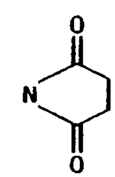 | |
| H | $OCH_3$ | H | Ethyl | O | " | |
| H | $OCH_3$ | H | n-Propyl | O | " | |
| H | $OCH_3$ | H | iso-Propyl | O | " | |
| H | $OCH_3$ | H | Cyclopropyl | O | " | |
| H | $OCH_3$ | H | n-Butyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | iso-Butyl | O | 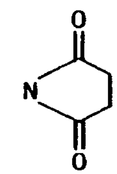 | |
| H | $OCH_3$ | H | sek.-Butyl | O | " | |
| H | $OCH_3$ | H | tert.-Butyl | O | " | |
| H | $OCH_3$ | H | n-Pentyl | O | " | |
| H | $OCH_3$ | H | 2-Pentyl | O | " | |
| H | $OCH_3$ | H | 3-Pentyl | O | " | |
| H | $OCH_3$ | H | n-Hexyl | O | " | |
| H | $OCH_3$ | H | 2-Hexyl | O | " | |
| H | $OCH_3$ | H | 3-Hexyl | O | " | |
| H | $OCH_3$ | H | 2-Methyl-2-pentyl | O | " | |
| H | $OCH_3$ | H | cyclo-Propylmethyl | O | " | |
| H | $OCH_3$ | H | cyclo-Butyl | O | " | |
| H | $OCH_3$ | H | cyclo-Pentyl | O | " | |
| H | $OCH_3$ | H | cyclo-Hexyl | O | " | |
| H | $OCH_3$ | H | 1-Methylcyclohexyl | O | " | |
| H | $OCH_3$ | H | 3-Triflormethylcyclohexyl | O | " | |
| H | $OCH_3$ | H | Allyl | O | " | |
| H | $OCH_3$ | H | 1-Buten-3-yl | O | " | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | Crotyl | O | 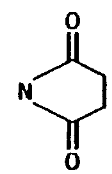 | |
| H | $OCH_3$ | H | Propargyl | O | " | |
| H | $OCH_3$ | H | 1-Butin-3-yl | O | " | |
| H | $OCH_3$ | H | 3-Methyl-1-butin-3-yl | O | " | |
| H | $OCH_3$ | H | 2-Pentin-4-yl | O | " | |
| H | $OCH_3$ | H | Benzyl | O | " | |
| H | $OCH_3$ | H | 2-Phenylethyl | O | " | |
| H | $OCH_3$ | H | 2-Methylthioethyl | O | " | |
| H | $OCH_3$ | H | 2-Chlorethyl | O | " | |
| H | $OCH_3$ | H | 2-Methoxyethyl | O | " | |
| H | $OCH_3$ | H | 2-(N,N-Dimethylamino)ethyl | O | " | |
| H | $OCH_3$ | H | Phenyl | O | " | |
| H | $OCH_3$ | H | 2-$CH_3$-Phenyl | O | " | |
| H | $OCH_3$ | H | 3-$CH_3$-Phenyl | O | " | |
| H | $OCH_3$ | H | 4-$CH_3$-Phenyl | O | " | |
| H | $OCH_3$ | H | 2,4-($CH_3$,$CH_3$)-Phenyl | O | " | |
| H | $OCH_3$ | H | 2,3,5-($CH_3$,$CH_3$,$CH_3$)-Phenyl | O | " | |
| H | $OCH_3$ | H | 3-$CF_3$-Phenyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | 3-F-Phenyl | O | 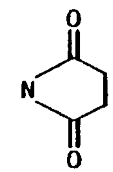 | |
| H | $OCH_3$ | H | 2-Cl-Phenyl | O | " | |
| H | $OCH_3$ | H | 4-Cl-Phenyl | O | " | |
| H | $OCH_3$ | H | 2,4-(F,F)-Phenyl | O | " | |
| H | $OCH_3$ | H | 2,3,5-(Cl,Cl,Cl)-Phenyl | O | " | |
| H | $OCH_3$ | H | 2-CN-Phenyl | O | " | |
| H | $OCH_3$ | H | 2-$OCH_3$-Phenyl | O | " | |
| H | $OCH_3$ | H | 2,3-($OCH_3$,$OCH_3$)-Phenyl | O | " | |
| H | $OCH_3$ | H | 3,4,5-($OCH_3$,$OCH_3$,$OCH_3$)-Phenyl | O | " | |
| H | $OCH_3$ | H | 3-$OCF_3$-Phenyl | O | " | |
| H | $OCH_3$ | H | 3-$OCF_2OHF_2$-Phenyl | O | " | |
| H | $OCH_3$ | H | 4-$OCF_2CHF_2$-Phenyl | O | " | |
| H | $OCH_3$ | H | 2-$SCH_3$-Phenyl | O | " | |
| H | $OCH_3$ | H | 2,4-($SCH_3$,$SCH_3$)-Phenyl | O | " | |
| H | $OCH_3$ | H | 2-$SCF_3$-Phenyl | O | " | |
| H | $OCH_3$ | H | 4-$NO_2$-Phenyl | O | " | |
| H | $OCH_3$ | H | 2,4-($NO_2$,$NO_2$)-Phenyl | O | " | |
| H | $OCH_3$ | H | 3-$COCH_3$-Phenyl | O | " | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | 3-$COCF_3$-Phenyl | O | 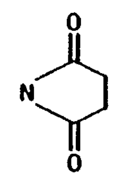 | |
| H | $OCH_3$ | H | 1-Naphthyl | O | " | |
| H | $OCH_3$ | H | 2-Naphthyl | O | " | |
| H | $OCH_3$ | H | Piperidino | O | " | |
| H | $OCH_3$ | H | 3-Tetrahydrofuranyl | O | " | |
| H | $OCH_3$ | H | 4-Tetrahydropyranyl | O | " | |
| H | $OCH_3$ | H | 2-Thiazolyl | O | " | |
| H | $OCH_3$ | H | 5-$CH_3$-2-Thiazolyl | O | " | |
| H | $OCH_3$ | H | 4-$CH_3$-5-COOH-2-Thiazolyl | O | " | |
| H | $OCH_3$ | H | Methyl | O | $N=C(CH_3)_2$ | |
| H | $OCH_3$ | H | Ethyl | O | $N=C(CH_3)_2$ | |
| H | $OCH_3$ | H | n-Propyl | O | $N=C(CH_3)_2$ | |
| H | $OCH_3$ | H | iso-Propyl | O | $N=C(CH_3)_2$ | |
| H | $OCH_3$ | H | Cyclopropyl | O | $N=C(CH_3)_2$ | |
| H | $OCH_3$ | H | n-Butyl | O | $N=C(CH_3)_2$ | |
| H | $OCH_3$ | H | iso-Butyl | O | $N=C(CH_3)_2$ | |
| H | $OCH_3$ | H | sek.-Butyl | O | $N=C(CH_3)_2$ | |
| H | $OCH_3$ | H | tert.-Butyl | O | $N=C(CH_3)_2$ | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | n-Pentyl | O | $N=C(CH_3)_2$ | |
| H | $OCH_3$ | H | 2-Pentyl | O | $N=C(CH_3)_2$ | |
| H | $OCH_3$ | H | 3-Pentyl | O | $N=C(CH_3)_2$ | |
| H | $OCH_3$ | H | n-Hexyl | O | $N=C(CH_3)_2$ | |
| H | $OCH_3$ | H | 2-Hexyl | O | $N=C(CH_3)_2$ | |
| H | $OCH_3$ | H | 3-Hexyl | O | $N=C(CH_3)_2$ | |
| H | $OCH_3$ | H | 2-Methyl-2-pentyl | O | $N=C(CH_3)_2$ | |
| H | $OCH_3$ | H | cyclo-Propylmethyl | O | $N=C(CH_3)_2$ | |
| H | $OCH_3$ | H | cyclo-Butyl | O | $N=C(CH_3)_2$ | |
| H | $OCH_3$ | H | cyclo-Pentyl | O | $N=C(CH_3)_2$ | |
| H | $OCH_3$ | H | cyclo-Hexyl | O | $N=C(CH_3)_2$ | |
| H | $OCH_3$ | H | 1-Methylcyclohexyl | O | $N=C(CH_3)_2$ | |
| H | $OCH_3$ | H | 3-Triflormethylcyclohexyl | O | $N=C(CH_3)_2$ | |
| H | $OCH_3$ | H | Allyl | O | $N=C(CH_3)_2$ | |
| H | $OCH_3$ | H | 1-Buten-3-yl | O | $N=C(CH_3)_2$ | |
| H | $OCH_3$ | H | Crotyl | O | $N=C(CH_3)_2$ | |
| H | $OCH_3$ | H | Propargyl | O | $N=C(CH_3)_2$ | |
| H | $OCH_3$ | H | 1-Butin-3-yl | O | $N=C(CH_3)_2$ | |
| H | $OCH_3$ | H | 3-Methyl-1-butin-3-yl | O | $N=C(CH_3)_2$ | |
| H | $OCH_3$ | H | 2-Pentin-4-yl | O | $N=C(CH_3)_2$ | |
| H | $OCH_3$ | H | Benzyl | O | $N=C(CH_3)_2$ | |
| H | $OCH_3$ | H | 2-Phenylethyl | O | $N=C(CH_3)_2$ | |
| H | $OCH_3$ | H | 2-Methylthioethyl | O | $N=C(CH_3)_2$ | |

Tabelle (Fortsetzung)

| $R^3$ | $R^4$ | $R^1$ | $R^2$ | Y | $R^6$ | X = O oder S |
|---|---|---|---|---|---|---|
| H | $OCH_3$ | H | 2-Chlorethyl | O | $N=C(CH_3)_2$ | |
| H | $OCH_3$ | H | 2-Methoxyethyl | O | $N=C(CH_3)_2$ | |
| H | $OCH_3$ | H | 2-(N,N-Dimethylamino)ethyl | O | $N=C(CH_3)_2$ | |
| H | $OCH_3$ | H | Phenyl | O | $N=C(CH_3)_2$ | |
| H | $OCH_3$ | H | 2-$CH_3$-Phenyl | O | $N=C(CH_3)_2$ | |
| H | $OCH_3$ | H | 3-$CH_3$-Phenyl | O | $N=C(CH_3)_2$ | |
| H | $OCH_3$ | H | 4-$CH_3$-Phenyl | O | $N=C(CH_3)_2$ | |
| H | $OCH_3$ | H | 2,4-($CH_3$,$CH_3$)-Phenyl | O | $N=C(CH_3)_2$ | |
| H | $OCH_3$ | H | 2,3,5-($CH_3$,$CH_3$,$CH_3$)-Phenyl | O | $N=C(CH_3)_2$ | |
| H | $OCH_3$ | H | 3-$CF_3$-Phenyl | O | $N=C(CH_3)_2$ | |
| H | $OCH_3$ | H | 3-F-Phenyl | O | $N=C(CH_3)_2$ | |
| H | $OCH_3$ | H | 2-Cl-Phenyl | O | $N=C(CH_3)_2$ | |
| H | $OCH_3$ | H | 4-Cl-Phenyl | O | $N=C(CH_3)_2$ | |
| H | $OCH_3$ | H | 2,4-(F,F)-Phenyl | O | $N=C(CH_3)_2$ | |
| H | $OCH_3$ | H | 2,3,5-(Cl,Cl,Cl)-Phenyl | O | $N=C(CH_3)_2$ | |
| H | $OCH_3$ | H | 2-CN-Phenyl | O | $N=C(CH_3)_2$ | |
| H | $OCH_3$ | H | 2-$OCH_3$-Phenyl | O | $N=C(CH_3)_2$ | |
| H | $OCH_3$ | H | 2,3-($OCH_3$,$OCH_3$)-Phenyl | O | $N=C(CH_3)_2$ | |
| H | $OCH_3$ | H | 3,4,5-($OCH_3$,$OCH_3$,$OCH_3$)-Phenyl | O | $N=C(CH_3)_2$ | |
| H | $OCH_3$ | H | 3-$OCF_3$-Phenyl | O | $N=C(CH_3)_2$ | |
| H | $OCH_3$ | H | 3-$OCF_2OHF_2$-Phenyl | O | $N=C(CH_3)_2$ | |
| H | $OCH_3$ | H | 4-$OCF_2CHF_2$-Phenyl | O | $N=C(CH_3)_2$ | |
| H | $OCH_3$ | H | 2-$SCH_3$-Phenyl | O | $N=C(CH_3)_2$ | |

Tabelle (Fortsetzung)

| $R^3$ | $R^4$ | $R^1$ | $R^2$ | Y | $R^6$ | X = 0 oder S |
|---|---|---|---|---|---|---|
| H | $OCH_3$ | H | 2,4-($SCH_3$,$SCH_3$)-Phenyl | O | $N=C(CH_3)_2$ | |
| H | $OCH_3$ | H | 2-$SCF_3$-Phenyl | O | $N=C(CH_3)_2$ | |
| H | $OCH_3$ | H | 4-$NO_2$-Phenyl | O | $N=C(CH_3)_2$ | |
| H | $OCH_3$ | H | 2,4-($NO_2$,$NO_2$)-Phenyl | O | $N=C(CH_3)_2$ | |
| H | $OCH_3$ | H | 3-$COCH_3$-Phenyl | O | $N=C(CH_3)_2$ | |
| H | $OCH_3$ | H | 3-$COCF_3$-Phenyl | O | $N=C(CH_3)_2$ | |
| H | $OCH_3$ | H | 1-Naphthyl | O | $N=C(CH_3)_2$ | |
| H | $OCH_3$ | H | 2-Naphthyl | O | $N=C(CH_3)_2$ | |
| H | $OCH_3$ | H | Piperidino | O | $N=C(CH_3)_2$ | |
| H | $OCH_3$ | H | 3-Tetrahydrofuranyl | O | $N=C(CH_3)_2$ | |
| H | $OCH_3$ | H | 4-Tetrahydropyranyl | O | $N=C(CH_3)_2$ | |
| H | $OCH_3$ | H | 2-Thiazolyl | O | $N=C(CH_3)_2$ | |
| H | $OCH_3$ | H | 5-$CH_3$-2-Thiazolyl | O | $N=C(CH_3)_2$ | |
| H | $OCH_3$ | H | 4-$CH_3$-5-COOH-2-Thiazolyl | O | $N=C(CH_3)_2$ | |
| Br | H | $CH_3$ | Methyl | O | H | |
| Br | H | $CH_3$ | Ethyl | O | H | |
| Br | H | $CH_3$ | n-Propyl | O | H | |
| Br | H | $CH_3$ | iso-Propyl | O | H | |
| Br | H | $CH_3$ | Cyclopropyl | O | H | |
| Br | H | $CH_3$ | n-Butyl | O | H | |
| Br | H | $CH_3$ | iso-Butyl | O | H | |
| Br | H | $CH_3$ | sek.-Butyl | O | H | |
| Br | H | $CH_3$ | tert.-Butyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | H | $CH_3$ | n-Pentyl | O | H | |
| Br | H | $CH_3$ | 2-Pentyl | O | H | |
| Br | H | $CH_3$ | 3-Pentyl | O | H | |
| Br | H | $CH_3$ | n-Hexyl | O | H | |
| Br | H | $CH_3$ | 2-Hexyl | O | H | |
| Br | H | $CH_3$ | 3-Hexyl | O | H | |
| Br | H | $CH_3$ | 2-Methyl-2-pentyl | O | H | |
| Br | H | $CH_3$ | cyclo-Propylmethyl | O | H | |
| Br | H | $CH_3$ | cyclo-Butyl | O | H | |
| Br | H | $CH_3$ | cyclo-Pentyl | O | H | |
| Br | H | $CH_3$ | cyclo-Hexyl | O | H | |
| Br | H | $CH_3$ | 1-Methylcyclohexyl | O | H | |
| Br | H | $CH_3$ | 3-Triflormethylcyclohexyl | O | H | |
| Br | H | $CH_3$ | Allyl | O | H | |
| Br | H | $CH_3$ | 1-Buten-3-yl | O | H | |
| Br | H | $CH_3$ | Crotyl | O | H | |
| Br | H | $CH_3$ | Propargyl | O | H | |
| Br | H | $CH_3$ | 1-Butin-3-yl | O | H | |
| Br | H | $CH_3$ | 3-Methyl-1-butin-3-yl | O | H | |
| Br | H | $CH_3$ | 2-Pentin-4-yl | O | H | |
| Br | H | $CH_3$ | Benzyl | O | H | |
| Br | H | $CH_3$ | 2-Phenylethyl | O | H | |
| Br | H | $CH_3$ | 2-Methylthioethyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | H | $CH_3$ | 2-Chlorethyl | O | H | |
| Br | H | $CH_3$ | 2-Methoxyethyl | O | H | |
| Br | H | $CH_3$ | 2-(N,N-Dimethylamino)ethyl | O | H | |
| Br | H | $CH_3$ | Phenyl | O | H | |
| Br | H | $CH_3$ | 2-$CH_3$-Phenyl | O | H | |
| Br | H | $CH_3$ | 3-$CH_3$-Phenyl | O | H | |
| Br | H | $CH_3$ | 4-$CH_3$-Phenyl | O | H | |
| Br | H | $CH_3$ | 2,4-($CH_3$,$CH_3$)-Phenyl | O | H | |
| Br | H | $CH_3$ | 2,3,5-($CH_3$,$CH_3$,$CH_3$)-Phenyl | O | H | |
| Br | H | $CH_3$ | 3-$CF_3$-Phenyl | O | H | |
| Br | H | $CH_3$ | 3-F-Phenyl | O | H | |
| Br | H | $CH_3$ | 2-Cl-Phenyl | O | H | |
| Br | H | $CH_3$ | 4-Cl-Phenyl | O | H | |
| Br | H | $CH_3$ | 2,4-(F,F)-Phenyl | O | H | |
| Br | H | $CH_3$ | 2,3,5-(Cl,Cl,Cl)-Phenyl | O | H | |
| Br | H | $CH_3$ | 2-CN-Phenyl | O | H | |
| Br | H | $CH_3$ | 2-$OCH_3$-Phenyl | O | H | |
| Br | H | $CH_3$ | 2,3-($OCH_3$,$OCH_3$)-Phenyl | O | H | |
| Br | H | $CH_3$ | 3,4,5-($OCH_3$,$OCH_3$,$OCH_3$)-Phenyl | O | H | |
| Br | H | $CH_3$ | 3-$OCF_3$-Phenyl | O | H | |
| Br | H | $CH_3$ | 3-$OCF_2OHF_2$-Phenyl | O | H | |
| Br | H | $CH_3$ | 4-$OCF_2CHF_2$-Phenyl | O | H | |
| Br | H | $CH_3$ | 2-$SCH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| Br | H | $CH_3$ | 2,4-($SCH_3$,$SCH_3$)-Phenyl | O | H | |
| Br | H | $CH_3$ | 2-$SCF_3$-Phenyl | O | H | |
| Br | H | $CH_3$ | 4-$NO_2$-Phenyl | O | H | |
| Br | H | $CH_3$ | 2,4-($NO_2$,$NO_2$)-Phenyl | O | H | |
| Br | H | $CH_3$ | 3-$COCH_3$-Phenyl | O | H | |
| Br | H | $CH_3$ | 3-$COCF_3$-Phenyl | O | H | |
| Br | H | $CH_3$ | 1-Naphthyl | O | H | |
| Br | H | $CH_3$ | 2-Naphthyl | O | H | |
| Br | H | $CH_3$ | Piperidino | O | H | |
| Br | H | $CH_3$ | 3-Tetrahydrofuranyl | O | H | |
| Br | H | $CH_3$ | 4-Tetrahydropyranyl | O | H | |
| Br | H | $CH_3$ | 2-Thiazolyl | O | H | |
| Br | H | $CH_3$ | 5-$CH_3$-2-Thiazolyl | O | H | |
| Br | H | $CH_3$ | 4-$CH_3$-5-COOH-2-Thiazolyl | O | H | |
| $CH_3$ | $CH_3$ | Allyl | Methyl | O | H | |
| $CH_3$ | $CH_3$ | Allyl | Ethyl | O | H | |
| $CH_3$ | $CH_3$ | Allyl | n-Propyl | O | H | |
| $CH_3$ | $CH_3$ | Allyl | iso-Propyl | O | H | |
| $CH_3$ | $CH_3$ | Allyl | Cyclopropyl | O | H | |
| $CH_3$ | $CH_3$ | Allyl | n-Butyl | O | H | |
| $CH_3$ | $CH_3$ | Allyl | iso-Butyl | O | H | |
| $CH_3$ | $CH_3$ | Allyl | sek.-Butyl | O | H | |
| $CH_3$ | $CH_3$ | Allyl | tert.-Butyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | Allyl | n-Pentyl | O | H | |
| $CH_3$ | $CH_3$ | Allyl | 2-Pentyl | O | H | |
| $CH_3$ | $CH_3$ | Allyl | 3-Pentyl | O | H | |
| $CH_3$ | $CH_3$ | Allyl | n-Hexyl | O | H | |
| $CH_3$ | $CH_3$ | Allyl | 2-Hexyl | O | H | |
| $CH_3$ | $CH_3$ | Allyl | 3-Hexyl | O | H | |
| $CH_3$ | $CH_3$ | Allyl | 2-Methyl-2-pentyl | O | H | |
| $CH_3$ | $CH_3$ | Allyl | cyclo-Propylmethyl | O | H | |
| $CH_3$ | $CH_3$ | Allyl | cyclo-Butyl | O | H | |
| $CH_3$ | $CH_3$ | Allyl | cyclo-Pentyl | O | H | |
| $CH_3$ | $CH_3$ | Allyl | cyclo-Hexyl | O | H | |
| $CH_3$ | $CH_3$ | Allyl | 1-Methylcyclohexyl | O | H | |
| $CH_3$ | $CH_3$ | Allyl | 3-Triflormethylcyclohexyl | O | H | |
| $CH_3$ | $CH_3$ | Allyl | Allyl | O | H | |
| $CH_3$ | $CH_3$ | Allyl | 1-Buten-3-yl | O | H | |
| $CH_3$ | $CH_3$ | Allyl | Crotyl | O | H | |
| $CH_3$ | $CH_3$ | Allyl | Propargyl | O | H | |
| $CH_3$ | $CH_3$ | Allyl | 1-Butin-3-yl | O | H | |
| $CH_3$ | $CH_3$ | Allyl | 3-Methyl-1-butin-3-yl | O | H | |
| $CH_3$ | $CH_3$ | Allyl | 2-Pentin-4-yl | O | H | |
| $CH_3$ | $CH_3$ | Allyl | Benzyl | O | H | |
| $CH_3$ | $CH_3$ | Allyl | 2-Phenylethyl | O | H | |
| $CH_3$ | $CH_3$ | Allyl | 2-Methylthioethyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | Allyl | 2-Chlorethyl | O | H | |
| $CH_3$ | $CH_3$ | Allyl | 2-Methoxyethyl | O | H | |
| $CH_3$ | $CH_3$ | Allyl | 2-(N,N-Dimethylamino)ethyl | O | H | |
| $CH_3$ | $CH_3$ | Allyl | Phenyl | O | H | |
| $CH_3$ | $CH_3$ | Allyl | 2-$CH_3$-Phenyl | O | H | |
| $CH_3$ | $CH_3$ | Allyl | 3-$CH_3$-Phenyl | O | H | |
| $CH_3$ | $CH_3$ | Allyl | 4-$CH_3$-Phenyl | O | H | |
| $CH_3$ | $CH_3$ | Allyl | 2,4-($CH_3$,$CH_3$)-Phenyl | O | H | |
| $CH_3$ | $CH_3$ | Allyl | 2,3,5-($CH_3$,$CH_3$,$CH_3$)-Phenyl | O | H | |
| $CH_3$ | $CH_3$ | Allyl | 3-$CF_3$-Phenyl | O | H | |
| $CH_3$ | $CH_3$ | Allyl | 3-F-Phenyl | O | H | |
| $CH_3$ | $CH_3$ | Allyl | 2-Cl-Phenyl | O | H | |
| $CH_3$ | $CH_3$ | Allyl | 4-Cl-Phenyl | O | H | |
| $CH_3$ | $CH_3$ | Allyl | 2,4-(F,F)-Phenyl | O | H | |
| $CH_3$ | $CH_3$ | Allyl | 2,3,5-(Cl,Cl,Cl)-Phenyl | O | H | |
| $CH_3$ | $CH_3$ | Allyl | 2-CN-Phenyl | O | H | |
| $CH_3$ | $CH_3$ | Allyl | 2-$OCH_3$-Phenyl | O | H | |
| $CH_3$ | $CH_3$ | Allyl | 2,3-($OCH_3$,$OCH_3$)-Phenyl | O | H | |
| $CH_3$ | $CH_3$ | Allyl | 3,4,5-($OCH_3$,$OCH_3$,$OCH_3$)-Phenyl | O | H | |
| $CH_3$ | $CH_3$ | Allyl | 3-$OCF_3$-Phenyl | O | H | |
| $CH_3$ | $CH_3$ | Allyl | 3-$OCF_2OHF_2$-Phenyl | O | H | |
| $CH_3$ | $CH_3$ | Allyl | 4-$OCF_2CHF_2$-Phenyl | O | H | |
| $CH_3$ | $CH_3$ | Allyl | 2-$SCH_3$-Phenyl | O | H | |

Tabelle (Fortsetzung)

| $R^3$ | $R^4$ | $R^1$ | $R^2$ | Y | $R^6$ | X = O oder S |
|---|---|---|---|---|---|---|
| $CH_3$ | $CH_3$ | Allyl | 2,4-($SCH_3$,$SCH_3$)-Phenyl | O | H | |
| $CH_3$ | $CH_3$ | Allyl | 2-$SCF_3$-Phenyl | O | H | |
| $CH_3$ | $CH_3$ | Allyl | 4-$NO_2$-Phenyl | O | H | |
| $CH_3$ | $CH_3$ | Allyl | 2,4-($NO_2$,$NO_2$)-Phenyl | O | H | |
| $CH_3$ | $CH_3$ | Allyl | 3-$COCH_3$-Phenyl | O | H | |
| $CH_3$ | $CH_3$ | Allyl | 3-$COCF_3$-Phenyl | O | H | |
| $CH_3$ | $CH_3$ | Allyl | 1-Naphthyl | O | H | |
| $CH_3$ | $CH_3$ | Allyl | 2-Naphthyl | O | H | |
| $CH_3$ | $CH_3$ | Allyl | Piperidino | O | H | |
| $CH_3$ | $CH_3$ | Allyl | 3-Tetrahydrofuranyl | O | H | |
| $CH_3$ | $CH_3$ | Allyl | 4-Tetrahydropyranyl | O | H | |
| $CH_3$ | $CH_3$ | Allyl | 2-Thiazolyl | O | H | |
| $CH_3$ | $CH_3$ | Allyl | 5-$CH_3$-2-Thiazolyl | O | H | |
| $CH_3$ | $CH_3$ | Allyl | 4-$CH_3$-5-COOH-2-Thiazolyl | O | H | |
| H | Cl | H | Methyl | S | $CH_3$ | |
| H | Cl | H | Ethyl | S | $CH_3$ | |
| H | Cl | H | n-Propyl | S | $CH_3$ | |
| H | Cl | H | iso-Propyl | S | $CH_3$ | |
| H | Cl | H | Cyclopropyl | S | $CH_3$ | |
| H | Cl | H | n-Butyl | S | $CH_3$ | |
| H | Cl | H | iso-Butyl | S | $CH_3$ | |
| H | Cl | H | sek.-Butyl | S | $CH_3$ | |
| H | Cl | H | tert.-Butyl | S | $CH_3$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | n-Pentyl | S | $CH_3$ | |
| H | Cl | H | 2-Pentyl | S | $CH_3$ | |
| H | Cl | H | 3-Pentyl | S | $CH_3$ | |
| H | Cl | H | n-Hexyl | S | $CH_3$ | |
| H | Cl | H | 2-Hexyl | S | $CH_3$ | |
| H | Cl | H | 3-Hexyl | S | $CH_3$ | |
| H | Cl | H | 2-Methyl-2-pentyl | S | $CH_3$ | |
| H | Cl | H | cyclo-Propylmethyl | S | $CH_3$ | |
| H | Cl | H | cyclo-Butyl | S | $CH_3$ | |
| H | Cl | H | cyclo-Pentyl | S | $CH_3$ | |
| H | Cl | H | cyclo-Hexyl | S | $CH_3$ | |
| H | Cl | H | 1-Methylcyclohexyl | S | $CH_3$ | |
| H | Cl | H | 3-Triflormethylcyclohexyl | S | $CH_3$ | |
| H | Cl | H | Allyl | S | $CH_3$ | |
| H | Cl | H | 1-Buten-3-yl | S | $CH_3$ | |
| H | Cl | H | Crotyl | S | $CH_3$ | |
| H | Cl | H | Propargyl | S | $CH_3$ | |
| H | Cl | H | 1-Butin-3-yl | S | $CH_3$ | |
| H | Cl | H | 3-Methyl-1-butin-3-yl | S | $CH_3$ | |
| H | Cl | H | 2-Pentin-4-yl | S | $CH_3$ | |
| H | Cl | H | Benzyl | S | $CH_3$ | |
| H | Cl | H | 2-Phenylethyl | S | $CH_3$ | |
| H | Cl | H | 2-Methylthioethyl | S | $CH_3$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2-Chlorethyl | S | $CH_3$ | |
| H | Cl | H | 2-Methoxyethyl | S | $CH_3$ | |
| H | Cl | H | 2-(N,N-Dimethylamino)ethyl | S | $CH_3$ | |
| H | Cl | H | Phenyl | S | $CH_3$ | |
| H | Cl | H | 2-$CH_3$-Phenyl | S | $CH_3$ | |
| H | Cl | H | 3-$CH_3$-Phenyl | S | $CH_3$ | |
| H | Cl | H | 4-$CH_3$-Phenyl | S | $CH_3$ | |
| H | Cl | H | 2,4-($CH_3$,$CH_3$)-Phenyl | S | $CH_3$ | |
| H | Cl | H | 2,3,5-($CH_3$,$CH_3$,$CH_3$)-Phenyl | S | $CH_3$ | |
| H | Cl | H | 3-$CF_3$-Phenyl | S | $CH_3$ | |
| H | Cl | H | 3-F-Phenyl | S | $CH_3$ | |
| H | Cl | H | 2-Cl-Phenyl | S | $CH_3$ | |
| H | Cl | H | 4-Cl-Phenyl | S | $CH_3$ | |
| H | Cl | H | 2,4-(F,F)-Phenyl | S | $CH_3$ | |
| H | Cl | H | 2,3,5-(Cl,Cl,Cl)-Phenyl | S | $CH_3$ | |
| H | Cl | H | 2-CN-Phenyl | S | $CH_3$ | |
| H | Cl | H | 2-$OCH_3$-Phenyl | S | $CH_3$ | |
| H | Cl | H | 2,3-($OCH_3$,$OCH_3$)-Phenyl | S | $CH_3$ | |
| H | Cl | H | 3,4,5-($OCH_3$,$OCH_3$,$OCH_3$)-Phenyl | S | $CH_3$ | |
| H | Cl | H | 3-$OCF_3$-Phenyl | S | $CH_3$ | |
| H | Cl | H | 3-$OCF_2OHF_2$-Phenyl | S | $CH_3$ | |
| H | Cl | H | 4-$OCF_2CHF_2$-Phenyl | S | $CH_3$ | |
| H | Cl | H | 2-$SCH_3$-Phenyl | S | $CH_3$ | |

Tabelle (Fortsetzung)

| $R^3$ | $R^4$ | $R^1$ | $R^2$ | Y | $R^6$ | X = O oder S |
|---|---|---|---|---|---|---|
| H | Cl | H | 2,4-($SCH_3$,$SCH_3$)-Phenyl | S | $CH_3$ | |
| H | Cl | H | 2-$SCF_3$-Phenyl | S | $CH_3$ | |
| H | Cl | H | 4-$NO_2$-Phenyl | S | $CH_3$ | |
| H | Cl | H | 2,4-($NO_2$,$NO_2$)-Phenyl | S | $CH_3$ | |
| H | Cl | H | 3-$COCH_3$-Phenyl | S | $CH_3$ | |
| H | Cl | H | 3-$COCF_3$-Phenyl | S | $CH_3$ | |
| H | Cl | H | 1-Naphthyl | S | $CH_3$ | |
| H | Cl | H | 2-Naphthyl | S | $CH_3$ | |
| H | Cl | H | Piperidino | S | $CH_3$ | |
| H | Cl | H | 3-Tetrahydrofuranyl | S | $CH_3$ | |
| H | Cl | H | 4-Tetrahydropyranyl | S | $CH_3$ | |
| H | Cl | H | 2-Thiazolyl | S | $CH_3$ | |
| H | Cl | H | 5-$CH_3$-2-Thiazolyl | S | $CH_3$ | |
| H | Cl | H | 4-$CH_3$-5-COOH-2-Thiazolyl | S | $CH_3$ | |
| H | $C_2H_5$ | H | Methyl | O | $CH_2C{\equiv}CH$ | |
| H | $C_2H_5$ | H | Ethyl | O | $CH_2C{\equiv}CH$ | |
| H | $C_2H_5$ | H | n-Propyl | O | $CH_2C{\equiv}CH$ | |
| H | $C_2H_5$ | H | iso-Propyl | O | $CH_2C{\equiv}CH$ | |
| H | $C_2H_5$ | H | Cyclopropyl | O | $CH_2C{\equiv}CH$ | |
| H | $C_2H_5$ | H | n-Butyl | O | $CH_2C{\equiv}CH$ | |
| H | $C_2H_5$ | H | iso-Butyl | O | $CH_2C{\equiv}CH$ | |
| H | $C_2H_5$ | H | sek.-Butyl | O | $CH_2C{\equiv}CH$ | |
| H | $C_2H_5$ | H | tert.-Butyl | O | $CH_2C{\equiv}CH$ | |

Tabelle (Fortsetzung)

| $R^3$ | $R^4$ | $R^1$ | $R^2$ | Y | $R^6$ | X = O oder S |
|---|---|---|---|---|---|---|
| H | $C_2H_5$ | H | n-Pentyl | O | $CH_2C{\equiv}CH$ | |
| H | $C_2H_5$ | H | 2-Pentyl | O | $CH_2C{\equiv}CH$ | |
| H | $C_2H_5$ | H | 3-Pentyl | O | $CH_2C{\equiv}CH$ | |
| H | $C_2H_5$ | H | n-Hexyl | O | $CH_2C{\equiv}CH$ | |
| H | $C_2H_5$ | H | 2-Hexyl | O | $CH_2C{\equiv}CH$ | |
| H | $C_2H_5$ | H | 3-Hexyl | O | $CH_2C{\equiv}CH$ | |
| H | $C_2H_5$ | H | 2-Methyl-2-pentyl | O | $CH_2C{\equiv}CH$ | |
| H | $C_2H_5$ | H | cyclo-Propylmethyl | O | $CH_2C{\equiv}CH$ | |
| H | $C_2H_5$ | H | cyclo-Butyl | O | $CH_2C{\equiv}CH$ | |
| H | $C_2H_5$ | H | cyclo-Pentyl | O | $CH_2C{\equiv}CH$ | |
| H | $C_2H_5$ | H | cyclo-Hexyl | O | $CH_2C{\equiv}CH$ | |
| H | $C_2H_5$ | H | 1-Methylcyclohexyl | O | $CH_2C{\equiv}CH$ | |
| H | $C_2H_5$ | H | 3-Triflormethylcyclohexyl | O | $CH_2C{\equiv}CH$ | |
| H | $C_2H_5$ | H | Allyl | O | $CH_2C{\equiv}CH$ | |
| H | $C_2H_5$ | H | 1-Buten-3-yl | O | $CH_2C{\equiv}CH$ | |
| H | $C_2H_5$ | H | Crotyl | O | $CH_2C{\equiv}CH$ | |
| H | $C_2H_5$ | H | Propargyl | O | $CH_2C{\equiv}CH$ | |
| H | $C_2H_5$ | H | 1-Butin-3-yl | O | $CH_2C{\equiv}CH$ | |
| H | $C_2H_5$ | H | 3-Methyl-1-butin-3-yl | O | $CH_2C{\equiv}CH$ | |
| H | $C_2H_5$ | H | 2-Pentin-4-yl | O | $CH_2C{\equiv}CH$ | |
| H | $C_2H_5$ | H | Benzyl | O | $CH_2C{\equiv}CH$ | |
| H | $C_2H_5$ | H | 2-Phenylethyl | O | $CH_2C{\equiv}CH$ | |
| H | $C_2H_5$ | H | 2-Methylthioethyl | O | $CH_2C{\equiv}CH$ | |

Tabelle (Fortsetzung)

| $R^3$ | $R^4$ | $R^1$ | $R^2$ | Y | $R^6$ | X = O oder S |
|---|---|---|---|---|---|---|
| H | $C_2H_5$ | H | 2-Chlorethyl | O | $CH_2C{\equiv}CH$ | |
| H | $C_2H_5$ | H | 2-Methoxyethyl | O | $CH_2C{\equiv}CH$ | |
| H | $C_2H_5$ | H | 2-(N,N-Dimethylamino)ethyl | O | $CH_2C{\equiv}CH$ | |
| H | $C_2H_5$ | H | Phenyl | O | $CH_2C{\equiv}CH$ | |
| H | $C_2H_5$ | H | 2-$CH_3$-Phenyl | O | $CH_2C{\equiv}CH$ | |
| H | $C_2H_5$ | H | 3-$CH_3$-Phenyl | O | $CH_2C{\equiv}CH$ | |
| H | $C_2H_5$ | H | 4-$CH_3$-Phenyl | O | $CH_2C{\equiv}CH$ | |
| H | $C_2H_5$ | H | 2,4-($CH_3$,$CH_3$)-Phenyl | O | $CH_2C{\equiv}CH$ | |
| H | $C_2H_5$ | H | 2,3,5-($CH_3$,$CH_3$,$CH_3$)-Phenyl | O | $CH_2C{\equiv}CH$ | |
| H | $C_2H_5$ | H | 3-$CF_3$-Phenyl | O | $CH_2C{\equiv}CH$ | |
| H | $C_2H_5$ | H | 3-F-Phenyl | O | $CH_2C{\equiv}CH$ | |
| H | $C_2H_5$ | H | 2-Cl-Phenyl | O | $CH_2C{\equiv}CH$ | |
| H | $C_2H_5$ | H | 4-Cl-Phenyl | O | $CH_2C{\equiv}CH$ | |
| H | $C_2H_5$ | H | 2,4-(F,F)-Phenyl | O | $CH_2C{\equiv}CH$ | |
| H | $C_2H_5$ | H | 2,3,5-(Cl,Cl,Cl)-Phenyl | O | $CH_2C{\equiv}CH$ | |
| H | $C_2H_5$ | H | 2-CN-Phenyl | O | $CH_2C{\equiv}CH$ | |
| H | $C_2H_5$ | H | 2-$OCH_3$-Phenyl | O | $CH_2C{\equiv}CH$ | |
| H | $C_2H_5$ | H | 2,3-($OCH_3$,$OCH_3$)-Phenyl | O | $CH_2C{\equiv}CH$ | |
| H | $C_2H_5$ | H | 3,4,5-($OCH_3$,$OCH_3$,$OCH_3$)-Phenyl | O | $CH_2C{\equiv}CH$ | |
| H | $C_2H_5$ | H | 3-$OCF_3$-Phenyl | O | $CH_2C{\equiv}CH$ | |
| H | $C_2H_5$ | H | 3-$OCF_2OHF_2$-Phenyl | O | $CH_2C{\equiv}CH$ | |
| H | $C_2H_5$ | H | 4-$OCF_2CHF_2$-Phenyl | O | $CH_2C{\equiv}CH$ | |
| H | $C_2H_5$ | H | 2-$SCH_3$-Phenyl | O | $CH_2C{\equiv}CH$ | |

Tabelle (Fortsetzung)

| $R^3$ | $R^4$ | $R^1$ | $R^2$ | Y | $R^6$ | X = O oder S |
|---|---|---|---|---|---|---|
| H | $C_2H_5$ | H | 2,4-($SCH_3$, $SCH_3$)-Phenyl | O | $CH_2C{\equiv}CH$ | |
| H | $C_2H_5$ | H | 2-$SCF_3$-Phenyl | O | $CH_2C{\equiv}CH$ | |
| H | $C_2H_5$ | H | 4-$NO_2$-Phenyl | O | $CH_2C{\equiv}CH$ | |
| H | $C_2H_5$ | H | 2,4-($NO_2$, $NO_2$)-Phenyl | O | $CH_2C{\equiv}CH$ | |
| H | $C_2H_5$ | H | 3-$COCH_3$-Phenyl | O | $CH_2C{\equiv}CH$ | |
| H | $C_2H_5$ | H | 3-$COCF_3$-Phenyl | O | $CH_2C{\equiv}CH$ | |
| H | $C_2H_5$ | H | 1-Naphthyl | O | $CH_2C{\equiv}CH$ | |
| H | $C_2H_5$ | H | 2-Naphthyl | O | $CH_2C{\equiv}CH$ | |
| H | $C_2H_5$ | H | Piperidino | O | $CH_2C{\equiv}CH$ | |
| H | $C_2H_5$ | H | 3-Tetrahydrofuranyl | O | $CH_2C{\equiv}CH$ | |
| H | $C_2H_5$ | H | 4-Tetrahydropyranyl | O | $CH_2C{\equiv}CH$ | |
| H | $C_2H_5$ | H | 2-Thiazolyl | O | $CH_2C{\equiv}CH$ | |
| H | $C_2H_5$ | H | 5-$CH_3$-2-Thiazolyl | O | $CH_2C{\equiv}CH$ | |
| H | $C_2H_5$ | H | 4-$CH_3$-5-COOH-2-Thiazolyl | O | $CH_2C{\equiv}CH$ | |

Als Salze der Verbindungen der Formel IA, IB und IC kommen landwirtschaftlich brauchbare Salze, beispielsweise Alkalimetallsalze, wie das Kalium-oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden Verbindungen IA, IB und IC bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen IA, IB und IC eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,01 und 95 Gew.%, vorzugsweise zwischen 0,1 und 90 Gew.-% Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen IA, IB und IC können beispielsweise wie folgt formuliert werden:

I. Man vermischt 90 Gewichtsteile der Verbindung Nr. 1.025 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 3.031 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5-Gewichtsteilen Calciumsalz der Dodecylbenzolsulfonsäure und 5 Gewichtsteilen des Anlgerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile der Verbindung Nr. 1.001 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlgerungsproduktes von 40 Mol

Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile des Wirkstoffs Nr. 1.025 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile des Wirkstoffs Nr. 3.031 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile des Wirkstoffs Nr. 2.005 werden mit 97 Gewichtsteilen feinteiligem Kaolin vermischt. Man erhält auf diese Weise ein Stäubemittel, das 3 Gew.-% des Wirkstoffs enthält.

VII. 30 Gewichtsteile des Wirkstoffs Nr. 3.001 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 3.031 werden mit 2 Gewichtsteilen Calciumsalz der Dodecylbenzolsulfonsäure, 8 Gewichtsteilen Fettalkohol-polyglykolether, 2 Gewichtsteilen Natriumsalze eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 1.022 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 2.006 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

XI. 20 Gewichtsteile der Verbindung Nr. 3.036 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

XII. 20 Gewichtsteile des Wirkstoffs Nr. 1.010 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

XIII. 20 Gewichtsteile des Wirkstoffs Nr. 3.015 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

XIV. 3 Gewichtsteile des Wirkstoffs Nr. 1.025 werden mit 97 Gewichtsteilen feinteiligem Kaolin vermischt. Man erhält auf diese Weise ein Stäubemittel, das 3 Gew.-% des Wirkstoffs enthält.

XV. 30 Gewichtsteile des Wirkstoffs Nr. 3.031 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 2.001 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt

werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay by).

Die Aufwandmengen an Wirkstoff bei Anwendung als Herbizide betragen je nach Bekämpfungsziel, Jahreszeit, Zielpflanzen und Wacchstumsstadium 0,001 bis 5, vorzugsweise 0,01 bis 3 kg/ha aktive Substanz (a.S.).

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die erfindungsgemäßen Wirkstoffe IA, IB und IC mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäurer, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäure sowie deren Salze, Ester und Amide und andere in Betracht.

Außerdem kann es von Nutzen sein, die Wirkstoffe IA, IB und IC allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen IA, IB und IC benutzt. Die so erhaltenen Verbindung sind in den nachstehenden Tabellen mit physikalischen Angaben aufgeführt.

Herstellungsbeispiele:

Beispiel 1

Thiophen-3,4-dicarbonsäureanhydrid

Thiophen-3,4-dicarbonsäure (12 g, 0,07 Mol) wird in Acetanhydrid (60 ml) gelöst und 2,5 Stunden zum Sieden erhitzt. Anschließend wird zur Trockene eingeengt. man erhält 10,55 g (98 %) Thiophen-3,4-dicarbonsäureanhydrid (Fp. 147 bis 149 °C).

Beispiel 2

4-(4-Chlorphenyl)aminocarbonyl-thiophen-3-carbonsäure

Thiophen-3,4-dicarbonsäureanhydrid (2 g, 0,013 Mol) wird in 60 ml Toluol vorgelegt und mit 4-Chloranilin (1,65 g, 0,013 Mol) versetzt. Man läßt 3 Stunden bei Raumtemperatur rühren, filtriert die ausgefallene Carbon säure ab und wäscht mit wenig Toluol nach. Nach Trocknen erhält man 3,7 g (100 %) des gewünschten Produktes, Fp. 204 bis 208 °C. (Wirkstoff Nr. 1.035)

Beispiel 3

4-(4-Chlorphenyl)aminocarbonyl-thiophen-3-carbonsäure-hydroxysuccinimidester

4-(4-Chlorphenyl)aminocarbonyl-thiophen-3-carbonsäure (1,7 g 0,006 Mol) wird in 60 ml THF gelöst, mit N-Hydroxysuccinimid (0,7 g 0,006 Mol) und N,N-Dicyclohexylcarbodiimid (1,25 g, 0,006 Mol) versetzt und mehrere Stunden bei Raumtemperatur gerührt. Anschließend kühlt man die Lösung für einige Stunden auf 0° C, saugt den ausgefallenen Harnstoff ab und engt zur Trockene ein. Ausbeute: 1,8 g, 66 %, Fp. 65 bis 67° C. (Wirkstoff Nr. 1.026)

Beispiel 4

4-Chlorthiophen--3-carbonsäureanilid

4-Chlorthiophen-3-carbonsäurechlorid (13,5 g 0,075 Mol) werden in 30 ml Dioxan gelöst und bei Raumtemperatur zu einer Lösung aus Anilin (7,7 g 0,083 Mol) in Pyridin (160 ml) getropft. Man läßt 12 Stunden bei Raumtemperatur rühren, engt dann zur Trockene ein und nimmt den Rückstand in Dichlormethan auf. Die organische Phase wird mit wäßriger Citronensäurelösung, Natriumhydrogencarbonatlösung und Wasser extrahiert, getrocknet und zur Trockne eingeengt. Ausbeute: 16,7 g, 94 %; Fp. 119 bis 121° C.

Beispiel 5

4-Chlor-3-phenylaminocarbonyl-thiophen-2-carbonsäure

4-Chlorthiophen-3-carbonsäureanilid (14,6 g, 0,061 Mol) wird in 450 ml THF gelöst, auf -70° C gekühlt und mit n-Butyllithium (0,13 Mol, 1,6 N Lösung in n-Hexan) versetzt. Nach 30 Minuten gast man bis zur Sättigung der Lösung Kohlendioxid ein und läßt langsam auf Raumtemperatur aufwärmen. Zur Aufarbeitung ent man bis zur Trockene ein, nimmt den festen Rückstand in einem Gemisch aus Wasser, Natronlauge und Ethylacetat auf, trennt die Phase und säuert die wäßrige Phase mit Salzsäure an. Dabei fällt die Carbonsäure aus. Ausbeute: 13,5 g: 78 %; Fp. 208 bis 210° C. (Wirkstoff Nr. 2.008)

Wirkstofftabelle 1

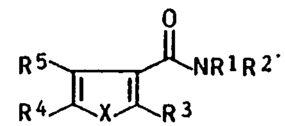

Ia/IA

| Nr. | $R^1$ | $R^2$ | $R^3$ | $R^4$ | X | $R^5$ | Fp. [°C] |
|---|---|---|---|---|---|---|---|
| 1.001 | H | $3-OCH_3-C_6H_4$ | H | H | S | 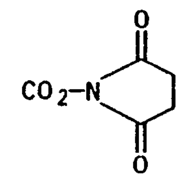 | 147-149 |
| 1.002 | H | $3-CH_3-C_6H_4$ | H | H | S | 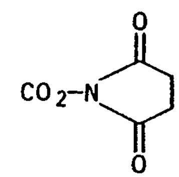 | 184-186 |
| 1.003 | H | $2-Cl-C_6H_4$ | H | H | S | 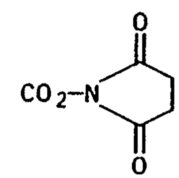 | 128-129 |
| 1.004 | H | Cyclopropyl | H | H | S | 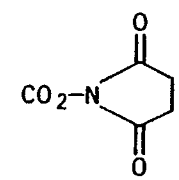 | 75- 77 |

Wirkstofftabelle 1 (Fortsetzung)

| Nr. | $R^1$ | $R^2$ | $R^3$ | $R^4$ | X | $R^5$ | Fp. [°C] |
|---|---|---|---|---|---|---|---|
| 1.005 | H | $C_6H_5$ | H | H | S | $CO_2$- 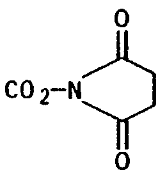 | 158-160 |
| 1.006 | H | $CH(CH_3)_2$ | H | H | S | $CO_2$- 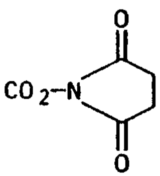 | 179-181 |
| 1.007 | H | 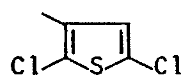 | H | H | S | $CO_2H$ | 200-202 |
| 1.008 | H | $CH(CH_3)_2$ | H | H | O | $CO_2CH_2CH_3$ | 32- 34 |
| 1.009 | H | $CH(CH_2)_2$ | H | H | O | $CO_2H$ | 171-174 |
| 1.010 | H | $C(CH_3)_3$ | H | H | O | $CO_2CH_2CH_3$ | 48- 50 |
| 1.011 | H | $C(CH_3)_3$ | H | H | O | $CO_2H$ | 190-194 |
| 1.012 | H | $C_6H_5$ | H | H | O | $CO_2CH_2CH_3$ | 154-156 |
| 1.013 | H | $C_6H_5$ | H | H | O | $CO_2H$ | 265-270 |
| 1.014 | H | $3-CF_3-C_6H_4$ | H | H | O | $CO_2CH_2CH_3$ | 85- 87 |
| 1.015 | H | $3-CF_3-C_6H_4$ | H | H | O | $CO_2H$ | 245-249 |

Wirkstofftabelle 1 (Fortsetzung)

| Nr. | $R^1$ | $R^2$ | $R^3$ | $R^4$ | X | $R^5$ | Fp. [°C] |
|---|---|---|---|---|---|---|---|
| 1.016 | H | 4-Cl-$C_6H_4$ | H | H | O | $CO_2CH_2CH_3$ | 111-114 |
| 1.017 | H | 4-Cl-$C_6H_4$ | H | H | O | $CO_2H$ | 251-255 |
| 1.018 | H | 3-F-$C_6H_4$ | H | H | S | $CO_2H$ | 200-204 |
| 1.019 | H | 3-$CF_3$-$C_6H_4$ | H | H | S | $CO_2H$ | 215-217 |
| 1.020 | H | $CH_2$-$C_6H_4$ | H | H | S | $CO_2H$ | 215-218 |
| 1.021 | H | 4-F-$C_6H_4$ | H | H | S | $CO_2H$ | 218-220 |
| 1.022 | H | Cyclopropyl | H | H | S | $CO_2H$ | 170-173 |
| 1.023 | H | -$CH_2$-Cyclopropyl | H | H | S | $CO_2H$ | 165-168 |
| 1.024 | H | 4-F-$C_6H_4$ | H | H | S | 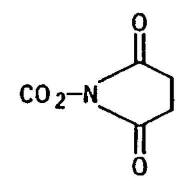 | 135-137 |
| 1.025 | H | 3-F-$C_6H_4$ | H | H | S | 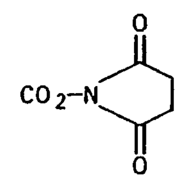 | 140-142 |
| 1.026 | H | 4-Cl-$C_6H_4$ | H | H | S | 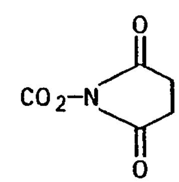 | 65- 67 |

Wirkstofftabelle 1 (Fortsetzung)

| Nr. | $R^1$ | $R^2$ | $R^3$ | $R^4$ | X | $R^5$ | Fp. [°C] |
|---|---|---|---|---|---|---|---|
| 1.027 | H | 3-Cl-$C_6H_4$ | H | H | S | 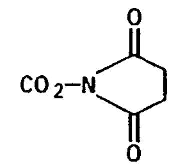 | 146-149 |
| 1.028 | H | 3,4-Cl,Cl-$C_6H_3$ | H | H | S | 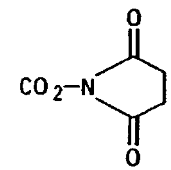 | 205-207 |
| 1.029 | H | $CH(CH_3)_2$ | H | H | S | $CO_2H$ | 161-169 |
| 1.030 | H | $C(CH_3)_3$ | H | H | S | $CO_2H$ | 127-139 |
| 1.031 | H | 3-$CF_3$-$C_6H_4$ | H | H | S | 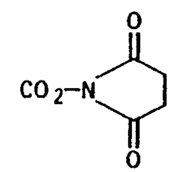 | 143-147 |
| 1.032 | H | $C_6H_5$ | H | H | S | $CO_2H$ | 205-208 |
| 1.033 | H | 3-Cl-$C_6H_4$ | H | H | S | $CO_2H$ | 235-237 |
| 1.034 | H | 2-Cl-$C_6H_4$ | H | H | S | $CO_2H$ | 186-187 |
| 1.035 | H | 4-Cl-$C_6H_4$ | H | H | S | $CO_2H$ | 204-208 |
| 1.036 | H | 3,4-Cl,Cl-$C_6H_3$ | H | H | S | $CO_2H$ | > 230 |
| 1.037 | H | 3-$OCH_3$-$C_6H_4$ | H | H | S | $CO_2H$ | 170-173 |
| 1.038 | H | 4-$OCH_3$-$C_6H_4$ | H | H | S | $CO_2H$ | 250-253 |
| 1.039 | H | 3-$CH_3$-$C_6H_4$ | H | H | S | $CO_2H$ | 220-223 |

Wirkstofftabelle 1 (Fortsetzung)

| Nr. | $R^1$ | $R^2$ | $R^3$ | $R^4$ | X | $R^5$ | Fp. [°C] |
|---|---|---|---|---|---|---|---|
| 1.040 | H | 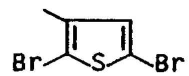 | H | H | S | $CO_2H$ | |
| 1.041 | H | 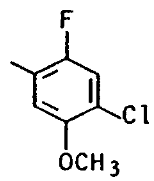 | H | H | S | $CO_2H$ | 183-185 |
| 1.042 | H | 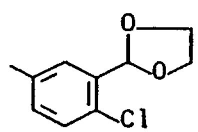 | H | H | S | $CO_2H$ | 206-208 |

Wirkstofftabelle 2

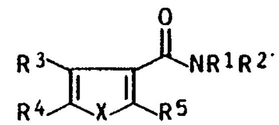

Ib/IB

| Nr. | $R^1$ | $R^2$ | $R^3$ | $R^4$ | X | $R^5$ | Fp. [°C] |
|---|---|---|---|---|---|---|---|
| 2.001 | H | $CH(CH_3)_2$ | H | H | S | $CO_2H$ | 160-162 |
| 2.002 | H | $3-CF_3-C_6H_4$ | H | H | S | $CO_2H$ | 158-160 |
| 2.003 | H | $C_6H_5$ | H | H | S | $CO_2H$ | 190-192 |
| 2.004 | H | $C(CH_3)_3$ | H | H | S | $CO_2H$ | 163-165 |
| 2.005 | H | $CH(CH_3)_2$ | H | H | S | $CO_2$- 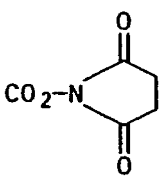 | 197-199 |
| 2.006 | H | $C(CH_3)_3$ | Cl | H | S | $CO_2H$ | 176-179 |
| 2.007 | H | $3-CF_3-C_6H_4$ | Cl | H | S | $CO_2H$ | 186-188 |
| 2.008 | H | $C_6H_5$ | Cl | H | S | $CO_2H$ | 208-210 |
| 2.009 | H | $CH(CH_3)_2$ | Cl | H | S | $CO_2H$ | 219-221 |
| 2.010 | H | $CH(CH_3)_2$ | CN | H | S | $CO_2H$ | 195-197 |
| 2.011 | H | $C(CH_3)_3$ | H | Cl | S | $CO_2H$ | 216-218 |
| 2.012 | H | $3-CF_3-C_6H_4$ | Cl | Cl | S | $CO_2H$ | 242-244 |

Wirkstofftabelle 3

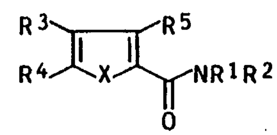

Ic/IC

| Nr. | $R^1$ | $R^2$ | $R^3$ | $R^4$ | X | $R^5$ | Fp. [°C] |
|---|---|---|---|---|---|---|---|
| 3.001 | H | $C(CH_3)_3$ | H | H | S | $CO_2H$ | 179-182 |
| 3.002 | H | $3-CF_3-C_6H_4$ | H | H | S | $CO_2H$ | 151-153 |
| 3.003 | H | $4-Cl-C_6H_4$ | H | H | S | $CO_2H$ | 218-220 |
| 3.004 | H | $C_6H_5$ | H | H | S | $CO_2H$ | 258-260 |
| 3.005 | $CH_3$ | $CH_3$ | H | H | S | $CO_2H$ | 155-160 |
| 3.006 | H | $C(CH_3)_2CH_2CH_3$ | H | H | S | $CO_2H$ | 77- 84 |
| 3.007 | H | $C(CH_3)_2CH=CH_2$ | H | H | S | $CO_2H$ | 98-100 |
| 3.008 | H | $C(C_2H_5)_2C\equiv CH$ | H | H | S | $CO_2H$ | öl |
| 3.009 | H | $C(CH_3)_2CN$ | H | H | S | $CO_2H$ | 200-205 |
| 3.010 | H | $C(CH_3)_2CH_2SCH_3$ | H | H | S | $CO_2H$ | 108-112 |
| 3.011 | H | 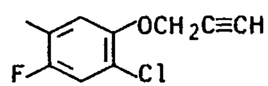 | H | H | S | $CO_2H$ | 242-247 |
| 3.012 | H | 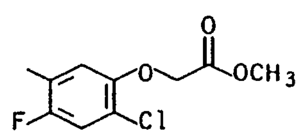 | H | H | S | $CO_2H$ | 222-226 |

Wirkstofftabelle 3 (Fortsetzung)

| Nr. | $R^1$ | $R^2$ | $R^3$ | $R^4$ | X | $R^5$ | Fp. [°C] |
|---|---|---|---|---|---|---|---|
| 3.013 | H | $C(CH_3)_3$ | H | Cl | S | $CO_2H$ | 233-235 |
| 3.014 | H | Cyclopropyl | H | Cl | S | $CO_2H$ | 204-206 |
| 3.015 | H | $CH(CH_3)_2$ | H | Cl | S | $CO_2H$ | 175-177 |
| 3.016 | H | $C_6H_5$ | H | Cl | S | $CO_2H$ | 220-222 |
| 3.017 | H | $3-CF_3-C_6H_4$ | H | Cl | S | $CO_2H$ | 158-160 |
| 3.018 | H | $CH(CH_3)_2$ | H | Cl | S | 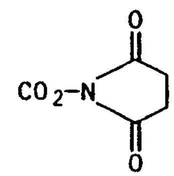 | 207-209 |
| 3.019 | H | $C(CH_3)_3$ | H | Cl | S | 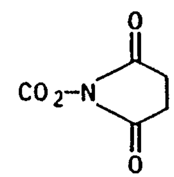 | 181-183 |
| 3.020 | H | $C(CH_3)_3$ | H | Cl | S | 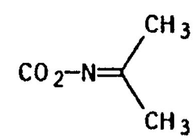 | 188-190 |
| 3.021 | H | $OC(CH_3)_3$ | H | Cl | S | $CO_2H$ | 206-208 |
| 3.022 | H | $C(CH_3)_3$ | H | $CH_3$ | S | $CO_2H$ | 162-166 |
| 3.023 | H | $CH(CH_3)_2$ | H | $CH_3$ | S | $CO_2H$ | 88- 90 |

Wirkstofftabelle 3 (Fortsetzung)

| Nr. | $R^1$ | $R^2$ | $R^3$ | $R^4$ | X | $R^5$ | Fp. [°C] |
|---|---|---|---|---|---|---|---|
| 3.024 | H | $C(CH_3)_3$ | H | $CH_3$ | S | 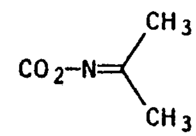 | 148-152 |
| 3.025 | H | $CH(CH_3)_2$ | H | $CH_3$ | S | 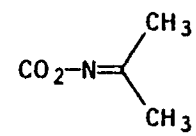 | 78- 80 |
| 3.026 | H | Cyclopropyl | H | $CH_3$ | S | $CO_2H$ | 147-149 |
| 3.027 | H | 4-Cl-$C_6H_4$ | H | $CH_3$ | S | $CO_2H$ | 230-235 |
| 3.028 | H | $C(CH_3)_3$ | H | H | O | $CO_2H$ | 123-126 |
| 3.029 | H | $CH(CH_3)_2$ | H | H | O | $CO_2H$ | 62- 70 |
| 3.030 | H | 3-$CF_3$-$C_6H_4$ | H | H | O | $CO_2H$ | 170-173 |
| 3.031 | H | $C(CH_3)_3$ | H | $CH_3$ | O | $CO_2H$ | 124-127 |
| 3.032 | H | $C(CH_3)_3$ | H | $CH_3$ | O | $CO_2N=C(CH_3)_2$ | 132-136 |
| 3.033 | H | $CH(CH_3)_2$ | H | $CH_3$ | O | $CO_2H$ | 138-141 |
| 3.034 | H | $CH(CH_3)_2$ | H | $CH_3$ | O | $CO_2N=C(CH_3)_2$ | 102-106 |
| 3.035 | H | Cyclopropyl | H | $CH_3$ | O | $CO_2H$ | 154-160 |
| 3.036 | H | 4-Cl-$C_6H_4$ | H | $CH_3$ | O | $CO_2H$ | 230-232 |
| 3.037 | H | 4-Cl-$C_6H_4$ | H | $CH_3$ | O | $CO_2N=C(CH_3)_2$ | 160-168 |

Wirkstofftabelle 3 (Fortsetzung)

| Nr. | $R^1$ | $R^2$ | $R^3$ | $R^4$ | X | $R^5$ | Fp. [°C] |
|---|---|---|---|---|---|---|---|
| 3.038 | H | $CH(CH_3)_2$ | H | H | S | $CO_2H$ | 141-143 |
| 3.039 | H | $CH(CH_3)_2$ | H | Cl | S | $CO_2CH_2C{\equiv}CH$ | 93- 95 |
| 3.040 | H | $C_6H_{11}$ | H | Cl | S | $CO_2H$ | 251-253 |
| 3.041 | H | Cyclopropyl | H | Cl | S | $CO_2N{=}C(CH_3)_2$ | 124-126 |
| 3.042 | H | $C(CH_3)_3$ | H | $OCH_3$ | S | $CO_2H$ | 176-178 |
| 3.043 | H | Cyclopropyl | H | $OCH_3$ | S | $CO_2H$ | 168-170 |
| 3.044 | H | Cyclopropyl | H | Cl | S | $CO_2$– 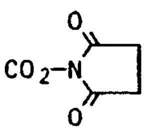 | 215-217 |
| 3.045 | H | 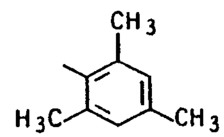 | H | Cl | S | $CO_2H$ | 246-248 |
| 3.046 | H | 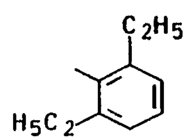 | H | Cl | S | $CO_2H$ | 170-172 |
| 3.047 | H | 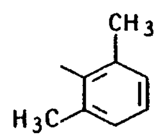 | H | $CH_3$ | S | $CO_2H$ | 237-239 |

Wirkstofftabelle 3 (Fortsetzung)

| Nr. | R1 | R2 | R3 | R4 | X | R5 | Fp. [°C] |
|---|---|---|---|---|---|---|---|
| 3.048 | H | $C_6H_5$ | $C_6H_5$ | $C_6H_5$ | O | $CO_2CH_3$ | |
| 3.049 | H | 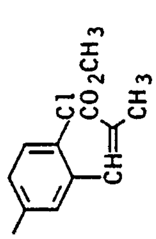 | H | H | S | $CO_2H$ | 178-184 |
| 3.050 | H | $3-CF_3-C_6H_4$ | H | $4-CH_3-C_6H_4$ | S | $CO_2H$ | 262-268 |
| 3.051 | H | $C(CH_3)_3$ | H | $4-CH_3-C_6H_4$ | S | $CO_2H$ | 144-146 |

Anwendungsbeispiele:

Die herbizide Wirkung der Carbonsäureamide der Formel IA, IB und IC ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und

Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,5 und 1,0 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Chenopodium album | Weißer Gänsefuß |
| Chrysanthemum coronarium | Kronenwucherblume |
| Euphorb ia heterophylla | Wolfsmilchart |
| Galium aparine | Klettenlabkraut |
| Triticum aestivum | Winterweizen |
| Hordeum vulgaris | Sommergerste |
| Polygonum persicaria | Flohknöterich |
| Sinapis alba | Weißer Senf |
| Matricaria inodora | duftlose Kamille |
| Ipomoea spp. | Prunkwindearten |
| Zea mays | Mais |

Mit 0,5 bzw. 1,0 kg/ha a.S. im Nachauflaufverfahren eingesetzt, lassen sich mit den Beispielen 1.025, 2.003, 3.014, 3.019, 3.031 und 3.032 breitblättrige unerwünschte Pflanzen sehr gut bekämpfen. Beispiele 1.025 und 3.014 zeigen dabei gleichzeitig gute Verträglichkeit bei der Kultur Weizen.

## Ansprüche

1. Carbonsäureamide der allgemeinen Formeln Ia bis Ic

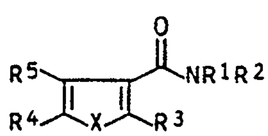

Ia

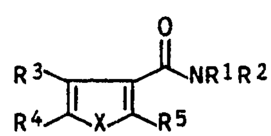

Ib

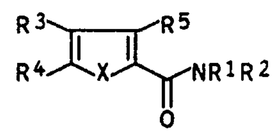

Ic

in denen die Substituenten folgende Bedeutung haben:

X

Sauerstoff oder Schwefel;

$R^1$

Wasserstoff;

$C_3$-$C_8$-Cycloalkyl, welches ein bis drei der folgenden Reste tragen kann: Halogen, $C_1$-$C_4$-Alkyl, $C_1$-$C_4$-Halogenalkyl, $C_1$-$C_4$-Alkoxy und/oder $C_1$-$C_4$-Halogenalkoxy;

$C_1$-$C_6$-Alkyl, welches ein bis drei der folgenden Reste tragen kann: Hydroxy, Halogen, $C_3$-$C_8$-Cycloalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylamino, Di-$C_1$-$C_4$-

alkylamino und/oder $C_3$-$C_6$-Cycloalkylamino und/oder einen Rest

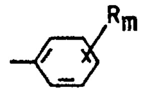

tragen kann, wobei

R Cyano; Nitro; Halogen; $C_1$-$C_4$-Alkyl; $C_1$-$C_4$-Halogenalkyl; $C_1$-$C_4$-Alkoxy, $C_1$-$C_4$-Halogenalkoxy, $C_2$-$C_4$-Alkinyloxy, $C_1$-$C_4$-Alkylthio; $C_1$-$C_4$-Halogenalkylthio; $C_3$-$C_6$-Alkoxycarbonylalkoxy und/oder $C_1$-$C_4$-Alkoxycarbonyl bedeutet und

m für 0 , 1, 2 oder 3 steht, wobei die Reste R verschieden sein können, wenn m 2 oder 3 bedeutet;

$R^2$

Hydroxy; $C_1$-$C_4$-Alkoxy; $C_2$-$C_6$-Cyanalkyl;

$C_3$-$C_6$-Alkenyl, $C_3$-$C_6$-Alkinyl, Phenyl oder Naphthyl, wobei diese Gruppen ein bis drei der bei R genannten Reste tragen können;

einen 5- bis 6-gliedrigen Heterocyclus, enthaltend ein oder zwei Heteroatome ausgewählt aus der Gruppe Stickstoff, Sauerstoff und Schwefel, wobei dieser Ring ein oder zwei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

eine der bei $R^1$ genannten Gruppen oder

$R^1$ und $R^2$ gemeinsam eine 4- bis 7-gliedrige Kette, welche neben Methylengruppen eine der folgenden Gruppen als Ringglied enthalten kann: Sauerstoff, Schwefel, N-Methyl oder Carbonyl;

$R^3$, $R^4$

Nitro, Cyano; Halogen;

Amino, welches ein oder zwei $C_1$-$C_4$-Alkylgruppen und/oder eine $C_1$-$C_4$-Alkylcarbonylgruppe tragen kann;

$C_1$-$C_4$-Alkoxy oder $C_1$-$C_4$-Alkylthio, wobei diese Gruppen ein bis neun Halogenatome tragen können;

einen 5- bis 6-gliedrigen Heterocyclus, enthaltend ein oder zwei Heteroatome ausgewählt aus der Gruppe Sauerstoff, Schwefel und Stickstoff, wobei dieser Ring ein oder zwei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy und/oder $C_1$-$C_4$-Alkylthio;

$C_2$-$C_6$-Alkenyl, $C_2$-$C_6$-Alkinyl, Phenyl, Phenoxy oder Phenylthio, wobei diese Gruppen ein bis drei der bei R genannten Reste tragen können, oder

eine der bei $R^1$ genannten Gruppen;

$R^5$

Formyl, 4,5-Dihydrooxazol-2-yl oder eine Gruppe $COYR^6$;

Y Sauerstoff oder Schwefel;

$R^6$ Wasserstoff;

$C_3$-$C_8$-Cycloalkyl;

$C_1$-$C_6$-Alkyl, welches ein bis fünf Halogenatome oder Hydroxygruppen und/ oder einen der folgenden Reste tragen kann: Cyano, Aminocarbonyl, Carboxyl, Trimethylsilyl, $C_1$-$C_4$-Alkoxy, $C_1$-$C_4$-Alkoxy-$C_1$-$C_4$-alkoxy, $C_1$-$C_4$-Alkylthio, $C_1$-$C_4$-Alkylamino, Di-$C_1$-$C_4$-alkylamino, $C_1$-$C_4$-Alkylsulfinyl, $C_1$-$C_4$-Alkylsulfonyl, $C_1$-$C_4$-Alkoxycarbonyl, $C_2$-$C_4$-Alkoxycarbonyl-$C_1$-$C_3$-alkoxy, $C_2$-$C_4$-Alkoxycarbonyl-$C_1$-$C_5$-alkoxycarbonyl, $C_1$-$C_4$-Alkylaminocarbonyl, Di-$C_1$-$C_4$-alkylaminocarbonyl, Di-$C_1$-$C_4$-alkylphosphonyl, $C_1$-$C_4$-Alkyliminoxi, Phenyl, Thienyl, Benzyloxy, Benzylthio, Furyl, Tetrahydrofuryl, Phthalimido, Pyridyl und/oder Benzoyl, wobei die cyclischen Reste ihrerseits ein bis drei der bei R genannten Reste tragen können;

$C_3$-$C_6$-Alkenyl, $C_3$-$C_6$-Alkinyl oder $C_5$-$C_7$-Cycloalkenyl, wobei diese Gruppen einen der folgenden Reste tragen können: Hydroxy, Halogen, $C_1$-$C_4$-Alkoxy oder Phenyl, wobei der Phenylrest seinerseits ein bis drei der bei R genannten Reste tragen kann;

einen 5- bis 6-gliedrigen Heterocyclus, enthaltend ein oder zwei Heteroatome, ausgewählt aus der Gruppe Stickstoff, Sauerstoff und Schwefel;

Phthalimido, Tetrahydrophthalimido; Succinimido; Maleinimido; Benzotriazolyl;

Phenyl, welches ein bis drei der bei R genannten Reste tragen kann;

eine Gruppe $-N=CR^7R^8$, wobei

$R^7$ Wasserstoff oder $C_1$-$C_6$-Alkyl und

$R^8$ $C_3$-$C_6$-Cycloalkyl, Phenyl, Furyl oder einen Rest $R^7$

bedeutet oder

$R^7$, $R^8$ gemeinsam eine 4- bis 7-gliedrige Alkylenkette bilden,

wobei sofern

- $R^5$ Carboxyl, Methoxycarbonyl oder Ethoxycarbonyl und $R^2$ Wasserstoff bedeutet, $R^3$ nicht Wasserstoff

bedeutet oder $R^4$ nicht Wasserstoff oder Methyl bedeutet und sofern

- $R^5$ Carboxyl, Methoxycarbonyl oder Ethoxycarbonyl und $R^4$ Wasserstoff bedeutet, $R^3$ nicht Wasserstoff oder $R^2$ nicht eine der folgenden Gruppen bedeutet: Wasserstoff; $C_1$-$C_4$-Alkyl; Phenyl; 2-(3,4-Dimethoxyphenyl)ethyl oder 2,5-Dichlorthien-3-yl,

sowie deren landwirtschaftlich brauchbaren Salze.

2. Verfahren zur Hertstellung der Verbindungen Ia gemäß Anspruch 1, in denen $R^5$ eine Carboxylgruppe bedeutet, dadurch gekennzeichnet, daß man ein entsprechendes Dicarbonsäureanhydrid der Formel II

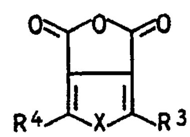

II

in an sich bekannter Weise mit einem Amin der Formel II

$HNR^1R^2$

umsetzt.

3. Verfahren zur Herstellung der Verbindungen Ia, Ib und Ic gemäß Anspruch 1, in denen $R^3$ und $R^4$ nicht Brom und Iod bedeuten und $R^5$ eine Formyl- oder eine Carboxylgruppe bedeutet, dadurch gekennzeichnet, daß man eine entsprechende Carbonsäure der Formel IVa, IVb bzw. IVc

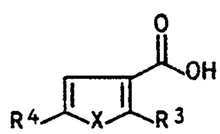

IVa

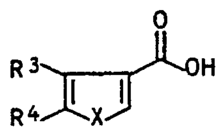

IVb

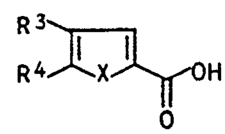

IVc

in an sich bekannter Weise in das Halogenid oder eine andere aktivierte Form der Carbonsäure überführt, diese Derivate anschließend mit einem Amin der Formel III umsetzt und das so erhaltene Carbonsäureamid Va, Vb bzw. Vc

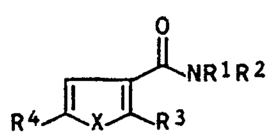

Va

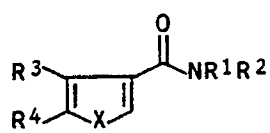

Vb

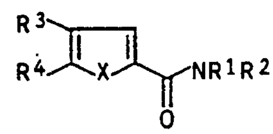

Vc

danach in Gegenwart einer Base mit einem Carboxylierungs- oder einem Formylierungsreagens umsetzt.

4. Verfahren zur Herstellung der Verbindungen Ib und Ic gemäß Anspruch 1, in denen $R^3$ oder $R^4$ Halogen, $R^5$ eine Gruppe $CO_2R^6$ und $R^6$ Alkyl bedeutet, dadurch gekennzeichnet, daß man einen Dicarbonsäureester der allgemeinen Formel VIa oder VIb

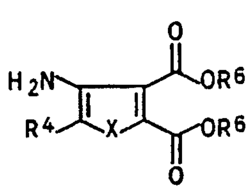

VIa

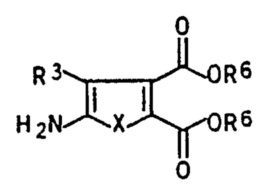

VIb

in an sich bekannter Weise zunächst diazotiert und die diazotierte Verbindung mit einem anorganischen Halogenid in das entsprechende Derivat VIIa bzw. VIIb

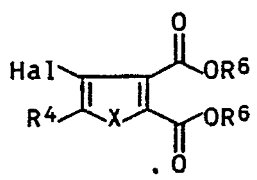

oder

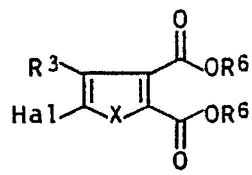

VIIa VIIb

überführt, VIIa bzw. VIIb anschließend mit einem Amin der Formel III gemäß Anspruch 2 amidiert und das so erhaltene Gemisch aus den isomeren Verbindungen Ib und Ic in die Einzelkomponenten auftrennt.

5. Verfahren zur Hertstellung der Verbindungen Ib und Ic gemäß Anspruch 1, in denen $R^5$ eine Carboxylgruppe bedeutet, dadurch gekennzeichnet, daß man ein entsprechendes Carbonsäureamid der Formel Ib bzw. Ic, in der $R^5$ eine Gruppe $CO_2R^6$ und $R^6$ Alkyl bedeutet in an sich bekannter Weise mit einer wäßrigen Base hydrolysiert.

6. Verfahren zur Herstellung der Verbindungen Ia, Ib und Ic gemäß Anspruch 1, in denen $R^5$ eine Grupe $COYR^6$ bedeutet, dadurch gekennzeichnet, daß man ein entsprechendes Dicarbonsäuremonoamid Ia, Ib bzw. Ic, in dem $R^5$ eine Carboxylgruppe bedeutet in an sich bekannter Weise in eine aktivierte Form der Carbonsäure überführt und dieses Derivat anschließend mit einer Verbindung VIII

$HYR^6$ VIII

verestert.

7. Verfahren zur Herstellung der Verbindungen Ia, Ib und Ic gemäß Anspruch 1, in denen $R^5$, 4,5-Dihydrooxazol-2-yl bedeutet, dadurch gekennzeichnet, daß man ein entsprechendes Dicarbonsäureamid Ia, Ib bzw. Ic, in dem $R^5$ eine Gruppe $CO_2H$ bedeutet in an sich bekannter Weise mit 2-Aminoethanol IX

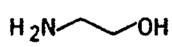

IX

cyclisiert.

8. Herbizides Mittel, enthaltend übliche inerte Zusatzstoffe und mindestens ein Carbonsäureamid der allgemeinen Formel Ia, Ib oder Ic gemäß Anspruch 1 und/oder ein Carbonsäureamid der allgemeinen Formel IA, IB oder IC

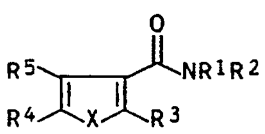

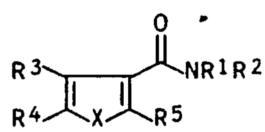

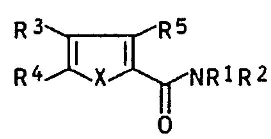

IA IB IC

in denen X und $R^1$ die in Anspruch 1 gegebene Bedeutung haben und

- $R^5$ Carboxyl, Methoxy oder Ethoxycarbonyl und $R^2$ Wasserstoff bedeutet, wenn $R^3$ Wasserstoff bedeutet oder $R^4$ Wasserstoff oder Methyl bedeutet und in denen
- $R^5$ Carboxyl, Methoxy oder Ethoxycarbonyl und $R^4$ Wasserstoff bedeutet, wenn $R^3$ Wasserstoff oder $R^2$ eine der folgenden Gruppen bedeutet: Wasserstoff; $C_1$-$C_4$-Alkyl; Phenyl; 2-(3,4-Dimethoxyphenyl)ethyl oder 2,5-Dichlorthien-3-yl.

9. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Carbonsäureamids Ia, Ib oder Ic gemäß Anspruch 1 und/oder eines Carbonsäureamids IA, IB oder IC gemäß Anspruch 8 behandelt.